(19) Europäisches Patentamt
European Patent Office
Office européen des brevets

(11) EP 1 584 681 A2

(12) EUROPEAN PATENT APPLICATION

(43) Date of publication:
12.10.2005 Bulletin 2005/41

(51) Int Cl.[7]: **C12N 15/11**, C12N 15/31

(21) Application number: **04023620.0**

(22) Date of filing: **10.07.1998**

(84) Designated Contracting States:
**AT BE CH CY DE DK ES FI FR GB GR IE IT LI LU MC NL PT SE**
Designated Extension States:
**AL LT LV MK RO SI**

(30) Priority: **10.07.1997 US 52160 P**

(62) Document number(s) of the earlier application(s) in accordance with Art. 76 EPC:
**98933393.5 / 1 025 219**

(71) Applicant: **GeneSense Technologies Inc.**
**Toronto, Ontario M9W 4Z7 (CA)**

(72) Inventors:
- **Wright, Jim A.**
  **Oakville, Ontario L6L 5Z3 (CA)**
- **Young, Aiping H.**
  **Toronto, Ontario M2N 6V4 (CA)**
- **Dugourd, Dominique**
  **Toronto, Ontario M4P 2M5 (CA)**

(74) Representative: **Brady, Paul Andrew et al**
**Abel & Imray,**
**20 Red Lion Street**
**London WC1R 4PQ (GB)**

Remarks:
This application was filed on 04 - 10 - 2004 as a divisional application to the application mentioned under INID code 62.

# (54) Antisense oligonucleotide sequences as inhibitors of microorganisms

(57) This invention relates to antisense oligonucleotides which modulate the expression of the ribonucleotide reductase or the secA genes in microorganisms. This invention is also related to methods of using such oligonucleotides in inhibiting the growth of microorganisms. These ancisense oligonucleotides are particularly useful in treating pathological conditions in mammals which are mediated by the growth of microorganisms.

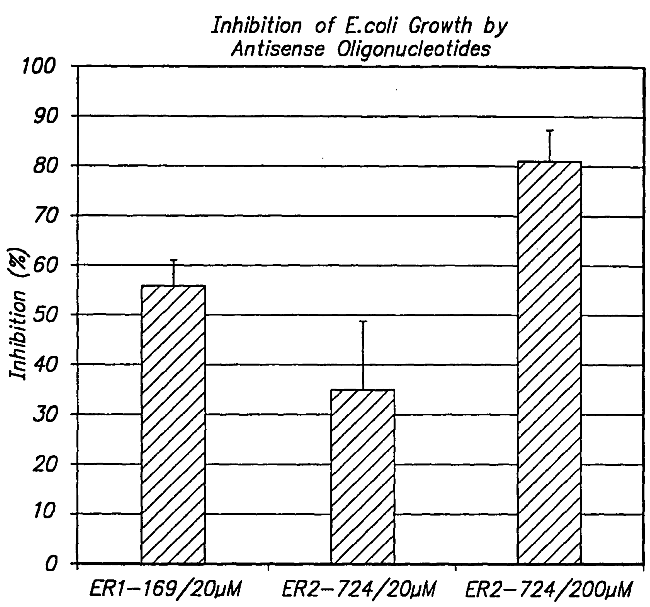


FIG. 15



Printed by Jouve, 75001 PARIS (FR)

SEQUENCE LISTING

<110> GeneSense Technologies Inc.

<120> ANTISENSE OLIGONUCLEOTIDE SEQUENCES AS INHIBITORS OF MICROORGANISMS

<130> 5609(1) EP PAB

<140> 04023620.0

<141> 1998-07-10

<150> US 60/052,160

<151> 1997-07-10

<160> 266

<170> PatentIn Ver. 2.0

<210> 1

<211> 2286

<212> DNA

<213> Escherichia coli

<400> 1

atgaatcaga atctgctggt gacaaagcgc gacggtagca cagagcgcat caatctcgac 60

aaaatccatc gcgttctgga ttgggcggca gaaggactgc ataacgtttc gatttcccag 120

gtcgagctgc gctcccacat tcagttttat gacggtatca agacctctga catccacgaa 180

accattatca aggctgccgc agacctgatc tcccgtgatg cgccggatta tcagtatctc 240

gccgcgcgcc tggcgatctt ccacctgcgt aaaaaagcct acggccagtt tgagccgcct 300

gcgctgtacg accacgtggt gaaaatggtc gagatgggca aatacgataa tcatctgctg 360

gaagactaca cggaagaaga gttcaagcag atggacacct ttatcgatca cgaccgtgat 420

atgaccttct cttatgctgc cgttaagcag ctggaaggca aatatctggt acagaaccgc 480

gtgaccggcg aaatctatga gagcgcccag ttcctttata ttctagttgc cgcgtgcttg 540

ttctcgaact acccgcgtga aacgcgcctg caatatgtga agcgttttta cgacgcggtt 600

tccacattta aaatttcgct gccgacgcca atcatgtccg gcgtgcgtac ccgactcgt 660

cagttcagct cctgcgtact gatcgagtgc ggtgacagcc tggattccat caacgccacc 720

tccagcgcga ttgttaaata cgtttcccag cgtgccggga tcggcatcaa cgccgggcgt 780

attcgtgcgc tgggtagccc gattcgcggt ggtgaagcgt tccataccgg ctgcattccg 840

ttctacaaac atttccagac agcggtgaaa tcctgctctc agggcggtgt gcgcggcggt 900

gcggcaacgc tgttctaccc gatgtggcat ctggaagtgg aaagcctgct ggtgttgaaa 960

aacaaccgtg gtgtggaagg caaccgcgtg cgtcatatgg actacggggt acaaatcaac 1020

aaactgatgt atacccgtct gctgaaaggt gaagatatca ccctgttcag cccgtccgac 1080

gtaccggggc tgtacgacgc gttcttcgcc gatcaggaag agtttgaacg tctgtatacc 1140

aaatatgaga aagacgacag catccgcaag cagcgtgtga aagccgttga gctgttctcg 1200

ctgatgatgc aggaacgtgc gtctaccggt cgtatctata ttcagaacgt tgaccactgc 1260

aatacccata gcccgtttga tccggccatc gcgccagtgc gtcagtctaa cctgtgcctg 1320

gagatagccc tgccgaccaa accgctgaac gacgtcaacg acgagaacgg tgaaatcgcg 1380

ctgtgtacgc tgtctgcttt caacctgggc gcaattaata acctggatga actggaagag 1440

ctggcaattc tggcggttcg tgcacttgac gcgctgctgg attatcagga ttacccgatc 1500

ccggccgcca aacgtggagc gatgggtcgt cgtacgctgg gtattggtgt gatcaacttc 1560

gcttactacc tggcgaacga cggtaaacgc tactccgacg gcagcgccaa caacctgacg 1620

cataaaacct tcgaagccat tcagtattac ctgctgaaag cctctaatga gctggcgaaa 1680

gagcaaggcg cgtgcccgtg gtttaacgaa accacttacg cgaaagggat cctgccgatc 1740

gatacctata agaaagatct ggataccatc gctaatgagc cgctgcatta cgactgggaa 1800

gctctgcgtg agtcaatcaa aacgcacggt ctgcgtaact ccacgctttc tgctctgatg 1860

ccgtccgaga cttcttcgca gatctctaac gccactaacg gtattgaacc gccgcgcggt 1920

tacgtcagca tcaaagcgtc gaaagacggt attttgcgcc aggtggtgcc ggactacgag 1980

cacctgcacg acgcctatga gctgctgtgg gaaatgccgg gtaacgatgg ttatctgcaa 2040

ctggtgggta tcatgcagaa atttatcgat cagtcgatct ctgccaacac caactacgat 2100

ccgtcacgct tcccgtcagg aaaagtgccg atgcagcagt tgctgaaaga cctgctcacc 2160

gcctacaaat tcggggtcaa aacactgtat tatcagaaca cccgtgacgg cgctgaagac 2220

gcacaagacg atctggtgcc gtcaatccag gacgatggct gcgaaagcgg cgcatgtaag 2280

atctga 2286

<210> 2

<211> 1560

<212> DNA

<213> Escherichia coli

<400> 2

ctggtgccgt caatccagga cgatggctgc gaaagcggcg catgtaagat ctgatattga 60

gatgccggat gcggcgtaaa cgccttatcc ggcctacggc tcggtttgta ggcctgataa 120

gacgcgccag cgtcgcatca ggctccgggt gccggatgca gcgtgaacgc cttatccggc 180

ctacggctcg gatttgtagg cctgataaga cgcgccagcg tcgcatcagg cacaggatgc 240

ggcgtaaaat gccttatccg gcattaaact cccaacagga cacactcatg gcatatacca 300

ccttttcaca gacgaaaaat gatcagctca aagaaccgat gttctttggt cagccggtca 360

acgtggctcg ctacgatcag caaaaatatg acatcttcga aaagctgatc gaaaagcagc 420

tctctttctt ctggcgtccg gaagaagttg acgtctcccg cgaccgtata gattaccagg 480

cgctgccgga gcacgaaaaa cacatcttta tcagcaacct gaaatatcag acgctgctgg 540

attccattca gggtcgtagc ccgaacgtgg cgctattgcc gcttatttct attccggaac 600

tggaaacctg ggtcgaaacc tgggcgttct cagaaacgat tcattcccgt tcctatactc 660

atatcattcg taatatcgtt aacgatccgt ctgttgtgtt tgacgatatc gtcaccaacg 720

agcagatcca gaaacgtgcg gaagggatct ccagctatta cgatgagctg atcgaaatga 780

ccagctactg gcatctgctg ggcgaaggta cccacaccgt taacggtaaa actgtgaccg 840

ttagcctgcg cgagctgaag aaaaaactgt atctctgcct gatgagcgtt aacgcgctgg 900

aagcgattcg tttctacgtc agctttgctt gttccttcgc atttgcagaa cgcgaattga 960

tggaaggcaa cgccaaaatt attcgcctga ttgcccgcga cgaagccctg cacctgaccg 1020

gcacccagca tatgctgaat ctgctgcgca gcggcgcgga cgatcctgag atggcggaaa 1080

ttgccgaaga gtgtaagcag gagtgctatg acctgtttgt tcaggcagct caacaggaga 1140

aagactgggc ggattatctg ttccgcgacg gttcgatgat tggtctgaat aaagacattc 1200

tctgccagta cgttgaatac atcaccaata tccgtatgca ggcagtcggt ttggatctgc 1260

cgttccagac gcgctccaac ccgatcccgt ggatcaacac ttggctggtg tctgataacg 1320

tgcaggttgc tccgcaggaa gtggaagtca gttcttatct ggtcgggcag attgactcgg 1380

aagtggacac cgacgatttg agtaacttcc agctctgatg gcccgcgtta ccctgcgcat 1440

cactggcaca caactgctgt gccaggatga acacccttcc cttctggcgg cgctggaatc 1500

ccacaatgtg gcggttgagt accagtgtcg cgaaggttac tgcggctcct gtcgcacacg 1560

<210> 3

<211> 4594

<212> DNA

<213> Salmonella typhimurium

<400> 3

gtgaacgtcg atctggtgcc ggatgcagcg gatacgctcc gggcgcaagg atttcgtcaa 60

ttaccggtgg tgatggcggg cgatttgagc tggtctggct tccgcccgga catgattaac 120

cgtctgcacc cgacacccca cgcggcaaac gcatgagcgc gctcgtctac ttctccagca 180

gctctgaaaa tacgcaccgc tttatgcagc gtctggggct gcctgccacg cgtattccgc 240

tcaatgagcg ggagcgaatt caggtagacg aaccgtacat tctggttgtg ccgtcatacg 300

gcggcggcgg gatggccggt gcggtgccgc gacaggtgat ccgcttttta aatgatgaac 360

acaaccgggc gcgcattcgc ggcgttatcg cctccggtaa tcgcaatttc ggcgatgcct 420

ggggatgcgc tggcgatgtg atagcacaaa aatgcggcgt cccctggctg taccgctttg 480

agctcatggg cacacaacgc gacatcgata atgtccgaaa aggagtaaat gaattttggc 540

aacaactacc ccggagcgcg taatgcagga aaccatggat taccacgccc tgaacgcgat 600

gctgaatctt tacgataaag caggccatat tcagttcgac aaggaccagc aggcgatcga 660

cgccttcttt gccacccacg tccgcccgca ttccgtgacg tttgccagcc agcatgaacg 720

tctggggacg ctggttcggg aagggtatta cgatgacgcc gtcctcgcgc gttacgaccg 780

cgccttcgtc cttcgcctgt tcgagcacgc ccatgccagc ggctttcgct tccagacgtt 840

tcttggcgcc tggaagttct ataccagtta cacgctgaaa accttcgacg gcaaacgtta 900

tctggaacac tttgaagatc gggtgacaat ggtggcgttg acgctggcgc agggtgacga 960

aacgctggcc acccaactga ccgatgaaat gctttctggt cgctttcagc ccgctacccc 1020

gactttttta aattgcggca aacagcagcg tggggaactg gtctcctgct tcctgctccg 1080

tatcgaagac aacatggagt cgatcgggcg ggcggtgaat tcggcgctgc aactctccaa 1140

acgcggcggc ggcgtcgcgt ttttactctc caatctgcgc gaggcgggcg cgccgatcaa 1200

acgcattgag aatcagtctt ccggcgtgat cccggtgatg aaaatgctgg aagacgcgtt 1260

ttcgtatgcc aaccaacttg gcgcgcgcca gggggccggc gcggtttatc tccatgcgca 1320

ccatccggat attctgcgtt ttctggatac caaacgagaa aacgctgacg aaaaaatccg 1380

gatcaaaacg ctctctctcg gcgtggtgat cccggatatc accttccggc tggcgaaaga 1440

aaacgcgcaa atggcgctct tttcgcccta tgacatacaa cgacgctacg gcaaaccgtt 1500

tggcgatatc gccattagcg aacggtacga tgaattaatt gccgatccgc acgtgcgcaa 1560

aacctatatt aacgcccgtg actttttca aacactggcg gagattcagt tcgaatccgg 1620

gtatccctac atcatgtttg aagatacggt aaaccgcgcg aatcccattg ctggtcgcat 1680

taatatgagc aacctgtgct cagaaatttt acaggtcaat agcgcttccc gttacgacga 1740

taaccttgac tatacccaca tcgggcatga catctcctgc aatctcggct cgctgaatat 1800

cgctcacgtc atggattcac cggacattgg ccgtaccgta gaaaccgcta ttcgcggcct 1860

gacggcggtg tcggacatga gccatatacg cagcgtgccc tcaatagccg ccggtaatgc 1920

cgcctctcat gccatcggtc tgggccagat gaatctgcat ggctatctgg cgagggaagg 1980

tattgcctac ggttcgccgg aggcgttgga tttcaccaat ctctattttt acaccattac 2040

ctggcatgcc gtgcatactt caatgcggct agcccgcgaa cgcggcaaaa ccttcgccgg 2100

atttgcgcag tcgcgctatg ccagcggcga ctattttacg cagtatttac aggacgactg 2160

gcaaccgaaa acagcgaaag tcagggcgct atttgcccgc agcggcatta cgctgcccac 2220

acgagaaatg tggctaaagc tgcgcgacga tgtgatgcgc tatggcatct ataaccaaaa 2280
tttgcaggcg gtgccgccga ccggttcgat ttcttacatt aatcatgcga cctccagcat 2340
tcatccgatt gtggccaaaa ttgagattcg caaagagggc aaaaccgggc gtgtgtatta 2400
ccccgcgccg tttatgacca atgaaaacct ggacatgtat caggatgctt acgatatcgg 2460
tccggaaaaa attattgata cctatgccga ggccacgcgc cacgtcgatc aagggctgtc 2520
gctcaccctg tttttccccg ataccgccac gacccgcgat atcaacaagg cgcagatcta 2580
tgcctggcga aaaggtatta agtccctgta ttacatccgg cttcgccagt tggcgctgga 2640
aggtactgaa attgaaggct gcgtatcctg cgcgctataa ggaaagccat atgaaattat 2700
ctcgtattag cgccatcaac tggaacaaga tccaggacga caaagatctg gaggtatgga 2760
accggctgac cagtaacttc tggctgccgg aaaaagtgcc gttatcgaat gatattccgg 2820
cctggcagac gctgagcgcc gccgaacagc agctcaccat tcgcgtgttt acgggactta 2880
cgctgctcga cactatccag aacatcgcag gcgcgccgtc gttaatggca gatgccatca 2940
cgccgcatga agaggcagtg ctgtcgaaca tcagctttat ggaagcggta cacgcccgct 3000
cttacagttc tattttctcc acgctgtgcc agacgaaaga ggttgatgcc gcctacgcct 3060
ggagcgaaga aaacccaccg cttcagcgta aggcgcagat tattttagct cattacgtca 3120
gcgatgaacc gctaaagaaa aagattgcca gcgtcttttt agagtctttt ctgttctatt 3180
ccggcttctg gttgccgatg tatttctcca gccgcggtaa gctcacgaac actgccgacc 3240
tgattcgttt aatcattcgc gatgaagcgg ttcacggtta ttatattggc tataagtatc 3300
agatagcgct acaaaaacta tcggcaatcg agcgtgaaga gttaaagctt ttcgcgctgg 3360
atttgttgat ggaactgtac gacaacgaaa tccgctacac agaagcgtta tatgcggaaa 3420
ccggctgggt taacgacgtc aaagccttct tgtgctacaa cgccaataaa gccttaatga 3480
acctgggtta tgaggcgtta tttccgccgg agatggcaga cgtgaatccc gcaatccttg 3540
ccgcgctctc gccgaatgcc gacgaaaacc atgatttctt ttccggctca ggttcatctt 3600

atgtgatggg gaaaacagtc gaaaccgaag acgaagactg gaatttttaa ccttacgggc 3660

atgggaaata acgttacatt tcccatgcct ttatttcaag caatagggag tcaaatcgcg 3720

caaatattac aacatgtcct acactcaata cgagtgacat tattcacctg gattccccca 3780

attcaggtgg atttttgctg gttgttccaa aaaatatctc ttcctcccca ttcgcgttca 3840

gcccttatat catgggaaat cacagccgat agcacctcgc aatattcatg ccagaagcaa 3900

attcagggtt gtctcagatt ctgagtatgt tagggtagaa aaaggtaact atttctatca 3960

ggtaacatat cgacataagt aaataacagg aatcattcta ttgcatggca attaaattag 4020

aagtgaagaa tctgtataaa atatttggag agcatccgca gcgtgccttc aaatatattg 4080

aaaagggact atcgaaagag caaatactgg aaaaaacggg gctatcgctt ggcgttaaag 4140

acgccagtct ggccattgaa gaaggcgaga tatttgtcat catgggatta tccggctcgg 4200

gtaaatccac aatggtacgc cttctcaatc gcctgattga acccacccgc ggacaggtac 4260

tgattgacgg cgttgatatt gccaaaatat cagacgctga gcttcgcgag gtgcgcagga 4320

aaaagattgc gatggtcttc cagtcatttg cgctcatgcc gcatatgacc gtgctggata 4380

atacggcatt cggtatggaa ttagcgggca tcgcggcgca agagcgtcgc gaaaaagcgc 4440

tggacgcctt gcgtcaggtg gggcttgaga attacgctca cgcctacccg gatgaacttt 4500

ccggtgggat gcgtcagcgt gttgggcttg cccgcgcgct ggcaatcaac cctgatatct 4560

tattaatgga tgaagcgttt tccgccctcg atcc 4594

<210> 4

<211> 1033

<212> DNA

<213> Lactococcus lactis

<400> 4

gaattcttat tttccctagc tttggattta ttctcacttc ctatgatctt ttattctcga 60
ttattatttt tgctttggca attattatca tttttcgaca taaaacaaac ctcaaaagaa 120
tcaaaaatca ttgtgaatcc cttgtcccct ttggtttaaa cttatcgaga caaaaagaaa 180
aatagcacaa tatattgtgt tgtttttctt tttttacata atttaacact atatctagta 240
tctttaattt gactagatat ttttttttacg ctaaataaga ctataaaaac tcgagaaaaa 300
gtcaaggact ttttactccc gtctaaaaaa tatattggcc caaaaggaga tttaaaatgg 360
ttacagttta ttctaaaaac aattgtatgc aatgcaaaat ggtcaaaaaa tggctttctg 420
aacacgaaat tgcatttaac gaaatcaata ttgatgaaca gcctgaattt gtcgaaaaag 480
taattgaaat gggttttcga gctgctcctg taatcacaaa agatgatttc gccttttctg 540
gtttccgtcc ttctgaatta gcaaagttgg cttaatatga aacttgctta tttcagtgtg 600
actggacaaa cgcgtcgttt tgtttctaaa acagacttgc cgaatgtcga aattacacct 660
gacgatgatt tagagatgga cgagcctttc cttttgataa ctccctctta tgctgaagaa 720
tcaccaaccg tttctaaatc aatagacgtt atggactcgg tttttgactt tatggcttat 780
aatgataatt ataaacattg tcgtggaatt atcggcactg gaaatcgtaa ttttgctggc 840
atctatattt ttaccgctaa agaagtttca gcaaaatatc aaattccact tttatatgat 900
tttgagttta atggtacgcc agctgatgtt gctgctgttg aaaaactcgc tgcacagctt 960
gatcaaggag cgaaagtcac ctttaaaaat ccgctgtgat tttttatggc ttcaccctat 1020
ttgagtgaag ctt 1033

<210> 5

<211> 3811

<212> DNA

<213> Escherichia coli

<400> 5

cagctgtact ggcataacga catttatact gtcgtataaa attcgactgg caaatctggc 60

actctctccg gccaggtgaa ccagtcgttt ttttttgaat tttataagag ctataaaaaa 120

cggtgcgaac gctgttttct taagcacttt tccgcacaac ttatcttcat tcgtgctgtg 180

gactgcaggc tttaatgata agatttgtgc gctaaatacg tttgaatatg atcgggatgg 240

caataacgtg agtggaatac tgacgcgctg gcgacagttt ggtaaacgct acttctggcc 300

gcatctctta ttagggatgg ttgcggcgag tttaggtttg cctgcgctca gcaacgccgc 360

cgaaccaaac gcgcccgcaa aagcgacaac ccgcaaccac gagccttcag ccaaagttaa 420

ctttggtcaa ttggccttgc tggaagcgaa cacacgccgc ccgaattcga actattccgt 480

tgattactgg catcaacatg ccattcgcac ggtaatccgt catctttctt tcgcaatggc 540

accgcaaaca ctgcccgttg ctgaagaatc tttgcctctt caggcgcaac atcttgcatt 600

actggatacg ctcagcgcgc tgctgaccca ggaaggcacg ccgtctgaaa agggttatcg 660

cattgattat gcgcatttta ccccacaagc aaaattcagc acgcccgtct ggataagcca 720

ggcgcaaggc atccgtgctg gccctcaacg cctcacctaa caacaataaa cctttacttc 780

attttattaa ctccgcaacg cggggcgttt gagattttat tatgctaatc aaattgttaa 840

ctaaagtttt cggtagtcgt aacgatcgca ccctgcgccg gatgcgcaaa gtggtcaaca 900

tcatcaatgc catggaaccg gagatggaaa aactctccga cgaagaactg aaagggaaaa 960

ccgcagagtt tcgtgcacgt ctggaaaaag gcgaagtgct ggaaaatctg atcccggaag 1020

ctttcgccgt ggtacgtgag gcaagtaagc gcgtctttgg tatgcgtcac ttcgacgttc 1080

agttactcgg cggtatggtt cttaacgaac gctgcatcgc cgaaatgcgt accggtgaag 1140

gaaaaaccct gaccgcaacc ctccctcctt acctgaacgc actaaccggt aaaggcgtgc 1200

acgtagttac cgtcaacgac tacctggcgc aacgtgacgc cgaaaacaac cgtccgctgt 1260

ttgaattcct tggcctgact gtcggtatca acctgccggg catgccagca ccggcaaagc 1320

gcgaagctta cgcagctgac atcacttacg gtaccaacaa cgaatacggc tttgactacc 1380

tgcgcgacaa catggcgttc agccctgaag aacgtgtaca gcgtaaactg cactatgcgc 1440

tggtggacga agtggactcc atcctgatcg atgaagcgcg tacaccgctg atcatttccg 1500

gcccggcaga agacagctcg gaaatgtata aacgcgtgaa taaaattatt ccgcacctga 1560

tccgtcagga aaaagaagac tccgaaacct tccagggcga aggccacttc tcggtggacg 1620

aaaaatctcg ccaggtgaac ctgaccgaac gtggtctggt gctgattgaa gaactgctgg 1680

tgaaagaggg catcatggat gaaggggagt ctctgtactc tccggccaac atcatgctga 1740

tgcaccacgt aacggcggcg ctgcgcgctc atgcgctgtt tacccgtgac gtcgactaca 1800

tcgttaaaga tggtgaagtt atcatcgttg acgaacacac cggtcgtacc atgcagggcc 1860

gtcgctggtc cgatggtctg caccaggctg tggaagcgaa agaaggtgtg cagatccaga 1920

acgaaaacca aacgctggct tcgatcacct tccagaacta cttccgtctg tatgaaaaac 1980

tggcggggat gaccggtact gctgataccg aagctttcga atttagctca atctacaagc 2040

tggataccgt cgttgttccg accaaccgtc caatgattcg taaagatctg ccggacctgg 2100

tctacatgac tgaagcggaa aaaattcagg cgatcattga agatatcaaa gaacgtactg 2160

cgaaaggcca gccggtgctg gtgggtacta tctccatcga aaaatcggag ctggtgtcaa 2220

acgaactgac caaagccggt attaagcaca acgtcctgaa cgccaaattc cacgccaacg 2280

aagcggcgat tgttgctcag gcaggttatc cggctgcggt gactatcgcg accaatatgg 2340

cgggtcgtgg tacagatatt gtgctcggtg gtagctggca ggcagaagtt gccgcgctgg 2400

aaaatccgac cgcagagcaa attgaaaaaa ttaaagccga ctggcaggta cgtcacgatg 2460

cggtactgga agcaggtggc ctgcatatca tcggtaccga gcgtcacgaa tcccgtcgta 2520

tcgataacca gttgcgcggt cgttctggtc gtcaggggga tgctggttct tcccgtttct 2580

acctgtcgat ggaagatgcg ctgatgcgta tttttgcttc cgaccgagta tccggcatga 2640

tgcgtaaact gggtatgaag ccaggcgaag ccattgaaca cccgtgggtg actaaagcga 2700

ttgccaacgc ccagcgtaaa gttgaaagcc gtaacttcga cattcgtaag caactgctgg 2760

aatatgatga cgtggctaac gatcagcgtc gcgccattta ctcccagcgt aacgaactgt 2820

tggatgtcag cgatgtgagc gaaaccatta acagcattcg tgaagatgtg ttcaaagcga 2880

ccattgatgc ctacattcca ccacagtcgc tggaagaaat gtgggatatt ccggggctgc 2940

aggaacgtct gaagaacgat ttcgacctcg atttgccaat tgccgagtgg ctggataaag 3000

aaccagaact gcatgaagag acgctgcgtg acggcattct ggcgcagtcc atcgaagtgt 3060

atcagcgtaa agaagaagtg gttggtgctg agatgatgcg tcacttcgag aaaggcgtca 3120

tgctgcaaac gcttgactcc ctgtggaaag agcacctggc agcgatggac tatctgcgtc 3180

agggtatcca cctgcgtggc tacgcacaga aagatccgaa gcaggaatac aaacgtgaat 3240

cgttctccat gtttgcagcg atgctggagt cgttgaaata tgaagttatc agtacgctga 3300

gcaaagttca ggtacgtatg cctgaagagg ttgaggagct ggaacaacag cgtcgtatgg 3360

aagccgagcg tttagcgcaa atgcagcagc ttagccatca ggatgacgac tctgcagccg 3420

cagctgcact ggcggcgcaa accggagagc gcaaagtagg acgtaacgat ccttgcccgt 3480

gcggttctgg taaaaaatac aagcagtgcc atggccgcct gcaataaaag ctaactgttg 3540

aagtaaaagg cgcaggattc tgcgcctttt ttataggttt aagacaatga aaaagctgca 3600

aattgcggta ggtattattc gcaacgagaa caatgaaatc tttataacgc gtcgcgcagc 3660

agatgcgcac atggcgaata aactggagtt tcccggcggt aaaattgaaa tgggtgaaac 3720

gccggaacag gcggtggtgc gtgaacttca ggaagaagtc gggattaccc cccaacattt 3780

ttcgctattt gaaaaactgg aatatgaatt c 3811

<210> 6

<211> 4045

<212> DNA

<213> Mycobacterium bovis

<400> 6

gatctacggc agaactcgtc gcttggagcg ttcgaccgac catctacctg ttcgacgtcg 60

aactcgacca ctgaacgtaa tcgccgccag cgcaagtcct gtcagcgcgt ggagatcacc 120

gcgcgtgggc gagggccggt ggtgcgaggt gaggcctgcg ccgacagctt ctatgccgcg 180

cttgaatcag cggtcgtcaa actggagagc gtgcgccgcg gtaaggatcg ccgcaaggtg 240

cactacggcg acaaaacccc ggtttcgctg gccgaggcga ccgcggtggt gccagcgccg 300

gagaacggct tcaacaccag accagccgag gcacacgatc acgacggtgc cgtcgtcgag 360

cgggagcctg ggcggatcgt tcgcaccaaa gaacacccgg ccaagccgat gtcggtcgat 420

gacgcgctct accagatgga gctggttgga cacgacttct tcttgttcta cgacaaggac 480

accgaacggc cgtcggtggt ctaccgccgg cacgcctacg actacggctt gatccgtctg 540

gcgtgatcgg cggcgcgcgc cgctcgtcac ctaccatggg agtcgcctta tctaaagact 600

cctacacatg cggggacata gctgtgctgt cgaagttgct gcgccttggc gaaggtcgca 660

tggtcaagcg cctcaagaag gtggcggact atgtcggcac tttgtccgac gatgtcgaga 720

aactcaccga cgccgagctg agggcgaaaa ccgacgagtt caagcggcgg ctggccgacc 780

agaaaaaccc agaaaccctc gacgacctgt tgcccgaggc cttcgccgtg gcccgcgagg 840

ccgcctggcg ggtgctggac cagcggccgt tcgacgtgca ggtgatgggt gcggccgccc 900

tgcacctggg caacgttgcc gagatgaaga ccggtgaagg caagaccctg acctgtgtgt 960

tgcccgctta cctcaatgcg ctggccggca acggcgtgca catcgtcacc gtcaacgact 1020

acctggctaa acgcgacagt gagtggatgg gccgcgtgca ccgcttcctc gggcttcagg 1080

tcggggtgat tttcgccacc atgacacccg atgaacgccg ggtggcctat aacgccgaca 1140

tcacctacgg caccaataac gagtttgggt tcgactacct gcgcgacaac atggcgcact 1200

cactggatga tctggtgcag cgcgggcacc attacgccat tgtcgacgag gtcgattcca 1260

tcctgatcga cgaggcccgc accccgctga tcatctccgg tcccgccgac ggcctccaac 1320

tggtacaccg agttcgccgg ttggcgccgc tgatggaaaa ggacgtccac tacgaggtcg 1380

atctacgcaa acgcaccgtc ggcgtgcacg agaagggtgt ggaattcgtc gaagaccagc 1440

tcggcatcga caacctgtac gaggccgcca actcgccgtt ggtcagctat ctcaacaacg 1500

ctctgaaggc caaagagctg ttcagccgcg acaaggacta catcgtccgc gatggtgagg 1560

tgctcatcgt cgacgagttc accggccggg tgctgatcgg ccgccgctac aacgagggca 1620

tcgaccaggc catcgaggcc aaggagcacg tcgagatcaa ggccgagaac cagacgctgg 1680

ccaccatcac gctgcagaac tacttccggc tctacgacaa gctcgccggc atgaccggca 1740

ccgcccagac ggaggcggcc gagctgcacg agatctacaa gctgggcgtg gtcagcatcc 1800

cgaccaacat gccgatgatc cgtgaagacc agtccgacct gatctacaag accgaggagg 1860

ccaagtacat cgcggtggtc gacgacgtcg ccgagcgcta cgcgaaggga cagccggtgc 1920

tgatcggcac caccagcgtg gagcgctcgg agtatctgtc gcggcagttc accaagcggc 1980

gcatcccgca caatgtgctc aacgccaagt accacgagca agaggcgacc atcatcgcgg 2040

tggcgggccg ccgcggcggc gtcaccgtcg ccaccaacat ggccggtcgc ggcaccgaca 2100

ttgtgctggg cggcaacgtc gactttctca ccgatcagcg gctgcgcgaa cggcctggat 2160

ccggtggaga cgcccgagga gtacgaggcg gcctggcact ccgaactgcc catcgtcaaa 2220

gaggaagcca gcaaggaggc caaggaagta atcgaggccg gcggctgtac gtgctgggca 2280

ccgagcggcc acgagtcgcg gcggatcgac aaccagttgc gtggccggtc cggccgccag 2340

gggaccccgg ggagtcgcgc ttctatttgt cgctgggtga cgagctgatg cgccgcttca 2400

atggcgcggc cttggagacc ttgttgacca ggctgaacct gcccgacgac gtgccgatcg 2460

aagccaagat ggtcacccgg gccatcaaga gcgcccagac ccaggtcgag cagcagaact 2520

ttgaggtccg caagaacgtc ctcaaatacg acgaggtgat gaaccagcag cgcaaggtca 2580

tctacgccga gcgccggcgc atcctcgaag gcgaaaacct caaggaccag gcgctggaca 2640

tggtccgcga tgtcatcacc gcctacgtcg acggcgcgac cggcgaaggc tatgccgaag 2700

attgggatct ggacgcgttg tggacggcac tcaaaaccct ctatccggag gggatcaccg 2760

ccgactcgct gacccgcaag gaccacgaat tcgagcgcga cgatctcacc cgcgaggagt 2820

tgctggaggc actactcaag gacgccgaac gtgcctatgc cgcacgggaa gccgaactcg 2880

aggaaatcgc cggcgagggt gcgatgcgcc agctggaacg caacgtgctg ctcaacgtca 2940

tagaccgtaa gtggcgtgaa cacctctacg agatggacta cctcaaggag ggtatcgggc 3000

tgcgcgcgat ggcgcacggc gatccgttgg tcgagtacca gcgtgagggc tacgacatgt 3060

tcatggccat gctcgacggc atgaaagagg aatcggtcgg cttcctgttc aacgtcaccg 3120

tggaggcggt ccccgccccg ccggttgccc cggctgccga acccgcagag cttgccgaat 3180

tcgccgccgc ggccgcagcc gcgggcagca acgcagcgcg gtcgatggtg gcgcgcgcga 3240

aagagctcca agtgcattac gcgccaaggg tgttgccagc gagtcgcccg ctttgaccta 3300

ttccggtccc gcggaggatg gctcggctca ggtgcagcgc aacggcggtg gagcccacaa 3360

gacgccggcc ggagtgccgg ccggtgctag ccggcgcgag cggcgcgaac gcgcccgccg 3420

acaaggccgc ggcgccaagc cgccgaaatc ggtcaagaag cgttagcgcg taggttgcag 3480

atgggtgtat cggtttctca gttcccagaa gtcacttccc ggcacacccc ggccccggcg 3540

cgcatgcaca tttcgttgca cggcgggcaa ggggttcgct aatctcaccc gttcgtcgac 3600

cttcgtcggc gtcggttctg ctggtagcgg ggttcggcgc tttcctggcg tttctcgact 3660

cgacaatcgt caacatcgcg ttcccggata tccagcgttc cttcccgtcc tacgacatcg 3720

ggagcctgtc ctggattctg aacggctata acatgctctt cgccgccttc atggttgcgg 3780

ccggcaggtt ggccgatttg ctgggccgca gacgacattc ctgtccggtg tgctggtgtt 3840

caccattgcg tccgggctgt gcgccgtcgc cggcagtgtc gagcagttgg tggcgttccg 3900

ggtgctgcag ggcatcgggg ctgcgatact cgtgcctcgt tcgctcgcac tggtcgttga 3960

gggcttcgac cgggccgccg cgcgcacgct atcggcctgt ggggtgcggc ggcagcgatc 4020

cactagttct agagcggcgc accgc 4045

<210> 7

<211> 1433

<212> DNA

<213> Mycobacterium tuberculosis

<400> 7

tcaaacacca gaccagaagg aggcaacacg atcacggacg gtgccgttcg tcgagcggga 60

gcctggggcg gatcgttcgc accaaagaac aacccggcca cgccgatgtc ggtcgatgac 120

gcgctctacc agatggagct ggttggacac gacttcttct tgttctacga caaggacacc 180

gaacggccgt cggtggtcta ccgccggcac gcctacgact acggcttgat ccgtctggcg 240

tcatcggcgg cgcgcgccgc gtcgtcacct accatgggag tcgccttatc taaagactcc 300

tacacatgcg gggacatagc tgtgctgtcg aagttgctgc gccttggcga aggtcgcatg 360

gtcaagcgcc tcaagaaggt ggccgactat gtcggcactt tgtccgacga tgtcgagaaa 420

ctcaccgacg ccgagctgag ggcgaaaacc gacgagttca agcaggctgg ccgaccagaa 480

aaacccagaa accctcgacg acctgttgcc cgaggccttc accgtgcccc gcgagacccg 540

cctgccgggt gctggaccaa cgaccgttcg acgtgcaggt gatgggtacg accgccctgc 600
acctgggcga cgttgccgag atgtagaccg gtgaaggcaa gaccctgacc tgtgttttac 660
ccgcttacct caatgccctg gccgccaacg gcgtgcacgt agttaccgtc aacgactacc 720
tggctaaacg cgacagtgag tggatgggcc gcgtgcaccg cttcctcggg cttcaggtcg 780
gggtgatttt ggccaccatg acacccgatg aacgccgggt ggcctataac gccgacatca 840
cctacggcac caataacgag tttgggttcg actacctgcg cgacaacatg gcgcactcac 900
tggatgatct ggtgcagcgc gggcaccatt acgccattgt cgacgaaggt cgattccatc 960
ctgatcgacg agggcggggc ccccccccca tctccgcccg gggcgcccgc ctccaactgg 1020
ttcaccgagt tcgcccggtt ggcgtgccgc ggctggtttt ggacgtccac tacgaggtcg 1080
atctacgcaa acgcaccgtc ggcgtgcacg agaagggtgt ggaattcgtc gaagaccagc 1140
tcggcatcga caacctgtac gagaccgcca actcgccgtt ggtcagctat ctcaacaacg 1200
ctctgaaggc caaagagctg ttcagccgcg acaaggacta catcgtccgc gatggtgagg 1260
tgctcatcgt cgacgagttc accggccggg tgctgatcgg ccgccgctac aacgagggca 1320
tgcaccaggc catcgaggcc aaggagcacg tcgagatcaa ggccgagaac cagacgctgg 1380
ccaccatcac gctgcagaac tacttccggc tctaggagaa gctcgccggg atg 1433

<210> 8

<211> 3124

<212> DNA

<213> Staphylococcus aureus

<400> 8

tggcttgatt caaactagtg aacaataaat taagtttaaa gcacttgtgt ttttgcacaa 60

gtttttttat actccaaaag caaattatga ctatttcata gttcgataat gtaatttgtt 120

gaatgaaaca tagtgactat gctaatgtta atggatgtat atatttgaat gttaagttaa 180

taatagtatg tcagtctatt gtatagtccg agtcgaaaat cgtaaaatat ttataatata 240

atttattagg aagtataatt gcgtattgag aatatattta ttagtgataa acttgttgac 300

aacagaatgt gaatgaagta tgtcataaat atatttatat tgattctaca aatgagtaaa 360

taagtataat tttctaacta taaatgataa gatatattgt tgtaggccaa acagtttttt 420

agctaaagga gcgaacgaaa tgggattttt atcaaaaatt cttgatggca ataataaaga 480

aattaaacag ttaggtaaac ttgctgataa agtaatcgct ttagaagaaa aaacggcaat 540

tttaactgat gaagaaattc gtaataaaac gaaacaattc caaacagaat tagctgacat 600

tgataatgtc aaaaagcaaa atgattattt acataaaatt ttaccagaag catatgcact 660

tgttagagaa ggctctaaac gtgtattcaa tatgacacca tataaagttc aaattatggg 720

tggtattgca attcataaag gtgatatcgc tgagatgaga acaggtgaag gtaaaacatt 780

aacagcgaca atgccaacat acttaaatgc attagctggt agaggtgttc acgttattac 840

agtcaatgaa tacttatcaa gtgttcaaag tgaagaaatg gctgagttat ataacttctt 900

aggtttgact gtcggattaa acttaaacag taagacgaca gaggaaaaac gtgaagcata 960

cgcacaagac attacttaca gtactaataa tgagctaggt tttgattact tacgagataa 1020

catggtgaat tattctgaag atagggtaat gcgtccatta cattttgcaa tcattgatga 1080

ggtggactca attttaatcg acgaggcacg tacgccatta attatttctg gtgaagctga 1140

aaagtcaacg tcactttata cacaagcaaa tgtttttgcg aaaatgttaa aacaggacga 1200

tgattataaa tacgatgaaa aaacgaaagc tgtacattta acagaacaag gtgcggataa 1260

agctgaacgt atgttcaaag ttgaaaactt atatgatgta caaaatgttg atgttattag 1320

tcatatcaac acagctttac gtgcgcacgt tacattacaa cgtgacgtag actatatggt 1380

tgttgatggc gaagtattaa ttgtcgatca atttacagga cgtacaatgc caggccgtcg 1440

tttctcggaa ggtttacacc aagctattga agcgaaggaa ggcgttcaaa ttcaaaatga 1500

atctaaaact atggcgtcta ttacattcca aaactatttc agaatgtaca ataaacttgc 1560

gggtatgaca ggtacagcta aaactgaaga agaagaattt agaaatattt ataacatgac 1620

agtaactcaa attccgacaa ataaacctgt gcaacgtaac gataagtctg atttaattta 1680

cattagccaa aaaggtaaat ttgatgcagt agtagaagat gttgttgaaa aacacaaggc 1740

agggcaacca gtgctattag gtactgttgc agttgagact tctgaatata tttcaaattt 1800

acttaaaaaa cgtggtatcc gtcatgatgt gttaaatgcg aaaaatcatg aacgtgaagc 1860

tgaaattgtt gcaggcgctg gacaaaaagg tgccgttact attgccacta acatggctgg 1920

tcggggtaca gatatcaaat taggtgaagg cgtagaggaa ttaggcggtt tagcagtaat 1980

aggtacagag cgacatgaat ctcgtcgtat tgatgaccag ttacgtggtc gttctggacg 2040

tcaaggtgat aaaggggata gtcgcttcta tttatcatta caagatgaat taatgattcg 2100

ttttggttct gaacgtttac agaaaatgat gagccgacta ggtttagatg actctacacc 2160

aattgaatca aaaatggtat caagagctgt tgaatcagca caaaaacgtg tagaaggtaa 2220

taacttcgac gcgcgtaaac gtatcttaga atacgatgaa gtattacgta aacaacgtga 2280

aattatctat aacgaaagaa atagtattat tgatgaagaa gacagctctc aagttgtaga 2340

tgcaatgcta cgttcaacgt tacaacgtag tatcaattac tatattaata cagcagatga 2400

cgagcctgaa tatcaaccat tcatcgacta cattaatgac atcttcttac aagaaggtga 2460

cattacagag gatgatatca aaggtaaaga tgctgaagat attttcgaag tcgtttgggc 2520

taagattgaa gcagcatatc aaagtcaaaa agatatctta gaagaacaaa tgaatgagtt 2580

tgagcgtatg attttacttc gttctattga tagccattgg actgatcata tcgacacaat 2640

ggatcaatta cgtcaaggta ttcacttacg ttcttatgca caacaaaatc cattacgtga 2700

ctatcaaaat gaaggtcatg aattatttga tatcatgatg caaaatattg aagaagatac 2760

ttgtaaattc attttaaaat ctgtagtaca agttgaagat aatattgaac gtgaaaaaac 2820

aacagagttt ggtgaagcga agcacgtttc agctgaagat ggtaaagaaa aagtgaaacc 2880

gaaaccaatc gttaaaggcg atcaagttgg tcgtaacgat gattgtccat gtggtagtgg 2940

taaaaaattc aaaaattgcc atggaaaata aatgatataa aataactcct tccaattaaa 3000

cacctatagt ttgtgttatg ggaggagtct ttttatttta caagcgttaa atactttaaa 3060

aaatgtgaag aagttgttaa acgttgttat gtacttagtt ttaaaaaatc ggtttaggca 3120

tatg 3124

<210> 9

<211> 3589

<212> DNA

<213> Staphylococcus carnosus

<400> 9

cttgaacgtt acttcactaa tgtgccgaat gtgaatgcac atgtaaaagt gaaaacttat 60

gcaaattcta gcacaaaatc gaagttacaa ttccgcttaa tgacgtgaca cttcgtgcag 120

aagaaagaaa cgatgattta tgctggaatt gacaagatca ctaacaaatt agaatgtcaa 180

gttcgtaaat acaaaacacg tgtcaatcgt aagaaacgta aagaaagcga acatgaacca 240

ttcccagcaa ctccggaaac tccgccggaa acagctgttg atcatgataa agatgatgaa 300

attgaaatca tccgttctaa acaattcagc ttgaaaccaa tggattctga agaagcggta 360

ttacaaatgg atttacttgg tactgatttc ttcatcttca atgaccgtga aactgatggt 420

acaagcattg tttaccgccg taaagacgga aaatatggtt tgattgaaac tgttgaaaaa 480

ctaatatgtg atatttgaaa gggctcttgc tgcattttct gctgcaagag tttctttttt 540

tgagaaagcc cttattaaga tttgattaat aaaaatacaa ttgattgatt tacacggggt 600
gtccatgtca aaataagagg gatgtattaa gttcataatt gtaatgtgag ctccgatgag 660
tgagcggcat atgattatga tatccatgtg gcacatgatg ttaacaaaaa gagaatgaaa 720
ctgtgagaag tacatcttga taaacacaac taggcagttt attaaaaaat aatgaacagt 780
atcctatgag tttttaagta taaattaagc catataaatg gtaagataaa ttgttgtaag 840
ccaaacagtt tttataccaa aggagcgaac agaatgggtt ttttaacaaa aattgttgac 900
ggcaataaga gagaaatcaa acgcctaagt aagcaagctg acaaagtaat ctcattagaa 960
gaagaaatgt caattcttac tgatgaagaa attagaaata aaacaaaagc attccaagaa 1020
agattgcaag cagaagaaca tgtaagcaaa caagataaaa ttttagaaga aatattacct 1080
gaagcatttg cgcttgtccg tgaaggagct aaacgtgtat ttaatatgac accttatcca 1140
gttcaaatca tgggtggtat cgccattcat aatggtgaca tttcagaaat gagaacaggt 1200
gaaggtaaaa cattaactgc aacgatgccg acttatttaa acgccttagc agcacgtggt 1260
gtgcatgtta ttacagtcaa tgaatacttg gcaagttctc aaagagaaga aatggccgag 1320
ttatataatt tccttggttt atcagtcgga ttgaacttga acagcttatc aacagaacaa 1380
aagcgtgaag cttataatgc agatattacg tatagtacaa ataatgaatt aggcttcgac 1440
tatttacgcg ataacatggt gaattattca gaagaacgtg ttatgcgtcc gcttcatttc 1500
gctatcattg atgaggtcga ctctatttta atcgatgaag cgcgtacacc attgattatt 1560
tcaggggaag ctgaaaaatc aacatctctt tatacacaag caaatgtttt cgctaaaatg 1620
ttaaaagcag aagatgatta taattatgat gaaaaaacaa aatcagtaca attaacagat 1680
caaggtgctg ataaagctga acgtatgttc aagttagata acttatatga tttgaaaaac 1740
gttgatatta tcacgcatat caatacagca ttacgtgcta actatacatt gcaacgcgat 1800
gtagattaca tggttgtaga tggagaagta ttgattgtcg accaatttac aggtcgaaca 1860

atgccaggtc gtcgattctc tgaaggactt caccaagcga ttgaggctaa agaaggggtt 1920
caaattcaaa atgaatctaa aacaatggct tctatcacat tccaaaacta cttccgtatg 1980
tataataaat tagccggtat gacaggtact gctaaaacag aggaagaaga attccgtaac 2040
atttataata tgacagttac acaaattcca acgaaccgtc ctgttcaacg tgaagataga 2100
cctgacttga ttttcatcag ccaaaaaggc aagttcgatg ctgttgttga agatgttgtt 2160
gaaaaacata aaaaaggcca accaattctt ttaggtactg tagcggttga aacaagtgaa 2220
tacatttcac aactattgaa aaaacgcggt gtgcgtcatg atgtcttaaa cgctaaaaac 2280
catgaacgcg aagctgaaat cgtatctaca gcaggtcaaa aaggtgcagt cacaatcgca 2340
acaaacatgg ctggtcgtgg taccgatatt aaattaggcg aaggtgttga agaattaggc 2400
ggccttgctg ttattggtac agaacgtcat gaatcacgcc gtatcgatga tcagttgcgt 2460
ggtcgttctg gacgacaagg tgaccgcgga gaaagccgtt tctatttatc attacaagat 2520
gagttgatgg tacgtttcgg ttctgaacgt ctgcaaaaaa tgatgggccg attaggtatg 2580
gatgactcta caccgattga atcaaaaatg gtatctcgag ctgttgaatc tgcacaaaaa 2640
cgtgttgaag gtaacaactt cgatgcacgt aaacgtatct tagaatacga tgaagtttta 2700
cgtaaacaac gtgaaatcat ttatggtgaa cgtaataata ttatcgattc agaatcaagt 2760
tctgaattag tcattacaat gatacgctct acattagatc gtgcaatcag ttattatgta 2820
aatgaagaat tggaagaaat tgactatgcg ccgtttatta attttgtgga agatgttttc 2880
ttdcacgaag gtgaagtcaa agaagatgaa atcaaaggta aaggtaaaga tcgtgaggat 2940
attttcgata cagtatgggc taaaattgaa aaagcttatg aagcacaaaa agccaatata 3000
cccgaccaat tcaatgaatt cgaacgtatg attttattac gttctattga tggaagatgg 3060
acagaccata tcgatacaat ggatcaatta cgtcaaggta tccatttacg ttcatacggt 3120
caacaaaacc cacttcgcga ctatcaaaat gaagggcacc aactatttga tacaatgatg 3180
gtcaatattg aagaagacgt cagcaaatat atcttgaaat caattatcac agtagatgat 3240

gatattgaac gtgataaagc aaaagaatat caaggacaac atgtatcagc tgaagatgga 3300

aaagaaaaag taaaaccgca accagttgtt aaagataatc acatcggaag aaatgatcct 3360

tgtccatgcg gcagcggtaa aaagtataaa aattgctgcg gtaaatagta agttgtatta 3420

ggaccactgt taaatagctt taagagagat gctcaattga aattgggtta tctttctaag 3480

ggctgtcagc ggtctttttt caatccaaca aaaatatgga tatatgctaa aataatagag 3540

taatctggaa aattaaactg gaattggaga gatatgaaaa tggaattat 3589

<210> 10

<211> 1242

<212> DNA

<213> Bovine herpes virus

<400> 10

cagtcaatgt cgctcttcgt gaccgagcca atggacggaa aggtgcccgc ctcccagatc 60

atgaacctcc tagtgtacgc ctataagaag ggccttaaga cggggctcta ctactgcaag 120

atccgcaagg ccaccaacaa cggcgtcttc acgggcggcg acctcgtgtg ctctgggtgc 180

cacctgtagc gacgcgcgcc gagcgcgatg gccgaggcgg cggacgcggc gaccctcacg 240

cgtaaataca aatactttta cgagaccgag tgccccgacc tagatcactt gcggtcgctc 300

agcgtcgcaa accgctggct ggagaccgag tttcccctag cggacgacgc caaggacgtg 360

gcgcggctca gcggcgccga gctggagttt taccgctttc tgttcgcgtt cctctcggcc 420

gccgatgacc tcgtgaacgt caacctcggg gacctgtccg agctgttcac ccaaaaagac 480

atcctgcatt actatatcga gcaggagtcc atcgaagtgg tgcactcgcg ggtgtacagc 540

gccatacagc tgctgctctt tagaaacgac gcggtggcgc gcgcgggcta cgtagagggc 600

gccctcggcg acccggcggt ccggcgcaag gtggactggc tcgagcggcg cgtggccgcg 660
gcagagtcgg tggccgaaaa gtacgtgctc atgattctaa tcgagggcat ttttttctcc 720
tcctcgtttg cggcgattgc ctacctgcgc acccacaacc ttttcgtcgt gacgtgccaa 780
accaacgacc tcatcagccg cgacgaagcc gtgcacacgg ccgcgtcgtg ctgcatcttc 840
gacaactacc tcggcgggga gcggccgccg ccggcccgca tctacgagct gttccgcgaa 900
gcgtggaaat tgagcgcgag tttatttggt tgcgcgccgc gcggcagtca tatacttgac 960
gtggaggcta tttctgcgta cgtcgagtac agcgcggacc gcctgctcgc tgctatccag 1020
ctgcctcctc tgtttggcac cccgcctcct gggaccgatt ttcctttggc cctgatgact 1080
gccgagaagc acacgaactt ctttgagcgc cgcagcacca actacacagg caccgtaatc 1140
aacgacctgt agggcacccc cgctgccctg ccagagcgcc ccgcctttcc tcctccttct 1200
cacccccacg ccgcgaataa aaaatgttcc atgtcaacga aa 1242

<210> 11

<211> 3518

<212> DNA

<213> Herpes simplex virus 1

<400> 11

tcgagcccgc cgaaacccgc cgcgtctgtt gaaatggcca gccgcccagc cgcatcctct 60
cccgtcgaag cgcgggcccc ggttggggga caggaggccg gcggccccag cgcagccacc 120
cagggggagg ccgccggggc ccctctcgcc cacggccacc acgtgtactg ccagcgagtc 180
aatggcgtga tggtgctttc cgacaagacg cccgggtccg cgtcctaccg catcagcgat 240

agcaactttg tccaatgtgg ttccaactgc accatgatca tcgacggaga cgtggtgcgc 300
gggcgccccc aggacccggg ggccgcggca tcccccgctc ccttcgttgc ggtgacaaac 360
atcggagccg gcagcgacgg cgggaccgcc gtcgtggcat tcgggggaac cccacgtcgc 420
tcggcgggga cgtctaccgg tacccagacg gccgacgtcc ccaccgaggc ccttgggggc 480
ccccctcctc ctccccgctt caccctgggt ggcggctgtt gttcctgtcg cgacacacgg 540
cgccgctctg cggtattcgg gggggagggg gatccagtcg gccccgcgga gttcgtctcg 600
gacgaccggt cgtccgattc cgactcggat gactcggagg acacggactc ggagacgctg 660
tcacacgcct cctcggacgt gtccggcggg gccacgtacg acgacgccct tgactccgat 720
tcgtcatcgg atgactccct gcagatagat ggccccgtgt gtcgcccgtg gagcaatgac 780
accgcgcccc tggatgtttg ccccgggacc cccggcccgg gcgccgacgc cggtggtccc 840
tcagcggtag acccacacgc gccgacgcca gaggccggcg ctggtcttgc ggccgatccc 900
gccgtggccc gggaagacgc ggaggggctt tcggaccccc ggccacgtct gggaacgggc 960
acggcctacc ccgtccccct ggaactcacg cccgagaacg cggaggccgt ggcgcgcttt 1020
ctgggagatg ccgtgaaccg cgaacccgcg ctcatgctgg agtacttttg ccggtgcgcc 1080
cgcgaggaaa ccaagcgtgt ccccccagg acattcggca gcccccctcg cctcacggag 1140
gacgactttg ggcttctcaa ctacgcgctc gtggagatgc agcgcctgtg tctggacgtt 1200
cctccggtcc cgccgaacgc atacatgccc tattatctca gggagtatgt gacgcggctg 1260
gtcaacgggt tcaagccgct ggtgagccgg tccgctcgcc tttaccgcat cctgggggtt 1320
ctggtgcacc tgcggatccg gacccgggag gcctcctttg aggagtggct gcgatccaag 1380
gaagtggccc tggattttgg cctgacggaa aggcttcgcg agcacgaagc ccagctggtg 1440
atcctggccc aggctctgga ccattacgac tgtctgatcc acagcacacc gcacacgctg 1500
gtcgagcggg ggctgcaatc ggccctgaag tatgaggagt tttacctaaa gcgttttggc 1560
gggcactaca tggagtccgt cttccagatg tacacccgca tcgccggctt tttggcctgc 1620

cgggccacgc gcggcatgcg ccacatcgcc ctggggcgag aggggtcgtg gtgggaaatg 1680

ttcaagttct ttttccaccg cctctacgac caccagatcg taccgtcgac ccccgccatg 1740

ctgaacctgg ggacccgcaa ctactacacc tccagctgct acctggtaaa cccccaggcc 1800

accacaaaca aggcgaccct gcgggccatc accagcaacg tcagtgccat cctcgcccgc 1860

aacgggggca tcgggctatg cgtgcaggcg tttaacgact ccggccccgg gaccgccagc 1920

gtcatgcccg ccctcaaggt ccttgactcg ctggtggcgg cgcacaacaa agagagcgcg 1980

cgtccgaccg gcgcgtgcgt gtacctggag ccgtggcaca ccgacgtgcg ggccgtgctc 2040

cggatgaagg gggtcctcgc cggcgaagag gcccagcgct gcgacaatat cttcagcgcc 2100

ctctggatgc cagacctgtt tttcaagcgc ctgattcgcc acctggacgg cgagaagaac 2160

gtcacatgga ccctgttcga ccgggacacc agcatgtcgc tcgccgactt tcacggggag 2220

gagttcgaga agctctacca gcacctcgag gtcatggggt tcggcgagca gatacccatc 2280

caggagctgg cctatggcat tgtgcgcagt gcggccacga ccgggagccc cttcgtcatg 2340

ttcaaagacg cggtgaaccg ccactacatc tacgacaccc agggggcggc catcgccggc 2400

tccaacctct gcaccgagat cgtccatccg gcctccaagc gatccagtgg ggtctgcaac 2460

ctgggaagcg tgaatctggc ccgatgcgtc tccaggcaga cgtttgactt tgggcggctc 2520

cgcgacgccg tgcaggcgtg cgtgctgatg gtgaacatca tgatcgacag cacgctacaa 2580

cccacgcccc agtgcacccg cggcaacgac aacctgcggt ccatgggaat cggcatgcag 2640

ggcctgcaca cggcctgcct gaagctgggg ctggatctgg agtctgccga atttcaggac 2700

ctgaacaaac acatcgccga ggtgatgctg ctgtcggcga tgaagaccag caacgcgctg 2760

tgcgttcgcg ggcccgtccc cttcaaccac tttaagcgca gcatgtatcg cgccggccgc 2820

tttcactggg agcgctttcc ggacgcccgg ccgcggtacg agggcgagtg ggagatgcta 2880

cgccagagca tgatgaaaca cggcctgcgc aacagccagt ttgtcgcgct gatgcccacc 2940

gccgcctcgg cgcagatctc ggacgtcagc gagggctttg ccccccctgtt caccaacctg 3000

ttcagcaagg tgacccggga cggcgagacg ctgcgcccca acacgctcct gctaaaggaa 3060
ctggaacgca cgtttagcgg gaagcgcctc ctggaggtga tggacagtct cgacgccaag 3120
cagtggtccg tgccgcaggc gctcccgtgc ctggagccca cccacccccct ccggcgattc 3180
aagaccgcgt ttgactacga ccagaagttg ctgatcgacc tgtgtgcgga ccgcgccccc 3240
tacgtcgacc atagccaatc catgaccctg tatgtcacgg agaaggcgga cgggaccctc 3300
ccagcctcca ccctggtccg ccttctggtc cacgcatata agcgcggact aaaaacaggg 3360
atgtactact gcaaggttcg caaggcgacc aacagcgggg tctttggcgg cgacgacaac 3420
attgtctgca tgagctgcgc gctgtgaccg acaaaccccc tccgcgccag gcccgccgcc 3480
actgtcgtcg ccgtcccaag ctctcccctg ctgccatg 3518

<210> 12

<211> 5956

<212> DNA

<213> Herpes simplex virus 2

<400> 12

gtgtgtttgg cgtgtgtctc tgaaatggcg gaaacccaca tgcaaatggg attcatggac 60
acgttacacc cccctgactc aggagatagg catatcctcc ttagattgac tcagcacacg 120
atcgcacccc acccctgtgt gccggggata aaagccaacg cgcgcggtct gggttaccac 180
aacaggtggg tgcttcgggg acttgacggt cgccactctc ctgcgagccc tcacgtcttc 240
gcccaccgat tcctgttgcg ttcctgtcgg ccggtgctgt cctgtcgaca gattgttggc 300
gactgcccgg gtgattcgtc ggccggtgcg tcctttcggt cgtaccgccc accccgcctc 360

ccacgggccc gccgctgttt ccgttcatcg cgtccgagcc accgtcacct tggttccaat 420
ggccaaccgc cctgccgcat ccgccctcgc cggagcgcgg tctccgtccg aacgacagga 480
accccgggag cccgaggtcg ccccccctgg cggcgaccac gtgttttgca ggaaagtcag 540
cggcgtgatg gtgctttcca gcgatccccc cggccccgcg gcctaccgca ttagcgacag 600
cagctttgtt caatgcggct ccaactgcag tatgataatc gacggagacg tggcgcgcgg 660
tcatttgcgt gacctcgagg gcgctacgtc caccggcgcc ttcgtcgcga tctcaaacgt 720
cgcagccggc ggggatggcc gaaccgccgt cgtggcgctc ggcggaacct cgggcccgtc 780
cgcgactaca tccgtgggga cccagacgtc cggggagttc ctccacggga acccaaggac 840
ccccgaaccc caaggacccc aggctgtccc cccgccccct cctcccccct ttccatgggg 900
ccacgagtgc tgcgcccgtc gcgatgccag gggcggcgcc gagaaggacg tcggggccgc 960
ggagtcatgg tcagacggcc cgtcgtccga ctccgaaacg gaggactcgg actcctcgga 1020
cgaggatacg ggctcgggtt cggagacgct gtctcgatcc tcttcgatct gggccgcagg 1080
ggcgactgac gacgatgaca gcgactccga ctcgcggtcg gacgactccg tgcagcccga 1140
cgttgtcgtt cgtcgcagat ggagcgacgg ccctgccccc gtggcctttc ccaagccccg 1200
gcgccccggc gactccccccg gaaaccccgg cctgggcgcc ggcaccgggc cgggctccgc 1260
gacggacccg cgcgcgtcgg ccgactccga ttccgcggcc cacgccgccg caccccaggc 1320
ggacgtggcg ccggttctgg acagccagcc cactgtggga acggaccccg gctacccagt 1380
ccccctagaa ctcacgcccg agaacgcgga ggcggtggcg cggtttctgg gggacgccgt 1440
cgaccgcgag cccgcgctca tgctggagta cttctgtcgg tgcgcccgcg aggagagcaa 1500
gcgcgtgccc ccacgaacct tcggcagcgc ccccccgcctc acggaggacg actttgggct 1560
cctgaactac gcgctcgctg agatgcgacg cctgtgcctg gaccttcccc cggtcccccc 1620
caacgcatac acgccctatc atctgaggga gtatgcgacg cggctggtta acgggttcaa 1680
acccctggtg cggcggtccg cccgcctgta tcgcatcctg gggattctgg ttcacctgcg 1740

catccgtacc cgggaggcct cctttgagga atggatgcgc tccaaggagg tggacctgga 1800
cttcgggctg acggaaaggc ttcgcgaaca cgaggcccag ctaatgatcc tggcccaggc 1860
cctgaacccc tacgactgtc tgatccacag caccccgaac acgctcgtcg agcgggggct 1920
gcagtcggcg ctgaagtacg aagagtttta cctcaagcgc ttcggcgggc actacatgga 1980
gtccgtcttc cagatgtaca cccgcatcgc cgggttcctg gcgtgccggg cgacccgcgg 2040
catgcgccac atcgccctgg ggcgacaggg gtcgtggtgg gaaatgttca agttcttttt 2100
ccaccgcctc tacgaccacc agatcgtgcc gtccaccccc gccatgctga acctcggaac 2160
ccgcaactac tacacgtcca gctgatacct ggtaaacccc caggccacca ctaaccaggc 2220
caccctccgg gccatcaccg gcaacgtgag cgccatcctc gcccgcaacg ggggcatcgg 2280
gctgtgcatg caggcgttca acgacgccag ccccggcacc gccagcatca tgccggccct 2340
gaaggtcctg gactccctgg tggcggcgca caacaaacag agcacgcgcc ccaccggggc 2400
gtgcgtgtac ctggaaccct ggcacagcga cgttcgggcc gtgctcagaa tgaagggcgt 2460
cctcgccggc gaggaggccc agcgctgcga caacatcttc agcgccctct ggatgccgga 2520
cctgttcttc aagcgcctga tccgccacct cgacggcgag aaaaacgtca cctggtccct 2580
gttcgaccgg gacaccagca tgtcgctcgc cgactttcac ggcgaggagt tcgagaagct 2640
gtacgagcac ctcgaggcca tggggttcgg cgaaacgatc cccatccagg acctggcgta 2700
cgccatcgtg cgcagcgcgg ccaccaccgg aagccccttc atcatgttta aggacgcggt 2760
aaacagccac tacatctacg acacgcaagg ggcggccatt gccggctcca acctctgcac 2820
ggagatcgtc cacccgtcct ccaaacgctc cagcggggtc tgcaacctgg gcagcgtgaa 2880
tctggcccga tgcgtctccc ggcggacgtt cgattttggc atgctccgcg acgccgtgca 2940
ggcgtgcgtg ctaatggtta atatcatgat agacagcacg ctgcagccga cgccccagtg 3000
cgcccgcggc cacgacaacc tgcggtccat gggcattggc atgcagggcc tgcacacggc 3060
gtgcctgaag atgggcctgg atctggagtc ggccgagttc cgggacctga acacacacat 3120

cgccgaggtg atgctgctcg cggccatgaa gaccagtaac gcgctgtgcg ttcgcggggc 3180

gcgtcccttc agccacttta agcgcagcat gtaccgggcc ggccgctttc actgggagcg 3240

cttttcgaac gccagcccgc ggtacgaggg cgagtgggag atgctacgcc agagcatgat 3300

gaaacacggc ctgcgcaaca gccagttcat cgcgctcatg cccaccgccg cctcggccca 3360

gatctcggac gtcagccagg gctttgcccc cctgttcacc aacctgttca gcaaggtgac 3420

cagggacggc gagacgctgc gccccaacac gctcttgctg aaggaactcg agcgcacgtt 3480

cggcgggaag cggctcctgg acgcgatgga cgggctcgag gccaagcagt ggtctgtggc 3540

ccaggccctg ccttgcctgg accccgccca ccccctccgg cggttcaaga cggccttcga 3600

ctacgaccag gaactgctga tcgacctgtg tgcagaccgc gccccctatg ttgatcacag 3660

ccaatccatg actctgtatg tcacagagaa ggcggacggg acgctccccg cctccaccct 3720

ggtccgcctt ctcgtccacg catataagcg cggcctgaag acggggatgt actactgcaa 3780

ggttcgcaag gcgaccaaca gcggggtgtt cgccggcgac gacaacatcg tctgcacaag 3840

ctgcgcgctg taagcaacag cgctccgatc ggggtcaggc gtcgctctcg gtcccgcata 3900

tcgccatgga tcccgccgtc tcccccgcga gcaccgaccc cctagatacc cacgcgtcgg 3960

gggccggggc ggccccgatt ccggtgtgcc ccacccccga gcggtacttc tacacctccc 4020

agtgccccga catcaaccac cttcgctccc tcagcatcct gaaccgctgg ctggagaccg 4080

agctcgtgtt cgtcggggac gaggaggacg tctccaagct ctccgagggc gagctcggct 4140

tctaccgctt tctgtttgcc ttcctgtcgg ccgcggacga cctggtgacg gaaaacctgg 4200

gcggcctctc cggcctcttc gaacagaagg acattcttca ctactacgtg gagcaggaat 4260

gcatcgaggt cgtccactcc cgcgtctaca acatcatcca gctggtgctc tttcacaaca 4320

acgaccaggc gcgccgcgcc tatgtggccc gcaccatcaa ccacccggcc attcgcgtca 4380

aggtggactg gctggaggcg cgggtgcggg aatgcgactc gatcccggag aagttcatcc 4440

tcatgatcct catcgagggc gtctttttg ccgcctcgtt cgccgccatc gcgtacctgc 4500

gcaccaacaa cctcctgcgg gtcacctgcc agtcgaacga cctcatcagc cgccacgagg 4560

ccgtgcatac gacagcctcg tgctacatct acaacaacta cctcgggggc cacgccaagc 4620

ccgaggcggc gcgcgtgtac cggctgtttc gggaggcggt ggatatcgag atcgggttca 4680

tccgatccca ggccccgacg gacagctcta tcctgagtcc gggggccctg gcggccatcg 4740

agaactacgt gcgattcagc gcggatcgcc tgctgggcct gatccatatg cagcccctgt 4800

attccgcccc cgcccccgac gccagctttc ccctcagcct catgtccacc gacaaacaca 4860

ccaacttctt cgagtgccgc agcacctcgt acgccggggc cgtcgtcaac gatctgtgag 4920

ggtctgggcg cccttgtagc gatgtctaac cgaaataaag gggtcgaaac ggactgttgg 4980

gtctccggtg tgattattac gcaggggagg ggggtggcgg ctggggaaag ggaaggaacg 5040

cccgaaacca gagaaaagga ccaaaaggga aacgcgtcca accgataaat caagcgccga 5100

ccagaacccc gagatgcata ataacaaacg attttattac tcttattatt aacaggtcgg 5160

gcatcgggag ggatgggggg cgcgcgtttc ctccgttccg gctactcgtc ccagaattta 5220

gccaggacgt ccttgtaaaa cgcgggcggg ggcgcgtggg cccacacctg cgccagaaac 5280

cggtcggcga tgtccggggc ggtgatatga cgagtcacga tggagcgcgc taaatcttcg 5340

tcgcggaggt cctgatagat gggcagtctt tttagaagag tccagggtcc ccgctccttg 5400

gggctgataa gcgatatgac gtacttgacg tatctgtgct ccaccagctc ggcgatggtc 5460

atcggatcgg gcagccagtc cagggcctcc ggggcgtcgt ggatgacgtg gcggcgacgt 5520

ccggcgacat agccgcggtg ttccgcgacc cgctgcgcgt tggggacctg cacgagctcg 5580

ggcggggtga gtatctccga ggaggacgac cgggcgccgt cgcgcggccc accggcgacg 5640

tccgggggct ggaggggggg gtcttcttcg tagtcgtcct cgcccgcgat ctgttgggcc 5700

agaatttcgg tccacgagat gcgcgtctcg aggccgaccg gggccgcggt cagcgtaggc 5760

atgctctcca gggagcgcga gttggcgcgc tcccgccggg ccgcccggcg ggcctgggat 5820

cggctcgggg cggtccagtg acactcgcgc agcacgtcct cgacggacgc gtaggtgtta 5880

ttggggtgca ggtctgtgtg gcagcggacg aacagcgcca ggaactgcgg gtaactcatc 5940

ttgaagtacc ctgcag 5956

<210> 13

<211> 3678

<212> DNA

<213> Equine herpesvirus 4

<400> 13

aaaccactgt tctttacact ttatgctcta gtttttggta atagtgtctt ggaacacttt 60

taccctaaac gaaattatgg ctttggattt tttgagcacc gactgtccac tggggattgt 120

ttccgatatt atatccaacg tgaataccat caaagagtat ggatattcca gcgaattatc 180

aacaacgctg gcacctcgcc cgtctcgaga acaggtgtta gagtatatca ccagagtcgt 240

ggataaactc aagccgctgt gcagagtcga cgaacgcctt tacattgcgt gcggggagct 300

tgtacaccta cgaattaaag cacgcaacac agacctgaaa tattggctaa aatcgtctga 360

gattgatctt agcgatgtcg tggaacaggc catattggaa cacattgact ttgttcagaa 420

aaccctcaac tcgtttgaaa catcggaata ccgagatttg tgttcattag gcctgcaatc 480

tgcgctaaag tatgaagaaa tgtatttagc caaaatgcga ggcggacgtc tagagtccat 540

ggggcaattt tttcttagac ttgcaactac tgctacgcac tatactatgg aacaaccagc 600

aatggctcgc gtgttggtta gcggtgaggt tggctggaca tatattttca gagccttttt 660

tactgcgcta gccggacagg ttgtcattcc ggccacgcca attatgctgt ttggtgggag 720

agactgtggg tctatggcca gctgttattt gctaaacccc agggtaacag atatgaactc 780

tgcaattccg gctcttatgg aagaggttgg acccattttg tgcaaccgag gaggaattgg 840
actgtcttta cagaggttta acactccacc cacagaaggt tgttcacggg gtgtcatggc 900
tctcctaaag ctactagact ctatgaccat ggccattaac agcgacggtg aaagaccaac 960
aggagtgtgt gtttatttcg aaccctggca cgcagacatc cgcgccattt taaatatgcg 1020
cggaatgctg gccagagacg aaactgtgcg ctgcgacaac atctttgctt gtatgtggac 1080
cccagacctg ttttttgacc gctatcaacg gtacgtcgat ggagaaagcg gcataatgtg 1140
gactctgttt gatgatactg catcgcacct ctgccatatg tacggaaatg atttcacacg 1200
ggaatatgag cgcctggagc ggtgtggatt tgggatagac gctattccca tacaggacat 1260
ggcctttatc atagttagaa gtgctgtaat gacaggaagc ccatttttga tgtttaaaga 1320
cgcgtgcaac aggcactacc actttgacat gcggcagaga ggtgcgataa tggggtctaa 1380
tctatgcaca gaaattatcc agcatgccga cgaaacccaa aacggggtgt gtaatctagc 1440
cagcatcaac ctcccaaaat gtctagccct tccacctcca aatattgcag gtgtgccata 1500
ttttgacttc gccgctctgg gccgcgctgc cgcaactgcc acaatttttg tcaatgcgat 1560
gatgtgtgcc agcacatatc caactgttaa atcccagaaa ggcgttgaag aaaaccggtc 1620
gctgggactt ggaattcagg ggctacatac cacgtttttg atgctggacc tggatatggc 1680
atctccagag gcgcaccaac taaacaagca aatagcagaa aggctgttat tgaactctat 1740
gaaggccagc gcaacgctct gcaagctggg tatgcaaccc tttaaagggt ttgaagacag 1800
caagtacagt cggggggaac taccctttga tgcctaccca aatgtaacac taacaaaccg 1860
caacgcctgg cgtagacttc gcactgacat aaaacaatac ggcttgtaca attctcagtt 1920
tgtagcctat atgccaacag tatcttcgtc acaggttacc gagagcagcg aggggttttc 1980
tcctgtttac acaaacctgt ttagcaaagt tactgctacc ggggaagtac tcaggcccaa 2040
tgtactgcta atgcgcacca tcagaagtat ttttccacag gaatgcgcgc gcttacaagc 2100

gctatctacg ctagaagctg cgaaatggtc agttgtggga gcgtttggtg atttgccagt 2160
tggtcacccc ctcagtaagt ttaaaacagc atttgagtac gaccagacta tgctaattaa 2220
catgtgtgct gacagggctg cgtttgtgga ccagagccaa tccatgtctt tgtttataac 2280
tgagcctgct gacggaaaac tccccgcctc cagaattatg aatcttttgg tccacgcata 2340
taaacgcgga cttaaaacag gcatgtacta ctgcaaaatc aagaaggcaa caaacaacgg 2400
agtctttgtt ggcggagacc tagtctgcac cagctgcagc ttgtagggca gcctcgccat 2460
tttgcccagg gcgggaaaat aattatggcc ctcgaaaact ctaaaaaaac agattttgct 2520
gacgagttat tgataaatgc gtatttctat acgccggaat gtcccgatat tgaacaccta 2580
cgcttgttga gcgttgccaa ccgctggctg gatacggacc ttccaatttc tgatgacctc 2640
aaggacgttg ctaaactcgc gccagccgag cgagagtttt accggttttt gtttgccttt 2700
ttatctgctg ctgacgactt ggtaaattta aacctgggag atttatccgc actatttact 2760
caaaaggaca ttcttcacta ctacattgag caagagtcta ttgaagtaac gcactccaga 2820
gtatatagcg ctatacagct tatgttgttt ggaaacgacg caacagcgcg cgctaggtat 2880
gtcgcatctg ttgtcaaaga cgtggccata gacctaaagg tatcttggtt gcaagcaaag 2940
gtgcgagaat gcaaatctgt ggcggaaaag tatattttga tgatattaat agagggcgtt 3000
ttcttcgcgt cgtcctttcc gtccatcgca tatcttcgca cccacaatct ctttgtggta 3060
acctgtcaaa gtaatgattt aattagccgc gacgaagcaa ttcacaccaa cgcctcgtgc 3120
tgtatctaca acaactacct tgggcgtttt gaaaagccag ctccaacgag gatttatgcg 3180
ctgttttctg aggccgtaaa catcgagtgt gaatttttgc tttcccatgc ccccaaaagc 3240
agccacctgt tggacattga agccatcata tgctacgtac gctatagcgc ggacaggctt 3300
ttgggggaaa ttggactatc tccgctgttt aatgctccca aaccccacc aagcttcccc 3360
ctagctttca tgactgtgga aaaacatacc aactttttg aaaggcgaag caccgcatac 3420
tcgggaactc ttataaacga tctgtaatgt aaaaataaaa actaattttg attcacttat 3480

ttgtcttgtt tgcgtgttgg atgtacgcga tttaaaaaaa tactgagaaa agatactccc 3540

gatttaactt tatttaagac cattgtcttc ggtgtccaca gtcatcccag tagttaacca 3600

acacagtgtt gtaatcagtg ggggtgggaa tgtggttcca aaacatatta gcaagctctc 3660

tgacaatttc gtgttcgg 3678

<210> 14

<211> 20

<212> DNA

<213> Escherichia coli

<400> 14

ccgtcgcgct ttgtcaccag 20

<210> 15

<211> 20

<212> DNA

<213> Escherichia coli

<400> 15

ctgtgctacc gtcgcgcttt 20

<210> 16

<211> 20

<212> DNA

<213> Escherichia coli

<400> 16

tgatgcgctc tgtgctaccg 20

<210> 17

<211> 20

<212> DNA

<213> Escherichia coli

<400> 17

tttgtcgaga ttgatgcgct 20

<210> 18

<211> 20

<212> DNA

<213> Escherichia coli

<400> 18

agaacgcgat ggattttgtc 20

<210> 19

<211> 20

<212> DNA

<213> Escherichia coli

<400> 19

tgccgcccaa tccagaacgc 20

<210> 20

<211> 20

<212> DNA

<213> Escherichia coli

<400> 20

agtccttctg ccgcccaatc 20

<210> 21

<211> 20

<212> DNA

<213> Escherichia coli

<400> 21

aaactgaatg tgggagcgca 20

<210> 22

<211> 20

<212> DNA

<213> Escherichia coli

<400> 22

ataatggttt cgtggatgtc 20

<210> 23

<211> 20

<212> DNA

<213> Escherichia coli

<400> 23

cggcagcctt gataatggtt 20

<210> 24

<211> 20

<212> DNA

<213> Escherichia coli

<400> 24

atactgataa tccggcgcat 20

<210> 25

<211> 20

<212> DNA

<213> Escherichia coli

<400> 25

tacgcaggtg gaagatcgcc 20

<210> 26

<211> 20

<212> DNA

<213> Escherichia coli

<400> 26

ggtcgtacag cgcaggcggc 20

<210> 27

<211> 20

<212> DNA

<213> Escherichia coli

<400> 27

gcccatctcg accattttca 20

<210> 28

<211> 20

<212> DNA

<213> Escherichia coli

<400> 28

tatcgtattt gcccatctcg 20

<210> 29

<211> 20

<212> DNA

<213> Escherichia coli

<400> 29

cggcagcata agagaaggtc 20

<210> 30

<211> 20

<212> DNA

<213> Escherichia coli

<400> 30

ccttccagct gcttaacggc 20

<210> 31

<211> 20

<212> DNA

<213> Escherichia coli

<400> 31

ccagatattt gccttccagc 20

<210> 32

<211> 20

<212> DNA

<213> Escherichia coli

<400> 32

atagatttcg ccggtcacgc 20

<210> 33

<211> 20

<212> DNA

<213> Escherichia coli

<400> 33

ggaactgggc gctctcatag 20

<210> 34

<211> 20

<212> DNA

<213> Escherichia coli

<400> 34

gaatataaag gaactgggcg 20

<210> 35

<211> 20

<212> DNA

<213> Escherichia coli

<400> 35

gcacgcggca actagaatat 20

<210> 36

<211> 20

<212> DNA

<213> Escherichia coli

<400> 36

ttcgagaaca agcacgcggc 20

<210> 37

<211> 20

<212> DNA

<213> Escherichia coli

<400> 37

tttcacgcgg gtagttcgag 20

<210> 38

<211> 20

<212> DNA

<213> Escherichia coli

<400> 38

acgcttcaca tattgcaggc 20

<210> 39

<211> 20

<212> DNA

<213> Escherichia coli

<400> 39

ggaaaccgcg tcgtaaaaac 20

<210> 40

<211> 20

<212> DNA

<213> Escherichia coli

<400> 40

ttaaatgtgg aaaccgcgtc 20

<210> 41

<211> 20

<212> DNA

<213> Escherichia coli

<400> 41

catgattggc gtcggcagcg 20

<210> 42

<211> 20

<212> DNA

<213> Escherichia coli

<400> 42

cgcacgccgg acatgattgg 20

<210> 43

<211> 20

<212> DNA

<213> Escherichia coli

<400> 43

cgagtcgggg tacgcacgcc 20

<210> 44

<211> 20

<212> DNA

<213> Escherichia coli

<400> 44

tcgatcagta cgcaggagct 20

<210> 45

<211> 20

<212> DNA

<213> Escherichia coli

<400> 45

gctgtcaccg cactcgatca 20

<210> 46

<211> 20

<212> DNA

<213> Escherichia coli

<400> 46

ggaatccagg ctgtcaccgc 20

<210> 47

<211> 20

<212> DNA

<213> Escherichia coli

<400> 47

ggaggtggcg ttgatggaat 20

<210> 48

<211> 20

<212> DNA

<213> Escherichia coli

<400> 48

aacaatcgcg ctggaggtgg 20

<210> 49

<211> 20

<212> DNA

<213> Escherichia coli

<400> 49

ctacccagcg cacgaatacg 20

<210> 50

<211> 20

<212> DNA

<213> Escherichia coli

<400> 50

atgcagccgg tatggaacgc 20

<210> 51

<211> 20

<212> DNA

<213> Escherichia coli

<400> 51

ttgtagaacg gaatgcagcc 20

<210> 52

<211> 20

<212> DNA

<213> Escherichia coli

<400> 52

ccgctgtctg gaaatgtttg 20

<210> 53

<211> 20

<212> DNA

<213> Escherichia coli

<400> 53

aggatttcac cgctgtctgg 20

<210> 54

<211> 20

<212> DNA

<213> Escherichia coli

<400> 54

cgcacaccgc cctgagagca 20

<210> 55

<211> 20

<212> DNA

<213> Escherichia coli

<400> 55

cacatcgggt agaacagcgt 20

<210> 56

<211> 20

<212> DNA

<213> Escherichia coli

<400> 56

ctttccactt ccagatgcca 20

<210> 57

<211> 20

<212> DNA

<213> Escherichia coli

<400> 57

ttgccttcca caccacggtt 20

<210> 58

<211> 20

<212> DNA

<213> Escherichia coli

<400> 58

cacgcggttg ccttccacac 20

<210> 59
<211> 20
<212> DNA
<213> Escherichia coli

<400> 59
ccatatgacg cacgcggttg 20

<210> 60
<211> 20
<212> DNA
<213> Escherichia coli

<400> 60
ttcacctttc agcagacggg 20

<210> 61
<211> 20
<212> DNA
<213> Escherichia coli

<400> 61

cgggctgaac agggtgatat 20

<210> 62

<211> 20

<212> DNA

<213> Escherichia coli

<400> 62

cggacgggct gaacagggtg 20

<210> 63

<211> 20

<212> DNA

<213> Escherichia coli

<400> 63

gtcggacggg ctgaacaggg 20

<210> 64

<211> 20

<212> DNA

<213> Escherichia coli

<400> 64

aaactcttcc tgatcggcga 20

<210> 65

<211> 20

<212> DNA

<213> Escherichia coli

<400> 65

gcggatgctg tcgtctttct 20

<210> 66

<211> 20

<212> DNA

<213> Escherichia coli

<400> 66

gctgcttgcg gatgctgtcg 20

<210> 67

<211> 20

<212> DNA

<213> Escherichia coli

<400> 67

ggctttcaca cgctgcttgc 20

<210> 68

<211> 20

<212> DNA

<213> Escherichia coli

<400> 68

gctcaacggc tttcacacgc 20

<210> 69

<211> 20

<212> DNA

<213> Escherichia coli

<400> 69

gaccggtaga cgcacgttcc 20

<210> 70

<211> 20

<212> DNA

<213> Escherichia coli

<400> 70

gggctatggg tattgcagtg 20

<210> 71

<211> 20

<212> DNA

<213> Escherichia coli

<400> 71

aaacgggcta tgggtattgc 20

<210> 72

<211> 20

<212> DNA

<213> Escherichia coli

<400> 72

cggatcaaac gggctatggg 20

<210> 73

<211> 20

<212> DNA

<213> Escherichia coli

<400> 73

gggctatctc caggcacagg 20

<210> 74

<211> 20

<212> DNA

<213> Escherichia coli

<400> 74

ggcagggcta tctccaggca 20

<210> 75

<211> 20

<212> DNA

<213> Escherichia coli

<400> 75

tggtcggcag ggctatctcc 20

<210> 76

<211> 20

<212> DNA

<213> Escherichia coli

<400> 76

gcggtttggt cggcagggct 20

<210> 77

<211> 20

<212> DNA

<213> Escherichia coli

<400> 77

ttcagcggtt tggtcggcag 20

<210> 78

<211> 20

<212> DNA

<213> Escherichia coli

<400> 78

acgtcgttca gcggtttggt 20

<210> 79

<211> 20

<212> DNA

<213> Escherichia coli

<400> 79

tttcaccgtt ctcgtcgttg 20

<210> 80

<211> 20

<212> DNA

<213> Escherichia coli

<400> 80

cagcgcgatt tcaccgttct 20

<210> 81

<211> 20

<212> DNA

<213> Escherichia coli

<400> 81

cgtacacagc gcgatttcac 20

<210> 82

<211> 20

<212> DNA

<213> Escherichia coli

<400> 82

agcagacagc gtacacagcg 20

<210> 83

<211> 20

<212> DNA

<213> Escherichia coli

<400> 83

caggttgaaa gcagacagcg 20

<210> 84

<211> 20

<212> DNA

<213> Escherichia coli

<400> 84

aattgcgccc aggttgaaag 20

<210> 85

<211> 20

<212> DNA

<213> Escherichia coli

<400> 85

ccaggttatt aattgcgccc 20

<210> 86

<211> 20

<212> DNA

<213> Escherichia coli

<400> 86

ttgccagctc ttccagttca 20

<210> 87

<211> 20

<212> DNA

<213> Escherichia coli

<400> 87

accgccagaa ttgccagctc 20

<210> 88

<211> 20

<212> DNA

<213> Escherichia coli

<400> 88

gtcaagtgca cgaaccgcca 20

<210> 89

<211> 20

<212> DNA

<213> Escherichia coli

<400> 89

atccagcagc gcgtcaagtg 20

<210> 90

<211> 20

<212> DNA

<213> Escherichia coli

<400> 90

tgataatcca gcagcgcgtc 20

<210> 91

<211> 20

<212> DNA

<213> Escherichia coli

<400> 91

gatcacacca atacccagcg 20

<210> 92

<211> 20

<212> DNA

<213> Escherichia coli

<400> 92

tcgttcgcca ggtagtaagc 20

<210> 93

<211> 20

<212> DNA

<213> Escherichia coli

<400> 93

cgtttaccgt cgttcgccag 20

<210> 94

<211> 20

<212> DNA

<213> Escherichia coli

<400> 94

tgccgtcgga gtagcgttta 20

<210> 95

<211> 20

<212> DNA

<213> Escherichia coli

<400> 95

tatgcgtcag gttgttggcg 20

<210> 96

<211> 20

<212> DNA

<213> Escherichia coli

<400> 96

cgaaggtttt atgcgtcagg 20

<210> 97

<211> 20

<212> DNA

<213> Escherichia coli

<400> 97

gttaaaccac gggcacgcgc 20

<210> 98

<211> 20

<212> DNA

<213> Escherichia coli

<400> 98

ttcgcgtaag tggtttcgtt 20

<210> 99

<211> 20

<212> DNA

<213> Escherichia coli

<400> 99

tataggtatc gatcggcagg 20

<210> 100

<211> 20

<212> DNA

<213> Escherichia coli

<400> 100

cagtcgtaat gcagcggctc 20

<210> 101

<211> 20

<212> DNA

<213> Escherichia coli

<400> 101

cgcagagctt cccagtcgta 20

<210> 102

<211> 20

<212> DNA

<213> Escherichia coli

<400> 102

tcagagcaga aagcgtggag 20

<210> 103

<211> 20

<212> DNA

<213> Escherichia coli

<400> 103

tcggacggca tcagagcaga 20

<210> 104

<211> 20

<212> DNA

<213> Escherichia coli

<400> 104

ggcgttagag atctgcgaag 20

<210> 105

<211> 20

<212> DNA

<213> Escherichia coli

<400> 105

tttgatgctg acgtaaccgc 20

<210> 106

<211> 20

<212> DNA

<213> Escherichia coli

<400> 106

tcgacgcttt gatgctgacg 20

<210> 107

<211> 20

<212> DNA

<213> Escherichia coli

<400> 107

cctggcgcaa aataccgtct 20

<210> 108

<211> 20

<212> DNA

<213> Escherichia coli

<400> 108

tagtccggca ccacctggcg 20

<210> 109

<211> 20

<212> DNA

<213> Escherichia coli

<400> 109

gcaggtgctc gtagtccggc 20

<210> 110

<211> 20

<212> DNA

<213> Escherichia coli

<400> 110

cgtcgtgcag gtgctcgtag 20

<210> 111

<211> 20

<212> DNA

<213> Escherichia coli

<400> 111

gctcataggc gtcgtgcagg 20

<210> 112

<211> 20

<212> DNA

<213> Escherichia coli

<400> 112

cccacagcag ctcataggcg 20

<210> 113

<211> 20

<212> DNA

<213> Escherichia coli

<400> 113

cggcatttcc cacagcagct 20

<210> 114

<211> 20

<212> DNA

<213> Escherichia coli

<400> 114

catcgttacc cggcatttcc 20

<210> 115

<211> 20

<212> DNA

<213> Escherichia coli

<400> 115

ggatcgtagt tggtgttggc 20

<210> 116

<211> 20

<212> DNA

<213> Escherichia coli

<400> 116

tcggcacttt tcctgacggg 20

<210> 117

<211> 20

<212> DNA

<213> Escherichia coli

<400> 117

aggcggtgag caggtctttc 20

<210> 118

<211> 20

<212> DNA

<213> Escherichia coli

<400> 118

cgaatttgta ggcggtgagc 20

<210> 119

<211> 20

<212> DNA

<213> Escherichia coli

<400> 119

gtgttttgac cccgaatttg 20

<210> 120

<211> 20

<212> DNA

<213> Escherichia coli

<400> 120

cgtcttgtgc gtcttcagcg 20

<210> 121

<211> 20

<212> DNA

<213> Escherichia coli

<400> 121

tcttacatgc gccgctttcg 20

<210> 122

<211> 20

<212> DNA

<213> Escherichia coli

<400> 122

cggctgacca aagaacatcg 20

<210> 123

<211> 20

<212> DNA

<213> Escherichia coli

<400> 123

ccacgttgac cggctgacca 20

<210> 124

<211> 20

<212> DNA

<213> Escherichia coli

<400> 124

tagcgagcca cgttgaccgg 20

<210> 125

<211> 20

<212> DNA

<213> Escherichia coli

<400> 125

cggacgccag aagaaagaga 20

<210> 126

<211> 20

<212> DNA

<213> Escherichia coli

<400> 126

caacttcttc cggacgccag 20

<210> 127

<211> 20

<212> DNA

<213> Escherichia coli

<400> 127

aatctatacg gtcgcgggag 20

<210> 128

<211> 20

<212> DNA

<213> Escherichia coli

<400> 128

tgtgtttttc gtgctccggc 20

<210> 129

<211> 20

<212> DNA

<213> Escherichia coli

<400> 129

gcaatagcgc cacgttcggg 20

<210> 130

<211> 20

<212> DNA

<213> Escherichia coli

<400> 130

agaaataagc ggcaatagcg 20

<210> 131

<211> 20

<212> DNA

<213> Escherichia coli

<400> 131

cggaatagaa ataagcggca 20

<210> 132

<211> 20

<212> DNA

<213> Escherichia coli

<400> 132

acccaggttt ccagttccgg 20

<210> 133

<211> 20

<212> DNA

<213> Escherichia coli

<400> 133

ataggaacgg gaatgaatcg 20

<210> 134

<211> 20

<212> DNA

<213> Escherichia coli

<400> 134

tcccttccgc acgtttctgg 20

<210> 135

<211> 20

<212> DNA

<213> Escherichia coli

<400> 135

cgcccagcag atgccagtag 20

<210> 136

<211> 20

<212> DNA

<213> Escherichia coli

<400> 136

gtaccttcgc ccagcagatg 20

<210> 137

<211> 20

<212> DNA

<213> Escherichia coli

<400> 137

cgcaggctaa cggtcacagt 20

<210> 138

<211> 20

<212> DNA

<213> Escherichia coli

<400> 138

tttcttcagc tcgcgcaggc 20

<210> 139

<211> 20

<212> DNA

<213> Escherichia coli

<400> 139

gcaaatgcga aggaacaagc 20

<210> 140

<211> 20

<212> DNA

<213> Escherichia coli

<400> 140

atcaattcgc gttctgcaaa 20

<210> 141

<211> 20

<212> DNA

<213> Escherichia coli

<400> 141

gcgaataatt ttggcgttgc 20

<210> 142

<211> 20

<212> DNA

<213> Escherichia coli

<400> 142

gcgggcaatc aggcgaataa 20

<210> 143

<211> 20

<212> DNA

<213> Escherichia coli

<400> 143

cagggcttcg tcgcgggcaa 20

<210> 144

<211> 20

<212> DNA

<213> Escherichia coli

<400> 144

cggtcaggtg cagggcttcg 20

<210> 145

<211> 20

<212> DNA

<213> Escherichia coli

<400> 145

tgctgggtgc cggtcaggtg 20

<210> 146

<211> 20

<212> DNA

<213> Escherichia coli

<400> 146

catatgctgg gtgccggtca 20

<210> 147

<211> 20

<212> DNA

<213> Escherichia coli

<400> 147

gcaatttccg ccatctcagg 20

<210> 148

<211> 20

<212> DNA

<213> Escherichia coli

<400> 148

tcctgcttac actcttcggc 20

<210> 149

<211> 20

<212> DNA

<213> Escherichia coli

<400> 149

atccgcccag tctttctcct 20

<210> 150

<211> 20

<212> DNA

<213> Escherichia coli

<400> 150

gaacagataa tccgcccagt 20

<210> 151

<211> 20

<212> DNA

<213> Escherichia coli

<400> 151

gggttggagc gcgtctggaa 20

<210> 152

<211> 20

<212> DNA

<213> Escherichia coli

<400> 152

cgggatcggg ttggagcgcg 20

<210> 153

<211> 20

<212> DNA

<213> Escherichia coli

<400> 153

cacgggatcg ggttggagcg 20

<210> 154

<211> 20

<212> DNA

<213> Escherichia coli

<400> 154

ctgacttcca cttcctgcgg 20

<210> 155

<211> 20

<212> DNA

<213> Escherichia coli

<400> 155

tgcccgacca gataagaact 20

<210> 156

<211> 20

<212> DNA

<213> Escherichia coli

<400> 156

ttccgagtca atctgcccga 20

<210> 157

<211> 20

<212> DNA

<213> Escherichia coli

<400> 157

aatcgtcggt gtccacttcc 20

<210> 158

<211> 20

<212> DNA

<213> Escherichia coli

<400> 158

gaccactttg cgcatccggc 20

<210> 159

<211> 20

<212> DNA

<213> Escherichia coli

<400> 159

gatgttgacc actttgcgca 20

<210> 160

<211> 20

<212> DNA

<213> Escherichia coli

<400> 160

atctccggtt ccatggcatt 20

<210> 161

<211> 20

<212> DNA

<213> Escherichia coli

<400> 161

tttttccatc tccggttcca 20

<210> 162

<211> 20

<212> DNA

<213> Escherichia coli

<400> 162

ccctttcagt tcttcgtcgg 20

<210> 163

<211> 20

<212> DNA

<213> Escherichia coli

<400> 163
gcggttttcc ctttcagttc 20

<210> 164
<211> 20
<212> DNA
<213> Escherichia coli

<400> 164
actctgcggt tttccctttc 20

<210> 165
<211> 20
<212> DNA
<213> Escherichia coli

<400> 165
cgccttttc cagacgtgca 20

<210> 166
<211> 20
<212> DNA

<213> Escherichia coli

<400> 166

cacttcgcct ttttccagac 20

<210> 167

<211> 20

<212> DNA

<213> Escherichia coli

<400> 167

tttccagcac ttcgcctttt 20

<210> 168

<211> 20

<212> DNA

<213> Escherichia coli

<400> 168

cagattttcc agcacttcgc 20

<210> 169

<211> 20

<212> DNA

<213> Escherichia coli

<400> 169

acttgcctca cgtaccacgg 20

<210> 170

<211> 20

<212> DNA

<213> Escherichia coli

<400> 170

gacgcgctta cttgcctcac 20

<210> 171

<211> 20

<212> DNA

<213> Escherichia coli

<400> 171

gtgacgcata ccaaagacgc 20

<210> 172

<211> 20

<212> DNA

<213> Escherichia coli

<400> 172

taagaaccat accgccgagt 20

<210> 173

<211> 20

<212> DNA

<213> Escherichia coli

<400> 173

atttcggcga tgcagcgttc 20

<210> 174

<211> 20

<212> DNA

<213> Escherichia coli

<400> 174

ttccttcacc ggtacgcatt 20

<210> 175

<211> 20

<212> DNA

<213> Escherichia coli

<400> 175

gtttttcctt caccggtacg 20

<210> 176

<211> 20

<212> DNA

<213> Escherichia coli

<400> 176

cgttgcggtc agggtttttc 20

<210> 177

<211> 20

<212> DNA

<213> Escherichia coli

<400> 177

tcaggtaagc aggcagcgtt 20

<210> 178

<211> 20

<212> DNA

<213> Escherichia coli

<400> 178

taccggttag tgcgttcagg 20

<210> 179

<211> 20

<212> DNA

<213> Escherichia coli

<400> 179

ttgcgccagg tagtcgttga 20

<210> 180

<211> 20

<212> DNA

<213> Escherichia coli

<400> 180

gtcacgttgc gccaggtagt 20

<210> 181

<211> 20

<212> DNA

<213> Escherichia coli

<400> 181

agcggacggt tgttttcggc 20

<210> 182

<211> 20

<212> DNA

<213> Escherichia coli

<400> 182

ggaattcaaa cagcggacgg 20

<210> 183

<211> 20

<212> DNA

<213> Escherichia coli

<400> 183

aggccaagga attcaaacag 20

<210> 184

<211> 20

<212> DNA

<213> Escherichia coli

<400> 184

ataccgacag tcaggccaag 20

<210> 185

<211> 20

<212> DNA

<213> Escherichia coli

<400> 185

ttcgcgcttt gccggtgctg 20

<210> 186

<211> 20

<212> DNA

<213> Escherichia coli

<400> 186

agccgtattc gttgttcgta 20

<210> 187

<211> 20

<212> DNA

<213> Escherichia coli

<400> 187

cgcaggtagt caaagccgta 20

<210> 188

<211> 20

<212> DNA

<213> Escherichia coli

<400> 188

atgttgtcgc gcaggtagtc 20

<210> 189

<211> 20

<212> DNA

<213> Escherichia coli

<400> 189

gccatgttgt cgcgcaggta 20

<210> 190

<211> 20

<212> DNA

<213> Escherichia coli

<400> 190

gtccacttcg tccaccagcg 20

<210> 191

<211> 20

<212> DNA

<213> Escherichia coli

<400> 191

ggtgtacgcg cttcatcgat 20

<210> 192

<211> 20

<212> DNA

<213> Escherichia coli

<400> 192

cggtgtacgc gcttcatcga 20

<210> 193

<211> 20

<212> DNA

<213> Escherichia coli

<400> 193

gcgtttatac atttccgagc 20

<210> 194

<211> 20

<212> DNA

<213> Escherichia coli

<400> 194

cggatcaggt gcggaataat 20

<210> 195

<211> 20

<212> DNA

<213> Escherichia coli

<400> 195

ttcacctggc gagattttc 20

<210> 196

<211> 20

<212> DNA

<213> Escherichia coli

<400> 196

cagcaccaga ccacgttcgg 20

<210> 197

<211> 20

<212> DNA

<213> Escherichia coli

<400> 197

gccctctttc accagcagtt 20

<210> 198

<211> 20

<212> DNA

<213> Escherichia coli

<400> 198

cccttcatcc atgatgccct 20

<210> 199

<211> 20

<212> DNA

<213> Escherichia coli

<400> 199

ttggccggag agtacagaga 20

<210> 200

<211> 20

<212> DNA

<213> Escherichia coli

<400> 200
agcatgatgt tggccggaga 20

<210> 201
<211> 20
<212> DNA
<213> Escherichia coli

<400> 201
agcgccgccg ttacgtggtg 20

<210> 202
<211> 20
<212> DNA
<213> Escherichia coli

<400> 202
gtcacgggta aacagcgcat 20

<210> 203
<211> 20

<212> DNA

<213> Escherichia coli

<400> 203

caccttcttt cgcttccaca 20

<210> 204

<211> 20

<212> DNA

<213> Escherichia coli

<400> 204

cagcgtttgg ttttcgttct 20

<210> 205

<211> 20

<212> DNA

<213> Escherichia coli

<400> 205

ggtgatcgaa gccagcgttt 20

<210> 206

<211> 20

<212> DNA

<213> Escherichia coli

<400> 206

gacggaagta gttctggaag 20

<210> 207

<211> 20

<212> DNA

<213> Escherichia coli

<400> 207

cccgccagtt tttcatacag 20

<210> 208

<211> 20

<212> DNA

<213> Escherichia coli

<400> 208

tcatccccgc cagtttttca 20

<210> 209

<211> 20

<212> DNA

<213> Escherichia coli

<400> 209

gaacaacgac ggtatccagc 20

<210> 210

<211> 20

<212> DNA

<213> Escherichia coli

<400> 210

gcctttcgca gtacgttctt 20

<210> 211

<211> 20

<212> DNA

<213> Escherichia coli

<400> 211

tagtacccac cagcaccggc 20

<210> 212
<211> 20
<212> DNA
<213> Escherichia coli

<400> 212
cggctttggt cagttcgttt 20

<210> 213
<211> 20
<212> DNA
<213> Escherichia coli

<400> 213
tgtgcttaat accggctttg 20

<210> 214
<211> 20
<212> DNA
<213> Escherichia coli

<400> 214

gttggcgtgg aatttggcgt 20

<210> 215

<211> 20

<212> DNA

<213> Escherichia coli

<400> 215

gcctgagcaa caatcgccgc 20

<210> 216

<211> 20

<212> DNA

<213> Escherichia coli

<400> 216

ctgtaccacg acccgccata 20

<210> 217

<211> 20

<212> DNA

<213> Escherichia coli

<400> 217

tatctgtacc acgacccgcc 20

<210> 218

<211> 20

<212> DNA

<213> Escherichia coli

<400> 218

ctgcctgcca gctaccaccg 20

<210> 219

<211> 20

<212> DNA

<213> Escherichia coli

<400> 219

tttccagcgc ggcaacttct 20

<210> 220

<211> 20

<212> DNA

<213> Escherichia coli

<400> 220

tttgctctgc ggtcggattt 20

<210> 221

<211> 20

<212> DNA

<213> Escherichia coli

<400> 221

tttttcaatt tgctctgcgg 20

<210> 222

<211> 20

<212> DNA

<213> Escherichia coli

<400> 222

accgcatcgt gacgtacctg 20

<210> 223

<211> 20

<212> DNA

<213> Escherichia coli

<400> 223

ccagtaccgc atcgtgacgt 20

<210> 224

<211> 20

<212> DNA

<213> Escherichia coli

<400> 224

gcttccagta ccgcatcgtg 20

<210> 225

<211> 20

<212> DNA

<213> Escherichia coli

<400> 225

accgatgata tgcaggccac 20

<210> 226

<211> 20

<212> DNA

<213> Escherichia coli

<400> 226

acgaccagaa cgaccgcgca 20

<210> 227

<211> 20

<212> DNA

<213> Escherichia coli

<400> 227

cccctgacga ccagaacgac 20

<210> 228

<211> 20

<212> DNA

<213> Escherichia coli

<400> 228

catcccctg acgaccagaa 20

<210> 229

<211> 20

<212> DNA

<213> Escherichia coli

<400> 229

gaaacgggaa gaaccagcat 20

<210> 230

<211> 20

<212> DNA

<213> Escherichia coli

<400> 230

cgacaggtag aaacgggaag 20

<210> 231

<211> 20

<212> DNA

<213> Escherichia coli

<400> 231

ggaagcaaaa atacgcatca 20

<210> 232

<211> 20

<212> DNA

<213> Escherichia coli

<400> 232

ggtcggaagc aaaaatacgc 20

<210> 233

<211> 20

<212> DNA

<213> Escherichia coli

<400> 233

cggatactcg gtcggaagca 20

<210> 234

<211> 20

<212> DNA

<213> Escherichia coli

<400> 234

acccagttta cgcatcatgc 20

<210> 235

<211> 20

<212> DNA

<213> Escherichia coli

<400> 235

acgggtgttc aatggcttcg 20

<210> 236

<211> 20

<212> DNA

<213> Escherichia coli

<400> 236

atcgctttag tcacccacgg 20

<210> 237

<211> 20

<212> DNA

<213> Escherichia coli

<400> 237

ctttcaactt tacgctgggc 20

<210> 238

<211> 20

<212> DNA

<213> Escherichia coli

<400> 238

acggctttca actttacgct 20

<210> 239

<211> 20

<212> DNA

<213> Escherichia coli

<400> 239

tggtttcgct cacatcgctg 20

<210> 240

<211> 20

<212> DNA

<213> Escherichia coli

<400> 240

gtaggcatca atggtcgctt 20

<210> 241

<211> 20

<212> DNA

<213> Escherichia coli

<400> 241

ccacatttct tccagcgact 20

<210> 242

<211> 20

<212> DNA

<213> Escherichia coli

<400> 242

atcccacatt tcttccagcg 20

<210> 243

<211> 20

<212> DNA

<213> Escherichia coli

<400> 243

tcacgcagcg tctcttcatg 20

<210> 244

<211> 20

<212> DNA

<213> Escherichia coli

<400> 244

cctttctcga agtgacgcat 20

<210> 245

<211> 20

<212> DNA

<213> Escherichia coli

<400> 245

ccacagggag tcaagcgttt 20

<210> 246
<211> 20
<212> DNA
<213> Escherichia coli

<400> 246
tcgctgccag gtgctctttc 20

<210> 247
<211> 20
<212> DNA
<213> Escherichia coli

<400> 247
gtccatcgct gccaggtgct 20

<210> 248
<211> 20
<212> DNA
<213> Escherichia coli

<400> 248

acgcagatag tccatcgctg 20

<210> 249

<211> 20

<212> DNA

<213> Escherichia coli

<400> 249

cttcggatct ttctgtgcgt 20

<210> 250

<211> 20

<212> DNA

<213> Escherichia coli

<400> 250

cgtttgtatt cctgcttcgg 20

<210> 251

<211> 20

<212> DNA

<213> Escherichia coli

<400> 251

atcgctgcaa acatggagaa 20

<210> 252

<211> 20

<212> DNA

<213> Escherichia coli

<400> 252

ccatacgacg ctgttgttcc 20

<210> 253

<211> 20

<212> DNA

<213> Escherichia coli

<400> 253

ggcttccata cgacgctgtt 20

<210> 254

<211> 20

<212> DNA

<213> Escherichia coli

<400> 254

cgctaaacgc tcggcttcca 20

<210> 255

<211> 20

<212> DNA

<213> Escherichia coli

<400> 255

gctaagctgc tgcatttgcg 20

<210> 256

<211> 20

<212> DNA

<213> Escherichia coli

<400> 256

ctactttgcg ctctccggtt 20

<210> 257

<211> 20

<212> DNA

<213> Escherichia coli

<400> 257

ttacgtccta ctttgcgctc 20

<210> 258

<211> 20

<212> DNA

<213> Escherichia coli

<400> 258

aaccgcacgg gcaaggatcg 20

<210> 259

<211> 20

<212> DNA

<213> Escherichia coli

<400> 259

accagaaccg cacgggcaag 20

<210> 260

<211> 20

<212> DNA

<213> Escherichia coli

<400> 260

tttttaccag aaccgcacgg 20

<210> 261

<211> 20

<212> DNA

<213> Escherichia coli

<400> 261

caggtagtcg ttgacggtaa 20

<210> 262

<211> 18

<212> DNA

<213> Escherichia coli

<400> 262

caggtagtcg ttgacggt 18

<210> 263

<211> 20

<212> DNA

<213> Escherichia coli

<400> 263

cggaagtagt tctggaaggt 20

<210> 264

<211> 20

<212> DNA

<213> Escherichia coli

<400> 264

cgaccgcgca actggttatc 20

<210> 265

<211> 20

<212> DNA

<213> Escherichia coli

<400> 265

ccgcacgggc aaggatcgtt 20

<210> 266

<211> 20

<212> DNA

<213> Artificial sequence

<223> Antisense Oligonucleotide

<400> 266

ggctaaatcg ctccaccaag 20

## Description

## BACKGROUND OF THE INVENTION

### Field of the Invention

This invention relates to antisense oligonucleotides which modulate the activity of the ribonucleotide reductase genes and the secA genes in microorganisms. This invention is also related to methods of using such compounds in inhibiting the growth of microorganisms.

These antisense oligonucleotides are particularly useful in treating pathological conditions in mammals which are mediated by the growth of microorganisms. Accordingly, this invention also relates to pharmaceutical compositions comprising a pharmaceutically acceptable excipient and an effective amount of a compound of this invention.

These antisense oligonucleotides may also be used as anti-microbial agents for agricultural applications such as crop protection.

### References

The following publications, patent applications and patents are cited in this application as superscript numbers:


1. Nordlund and Eklund "Structure and function of the *Escherichia coli* ribonucleotide reductase protein R2", *J. Mol. Biol.* (1993) **232**:123-164;

2. Carlson et al., "Primary structure of the *Escherichia coli* ribonucleoside diphosphate reductase operon", *PNAS* USA (1984) **81**:4294-4297;

3. Nilsson et al., "Nucleotide sequence of the gene coding for the large subunit of ribonucleotide reductase of *Escherichia coli* Correction", *Nucleic Acids Research* (1988) **16**:4174;

4. P. Reichard, "The anaerobic ribonucleotide reductase from *Escherichia coli", J. Biol. Chem*. (1993) **268**: 8383-8386;

5. Nordlund et al., *Nature* (1990) **345**:593-598;

6. der Blaauwen et al., "Inhibition of preprotein translocation and reversion of the membrane inserted state of secA by a carboxyl terminus binding Mab", *Biochemistry* (1997) **36**:9159-9168;

7. McNicholas et al., "Dual regulation of Escherichia coli secA translation by distinct upstream elements", *J. Mol. Biol.* (1997) **265**:128-141;

8. U.S. Patent No. 5,294,533;

9. Gasparro et al., "Photoactivatable antisense DNA: Suppression of ampicillin resistance in normally resistant *Escherichia coli ", Antisense Research and Development* (1991) **1**:117-140;

10. White et al., "Inhibition of the multiple antibiotic resistance (mar) operon in Escherichia coli by antisense DNA analogs", *Antimicrobial Agents and Chemotherapy* (1997) **41**:2699-2704;

11. Nielsen et al., *Science* (1991) **354**:1497;

12. Good and Nielsen, "Inhibition of translation and bacterial growth by peptide nucleic acid targeted to ribosomal RNA", *PNAS USA* (1998) **95**:2073-2076;

13. Buchardt, deceased, et al., U.S. Patent No. 5,766,855;

14. Buchardt, deceased, et al., U.S. Patent No. 5,719,262;

15. U.S. Patent No. 5,034,506;

16. Altschul, et al.,"Basic local alignment search tool", *J. Mol. Biol.* (1990) **215**:403-10;

17. Devereux. et al. , "A comprehensive set of sequence analysis programs for the VAX", *Nucleic Acids Res.* (1984) **12**:387-395;

18. Sambrook et al., *Molecular Cloning: A Laboratory Manual,* Cold Spring Harbor Laboratory, New York (1989, 1992);

19. Ausubel et al., *Current Protocols in Molecular Biology,* John Wiley and Sons, Baltimore Maryland (1989);

20. Chang et al., Somatic Gene Therapy, CRC Press, Ann Arbor MI (1995);

21. Vega et al., *Gene Targeting*, CRC Press, Ann Arbor MI (1995);

22. *Vectors: A Survey of Molecular Cloning Vectors and Their Uses,* Butterworths, Boston MA (1988)

23. U.S. Patent 5,023,252, issued June 11, 1991

24. Felgner et al., U.S. Patent No. 5,580,859.

25. U.S. Patent 5,011,472

26. *Remington's Pharmaceutical Sciences,* Mace Publishing Company, Philadelphia PA $17^{th}$ ed. (1985);

27. Perbal, *A Practical Guide to Molecular Cloning,* John Wiley & Sons, New York (1988).

28. *PCR Protocols: A Guide To Methods And Applications,* Academic Press, San Diego, CA (1990).

29. Dower, W.J., *Nucleic Acids Res.* (1988) **16**:6127;

30. Neuman et al., *EMBO J.* (1982) **1**:841;

31. Taketo A., *Biochim Biophys. Acta* (1988) **949**:318;

32. Miller J.H. *Experiments in Molecular Genetics,* Cold Spring Harbor Laboratory Press, Cold Spring Harbor, N. Y. (1972);

33. Horwitz J.P., *J. Med. Chem.* (1964) **7**:574;

34. Mann et al., *Biochem.*(1991) **30**:1939;

35. Olsvik, et al., *Acta Pathol. Microbiol. Immunol. Scand. [B]* (1982) **90**:319;

36. Laemmli, U.K., *Nature* (1970) **227**:680;

37. Choy et al., *Cancer Res.*(1988) **48**:2029;

38. Wright and Anazodo, *Cancer J.* (1988) **8**:185-189;

39. Chan et al., *Biochemistry* (1993) **32**:12835-12840;

40. Carpentier P.L., *Microbiology $4^{th}$ ed.* W.B.Saunders Company (1977); and

41. Wright et al., *Adv. Enzyme Regul.* (1981) **19**:105-127.

All of the above publications, patent applications and patents are herein incorporated by reference in their entirety to the same extent as if each individual publication, patent application or patent was specifically and individually indicated to be incorporated by reference in its entirety.

State of the Art

Ribonucleotide reductase catalyzes the *de novo* production of deoxyribonucleotides. The enzyme reduces the four main ribonucleotides to the corresponding deoxyribonucleotides required for DNA synthesis and repair (Wright et al.[41]).

In mammalian and bacterial cells, *de novo* production of deoxyribonucleotides by ribonucleotide reductase is usually highly regulated on different levels in order to produce the correct amount of deoxyribonucleotides for DNA synthesis. In the DNA viruses, the metabolism of the host cell is directed towards production of viral DNA by virus encoded ribonucleotide reductases (Nordlund and Eklund[1]).

Mammalian cells and many DNA viruses and prokaryotes, have a heterodimeric iron-containing ribonucleotide reductase enzyme of the $\alpha_2\beta_2$ type. For example, ribonucleotide reductase from *E. coli* is a multi-subunit $\alpha_2\beta_2$ enzyme where the two homo-dimeric proteins are denoted R1 and R2. The larger $\alpha_2$ protein, R1, contains the binding sites for substrate and allosteric effectors and also the redox-active cysteine residues. Protein R1 has a molecular mass of 2 x 86,000 where each subunit contains 761 residues. The smaller $\beta_2$ protein, denoted R2, contains the dinuclear ferric center and a stable free tyrosyl radical necessary for the enzymatic activity. The R2 protein has a molecular mass of 2 x 43,500, where each subunit contains 375 amino acid residues (Nordlund and Eklund[1]).

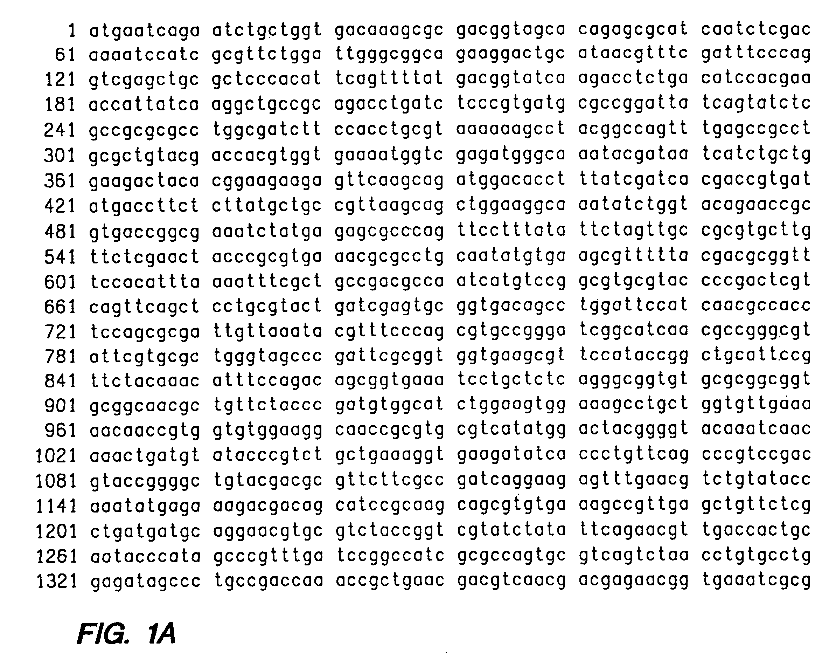

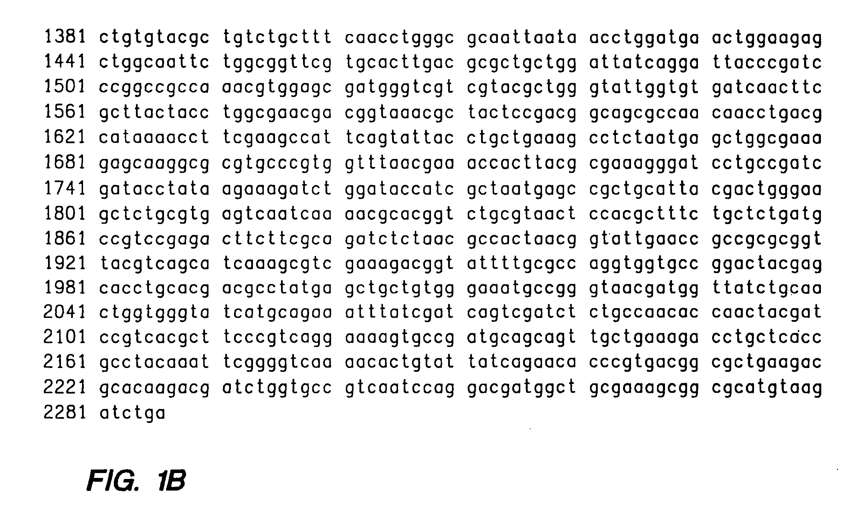

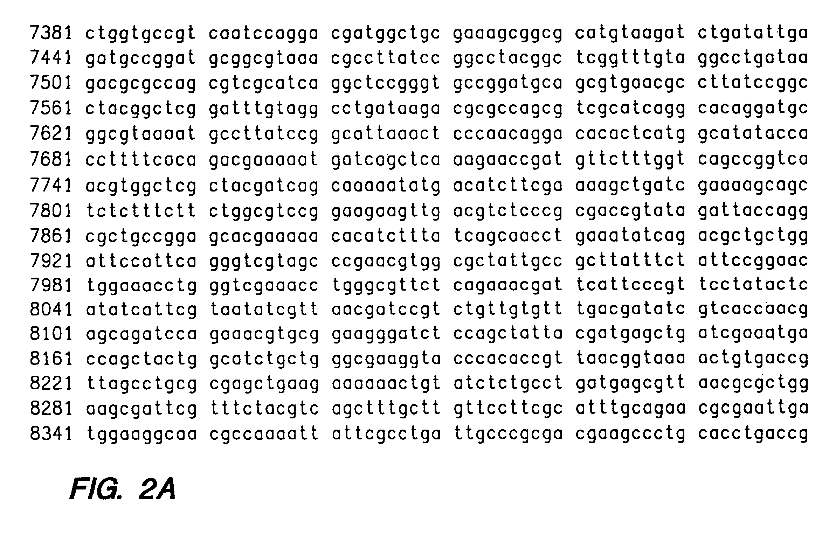

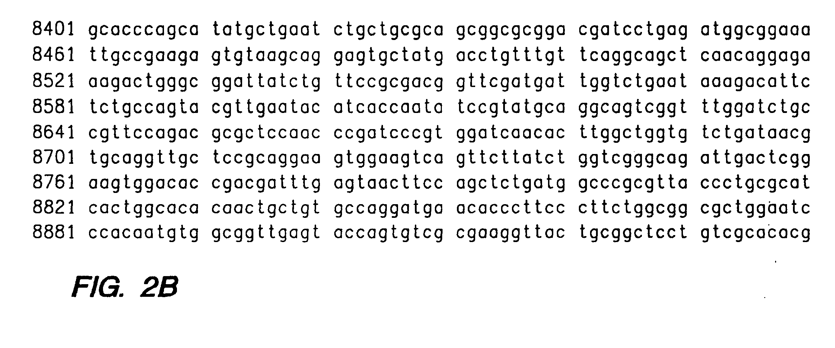

The nucleotide sequence of the *E. coli* K-12 DNA comprising the operon for the structural genes of the subunits of ribonucleotide reductase has been determined. The DNA sequence includes a total length of 8557 nucleotides. An open reading frame between nucleotides 3506 and 5834 has been identified as the nrdA gene. An open reading frame between nucleotides 6012 and 7139 encoding a 375-amino acid polypeptide has been identified as the nrdB gene (Carlson et al.[2], and Nilsson et al.[3]). The sequences of the nrdA and nrdB genes for *E. coli* are shown in Figures 1 and 2.

In *E. coli,* the synthesis of ribonucleotide reductase is controlled at the level of transcription. The nrdA and nrdB genes direct the synthesis of a 3.2 kilobase polycistronic mRNA. Perturbations in DNA replication, either a shift up in growth conditions or an inhibition of DNA synthesis leads to increased synthesis of nrd mRNA (Carlson et al.[2]).

A separate anaerobic ribonucleotide reductase has also been identified from *E.coli.* The anaerobic *E. coli* reductase has a molecular mass of 145 kD and is a homodimer. The gene for the anaerobic reductase (nrdD) has been cloned and sequenced (P. Reichard[4]).

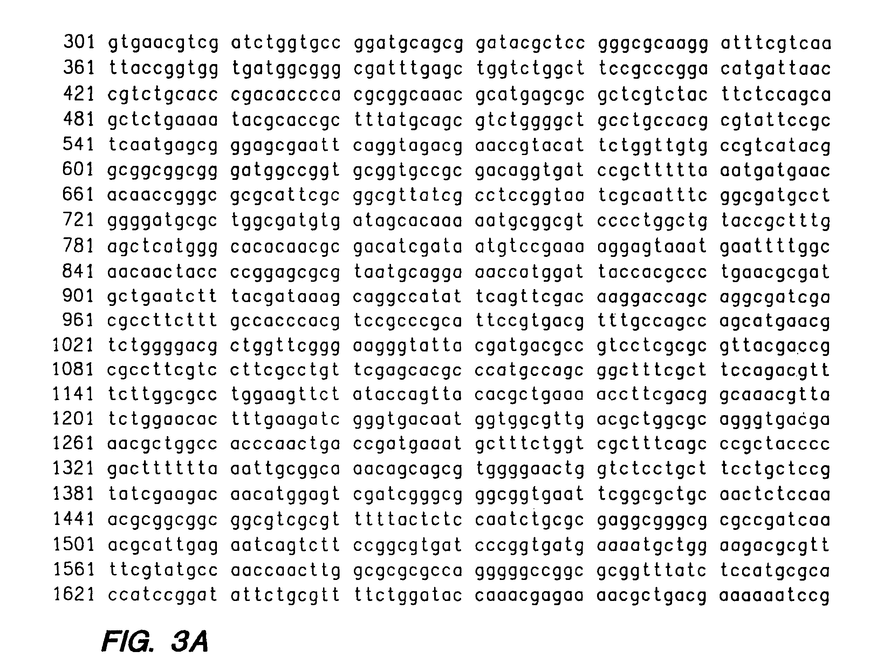

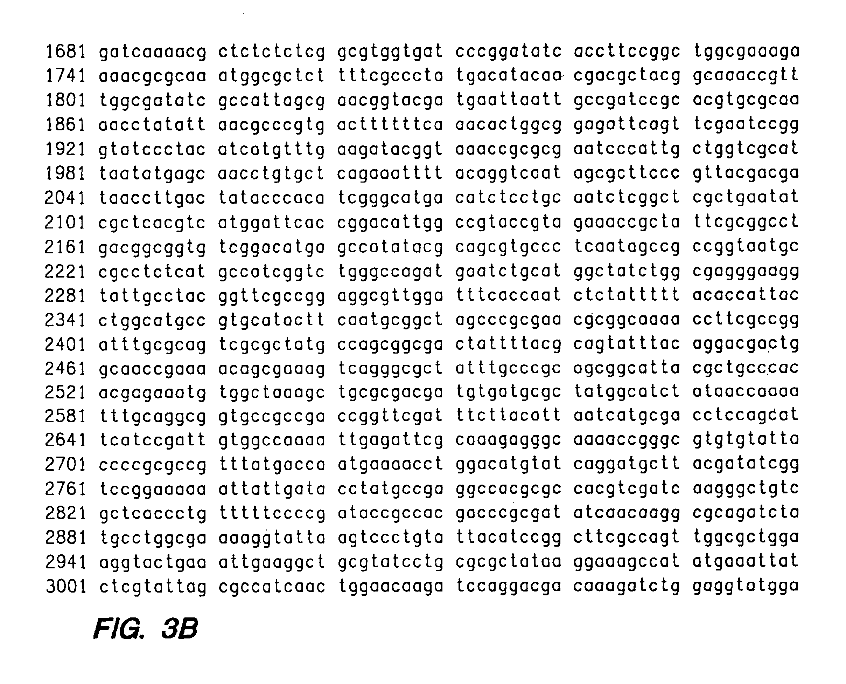

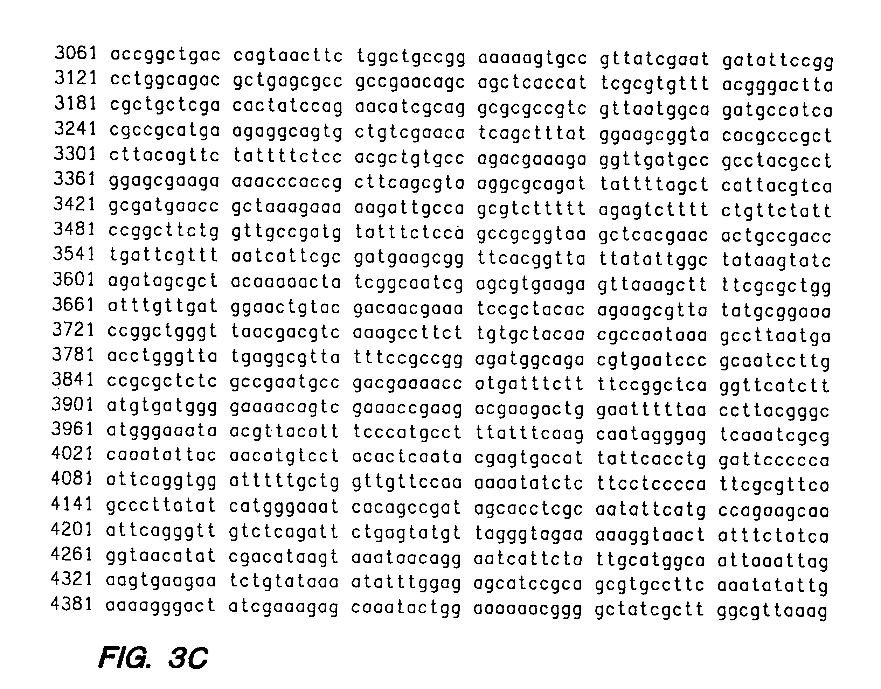

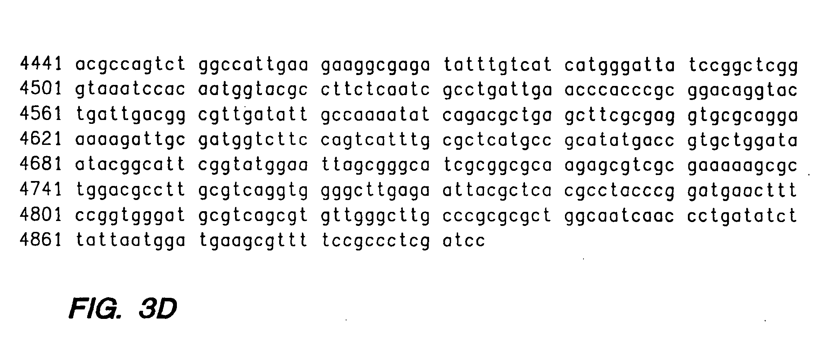

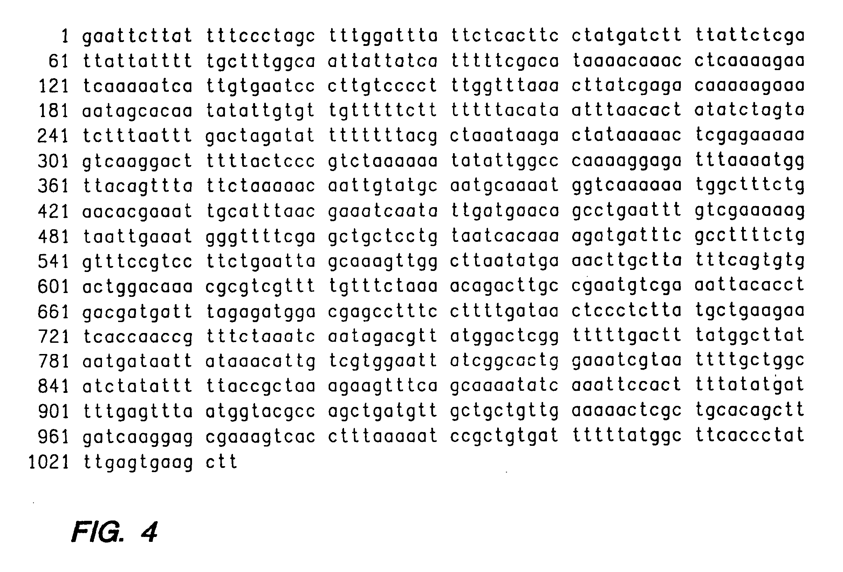

The ribonucleotide reductase R2 genomic or cDNA sequences are known for several other species such as bacteriophage T4, clam, mouse, *Saccharomyces cerevisiae,* vaccinia, herpes simplex virus types 1 and 2, varicella and Epstein-Barr virus (Nordlund et al.[5]). The sequence of the nrdE and nrdF which code for the ribonucleotide reductase genes of *S. typhimurium* are shown in Figure 3. The sequence of the ribonucleotide reductase gene of *Lactococcus lactis* is shown in Figure 4.

The secA gene of *E. coli* encodes for one component of a multi-component system for the secretion of proteins across the inner membrane of *E. coli* (der Blaauwen et al.[6]). The complete system consists of the SecB protein, a cytosolic chaperone, the SecA protein, the translocation ATPase and the heterotrimeric integral membrane SecY/SecE/SecG complex, which along with SecA serves as the preprotein channel. SecA protein plays a central role in the secretion process by binding the preprotein, secB protein, anionic phospholipids and SecY/SecE/SecG protein. These interactions allow SecA to recognize soluble preprotein and recruit it to translocation sites in the inner membrane. Once such protein translocation complexes have assembled; further steps require an ATP-driven cycle of insertion and de-insertion of secA protein with the inner membrane, where each cycle appears to be coupled to the translocation of a segment of the preprotein.

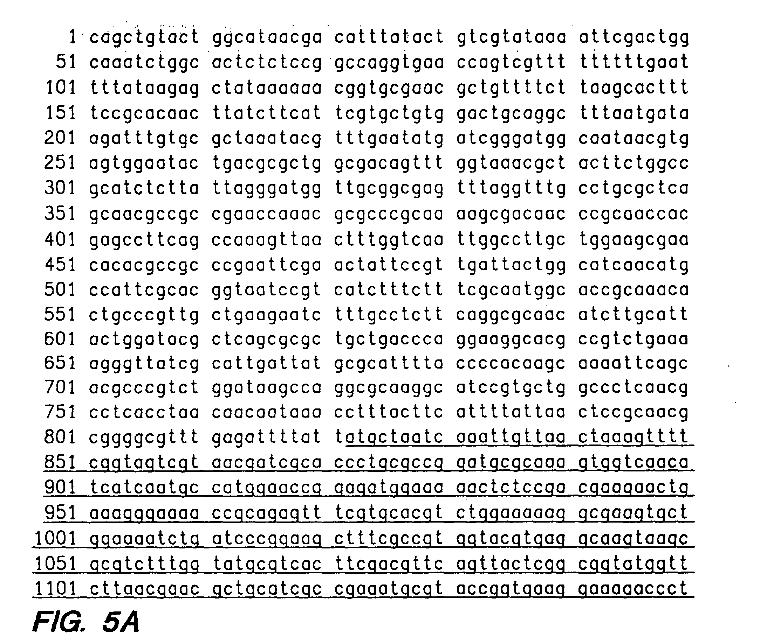

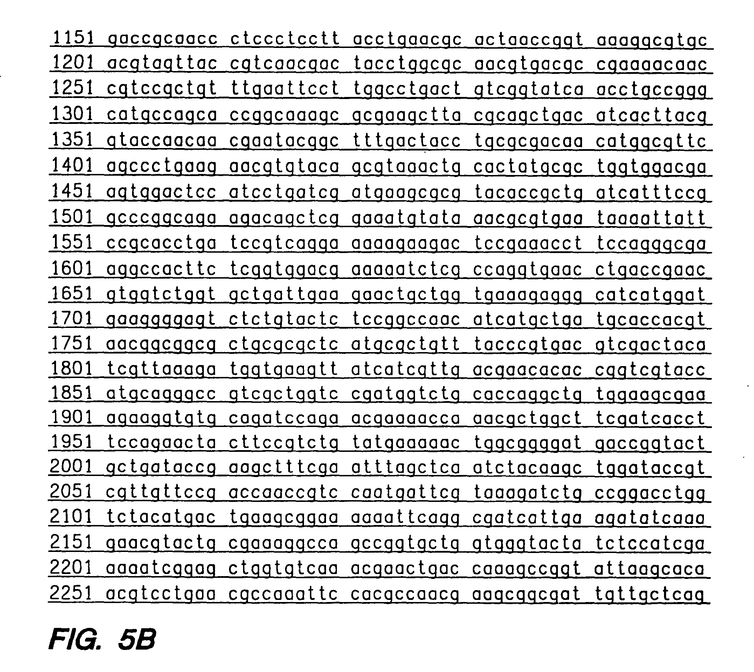

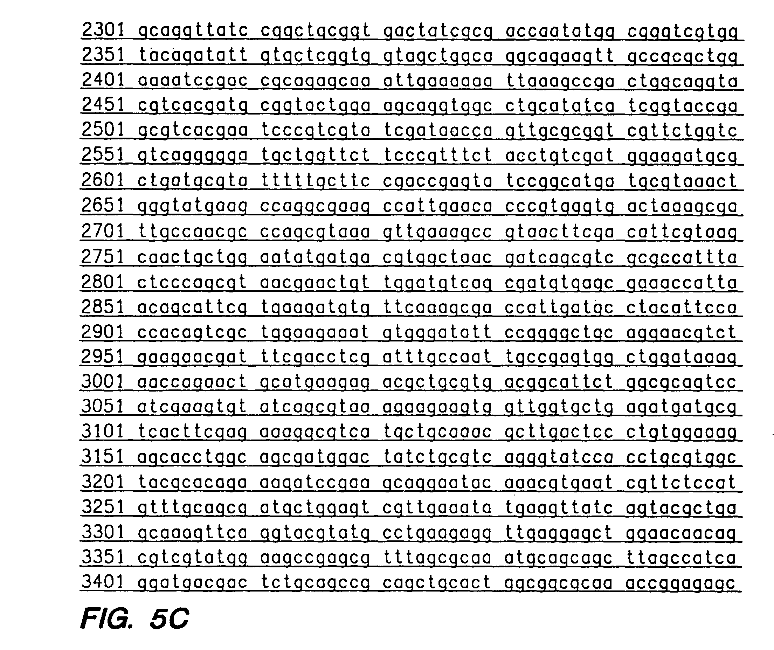

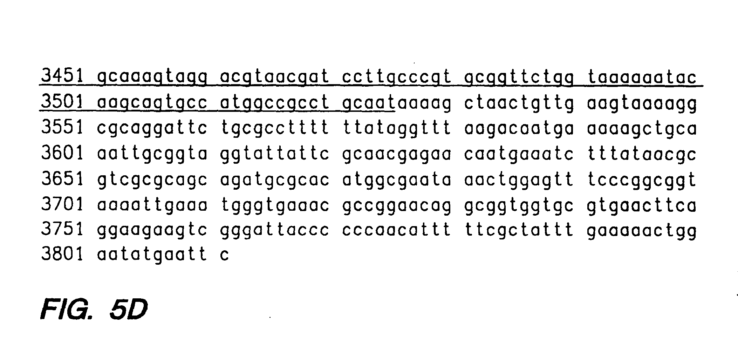

SecA is the only component of the secretion apparatus that has been shown to be regulated. SecA is the second gene in the geneX-secA operon and its translation varies over a tenfold range depending on the status of protein secretion in the cell. During protein-export proficient conditions secA auto-represses its translation by binding to a site that overlaps the secA ribosome-binding site of genes-secA RNA. SecA protein can also dissociate a preformed 30 S-$tRNA^{MET}$-genes-secA RNA ternary complex in vitro. However, during a protein export block secA translation increases substantially although the mechanism responsible for this regulatory response has not been elucidated (McNicholas et al.[7]). The sequence of the secA gene of *E. coli* is shown in Figure 5.

The secA gene sequence has been identified for a number of other species including *Mycobacterium bovis* (Figure 6), *Mycobacterium tuberculosis* (Figure 7), *Staphylococcus aureus* (Figure 8), *Staphylococcus carnosus* (Figure 9), *Bacillus subtilis, Bacillus firmus*, *Listeria monocytogenes, Mycobacterium smegmatis, Borrelia burgdorferi*, *P. sativum, S. griseus*, and *Synechoccus sp.*

Antibiotics are important pharmaceuticals for the treatment of infectious diseases in a variety of animals including man. The tremendous utility and efficacy of antibiotics results from the interruption of bacterial (prokaryotic) cell growth with minimal damage or side effects to the eukaryotic host harboring the pathogenic organisms. In general, antibiotics destroy bacteria by interfering with the DNA replication, DNA to RNA transcription, translation (that is RNA to protein) or cell wall synthesis.

Although bacterial antibiotic resistance has been recognized since the advent of antimicrobial agents, the consequence of the emergence of resistant microorganisms, such resistance was historically controlled by the continued availability of effective alternative drugs. Now, drug resistance has emerged as a serious medical problem in the community, leading to increasing morbidity and mortality. The problem is worsened by the growing number of pathogens resistant to multiple, structurally unrelated drugs. The situation has become so desperate that antibiotics once removed from use because of toxic effects may be prescribed in an attempt to deal with the otherwise untreatable drug resistant bacteria.

Antisense oligonucleotides have been used to decrease the expression of specific genes by inhibiting transcription or translation of the desired gene and thereby achieving a phenotypic effect based upon the expression of that gene (Wright and Anazado[38]). For example, antisense RNA is important in plasmid DNA copy number control, in development of bacteriophage P22. Antisense RNA's have been used experimentally to specifically inhibit *in vitro* translation of mRNA coding specifically from Drosophila hsp23, to inhibit Rous sarcoma virus replication and to inhibit 3T3 cell proliferation when directed toward the oncogene c-fos. Furthermore, it is not necessary to use the entire antisense mRNA since a short antisense oligonucleotide can inhibit gene expression. This is seen in the inhibition of chloramphenicol acetyltransferase gene expression and in the inhibition of specific antiviral activity to vesicular stomatitus virus by inhibiting the N-protein initiation site. Antisense oligonucleotides directed to the macromolecular synthesis operon of bacteria, containing the rpsU gene, the rpoD gene and the dnaG gene have been used for the detection of bacteria. (U.S. Patent No. 5,294,533[8]). Furthermore, photoactivatable antisense DNA complementary to a segment of the β-lactamase gene has been used to suppress ampicillin resistance in normally resistant *E. coli* (Gasparro et al.[9]). Antisense DNA analogs have also been used to inhibit the multiple antibiotic resistant (mar) operon in *Escherichia coli* (White et al.[10]).

Accordingly, there is a need to develop antisense oligonucleotides which will act to inhibit the growth of microorganisms.

## SUMMARY OF THE INVENTION

This invention is directed to antisense oligonucleotides which modulate the expression of the ribonucleotide reductase and secA genes in microorganisms and pharmaceutical compositions comprising such antisense oligonucleotides. This invention is also related to methods of using such antisense oligonucleotides for inhibiting the growth of microorganisms.

Accordingly, in one of its composition aspects, this invention is directed to an antisense oligonucleotide, which oligonucleotide is nuclease resistant and comprises from about 3 to about 50 nucleotides, which nucleotides are complementary to the ribonucleotide reductase gene or the secA gene of a microorganism. The antisense oligonucleotide may have one or more phosphorothioate intemucleotide linkages.

In another of its composition aspects, this invention is directed to an antisense oligonucleotide comprising from about 3 to about 50 nucleotides which is capable of binding to the ribonucleotide reductase gene or the secA gene of a microorganism, wherein the oligonucleotide comprises all or part of a sequence selected from the group consisting of SEQ ID NO:22; SEQ ID NO:43; SEQ ID NO:62; SEQ ID NO:74; SEQ ID NO:75; SEQ ID NO:76; SEQ ID NO:143; SEQ ID NO:145; SEQ ID NO:152; SEQ ID NO:164; SEQ ID NO:176; SEQ ID NO:186; SEQ ID NO:188; SEQ ID NO:189; SEQ ID NO:191; SEQ ID NO:192; SEQ ID NO:195; SEQ ID NO:197; SEQ ID NO:206; SEQ ID NO:212; SEQ ID NO:220; SEQ ID NO:229; SEQ ID NO:235; SEQ ID NO:254; SEQ ID NO:261; SEQ ID NO:262; SEQ ID NO: 263; SEQ ID NO:264; and SEQ ID NO:265.

In still another of its composition aspects, this invention is directed to a pharmaceutical composition comprising a pharmaceutically acceptable excipient and an effective amount of an antisense oligonucleotide, which oligonucleotide is nuclease resistant and comprises from about 3 to about 50 nucleotides, which nucleotides are complementary to the ribonucleotide reductase gene or the secA gene of a microorganism. The oligonucleotide may be modified, for example, the oligonucleotide may have one or more phosphorothioate intemucleotide linkages.

In one of its method aspects, this invention is directed to a method for inhibiting the expression of the ribonucleotide reductase gene in a microorganism having a ribonucleotide reductase gene comprising, administering to said microorganism or to a cell infected with said microorganism an effective amount of an antisense oligonucleotide comprising from at least about 3 nucleotides which are complementary to the ribonucleotide reductase gene of the microorganism under conditions such that the expression of the ribonucleotide reductase gene is inhibited.

In another of its method aspects, this invention is directed to a method for inhibiting the expression of the secA gene in a microorganism having a secA gene, comprising administering to said microorganism an effective amount of an antisense oligonucleotide comprising from at least about 3 nucleotides which are complementary to the secA gene of the microorganism under conditions such that expression of the secA gene is inhibited.

In one of its method aspects, this invention is directed to a method for inhibiting the growth of a microorganism encoding a ribonucleotide reductase gene or a secA gene, which method comprises administering to said microorganism or a cell infected with said microorganism an effective amount of an antisense oligonucleotide comprising from at least about 3 nucleotides which are complementary to either the ribonucleotide reductase gene or the secA gene of the microorganism under conditions such that the growth of the microorganism is inhibited. Preferably, the antisense oligonucleotide is selected from the group consisting of SEQ ID NO:22; SEQ ID NO:43; SEQ ID NO:62; SEQ ID NO: 74; SEQ ID NO:75; SEQ ID NO:76; SEQ ID NO:143; SEQ ID NO:145; SEQ ID NO:152; SEQ ID NO:164; SEQ ID NO: 176; SEQ ID NO:186; SEQ ID NO:188; SEQ ID NO:189; SEQ ID NO:191; SEQ ID NO:192; SEQ ID NO:195; SEQ ID NO:197; SEQ ID NO:206; SEQ ID NO:212; SEQ ID NO:220; SEQ ID NO:229; SEQ ID NO:235; SEQ ID NO:254; SEQ ID NO:261; SEQ ID NO:262; SEQ ID NO:263; SEQ ID NO:264; and SEQ ID NO:265.

In another of its method aspects, this invention is directed to a method for treating a mammalian pathologic condition mediated by a microorganism, which method comprises identifying a mammal having a pathologic condition mediated by a microorganism having a ribonucleotide reductase gene or a secA gene and administering to said mammal an effective amount of an antisense oligonucleotide comprising from at least about 3 nucleotides which are complementary to either the ribonucleotide reductase gene or the secA gene of the microorganism under conditions such that the growth of the microorganism is inhibited.

## BRIEF DESCRIPTION OF THE DRAWINGS

Figure 1 is the sequence of the *E. coli* nrdA gene encoding the ribonucleotide reductase R1 subunit [SEQ ID NO:1].

Figure 2 is the sequence of the *E. coli* nrdB gene encoding the ribonucleotide reductase R2 subunit [SEQ ID NO: 2]. The nrdB gene is encoded by nucleotides 7668 to 8798 of SEQ ID NO:2.

Figure 3 is the sequence of the *S. typhimurium* nrdE and nrdF genes encoding the ribonucleotide reductase subunits [SEQ ID NO:3]. The nrdE gene is encoded by nucleotides 836 to 2980 and the nrdF gene is encoded by nucleotides 2991 to 3950 of SEQ ID NO:3.

Figure 4 is the sequence of the *Lactococcus lactis* nrdEF operon encoding ribonucleotide reductase [SEQ ID NO:4].

Figure 5 is the sequence of the *E. coli* secA gene [SEQ ID NO:5].

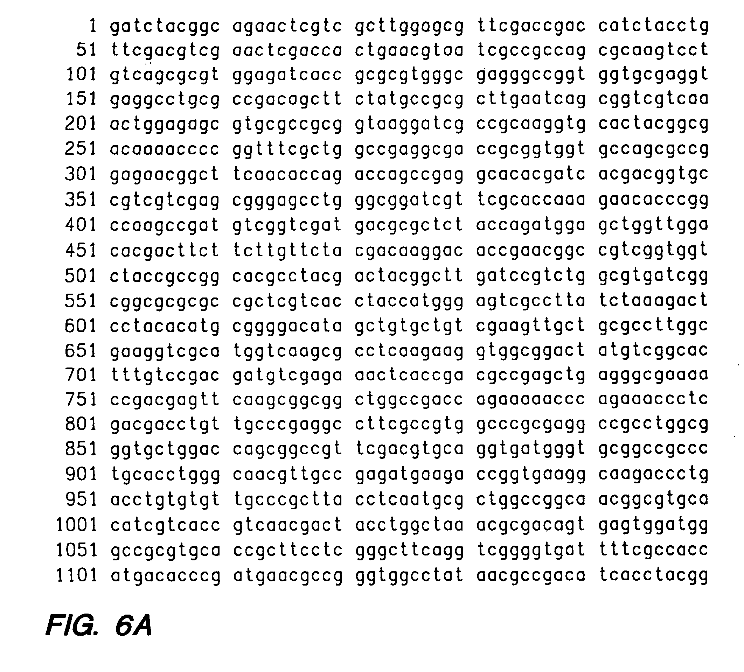

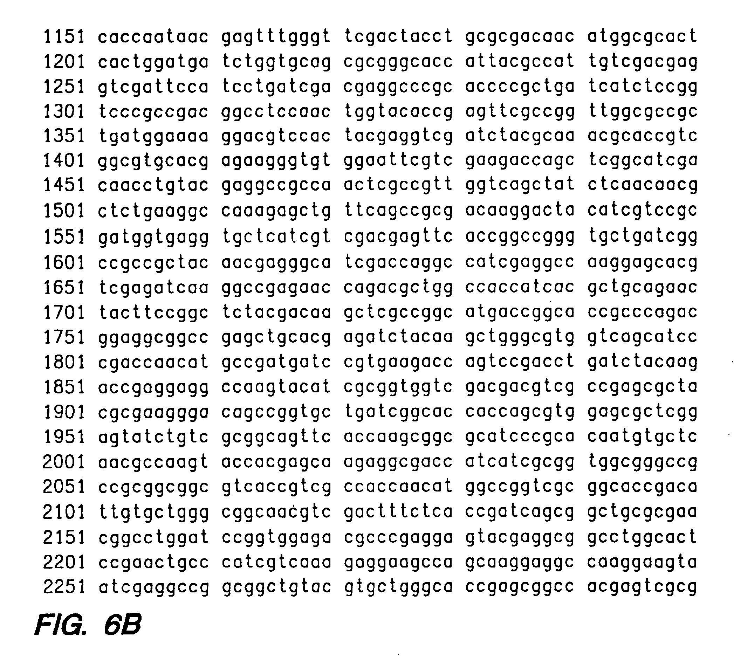

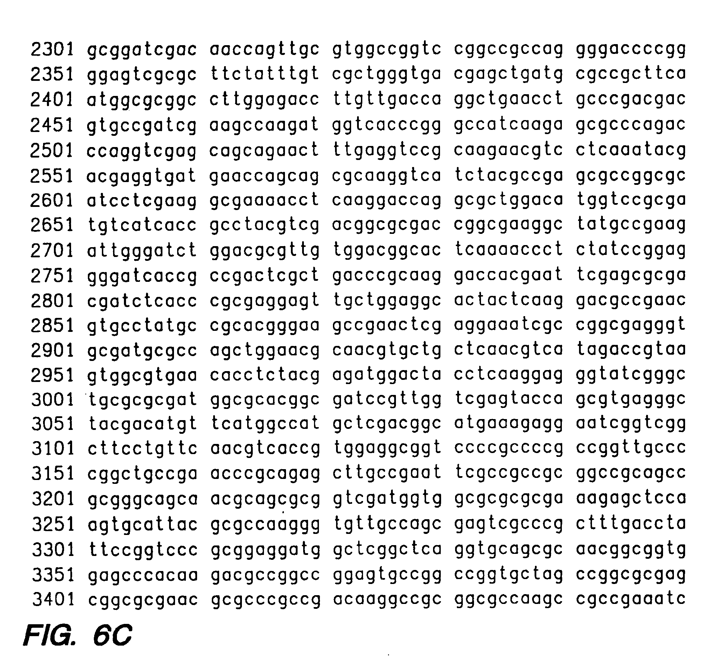

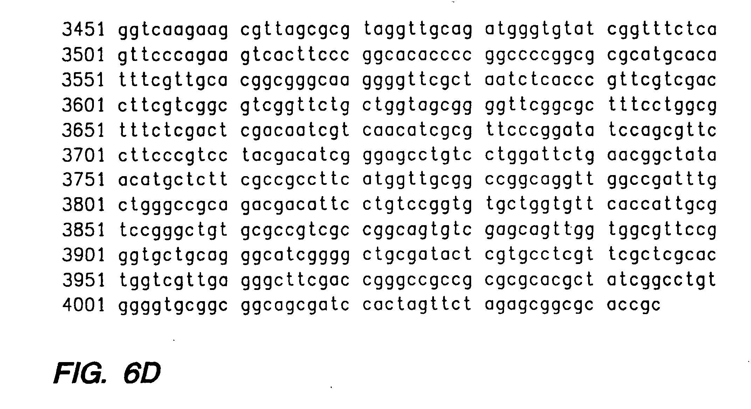

Figure 6 is the sequence of the Mycobacterium bovis secA gene [SEQ ID NO:6].

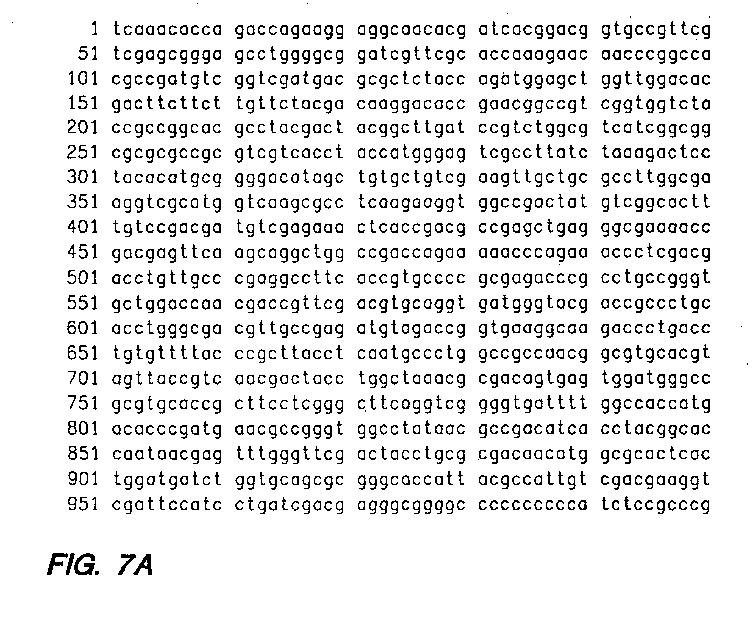

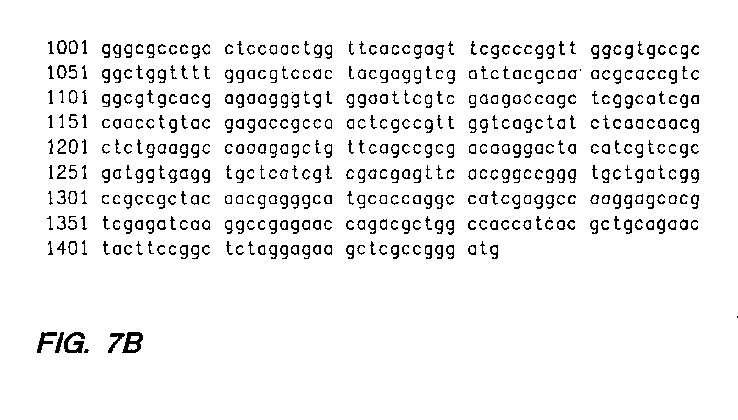

Figure 7 is the sequence of the *Mycobacterium tuberculosis* secA gene [SEQ ID NO:7].

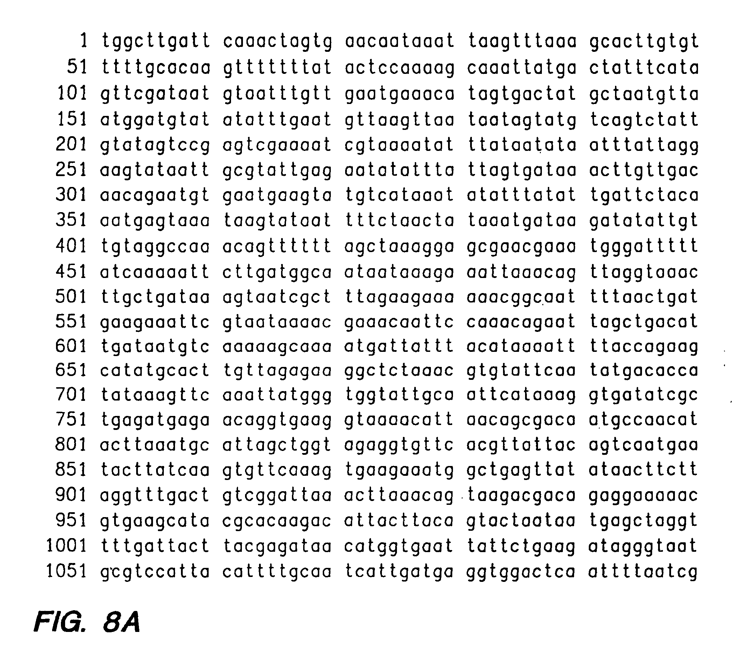

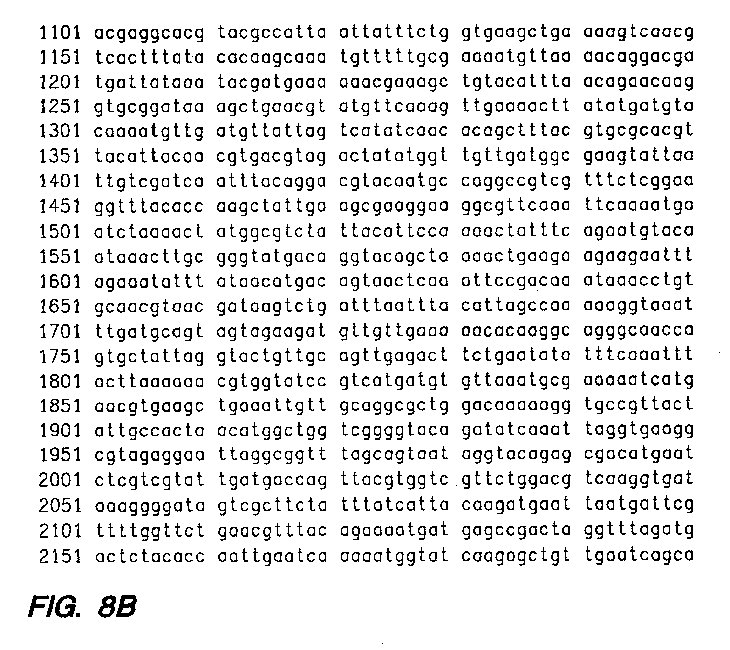

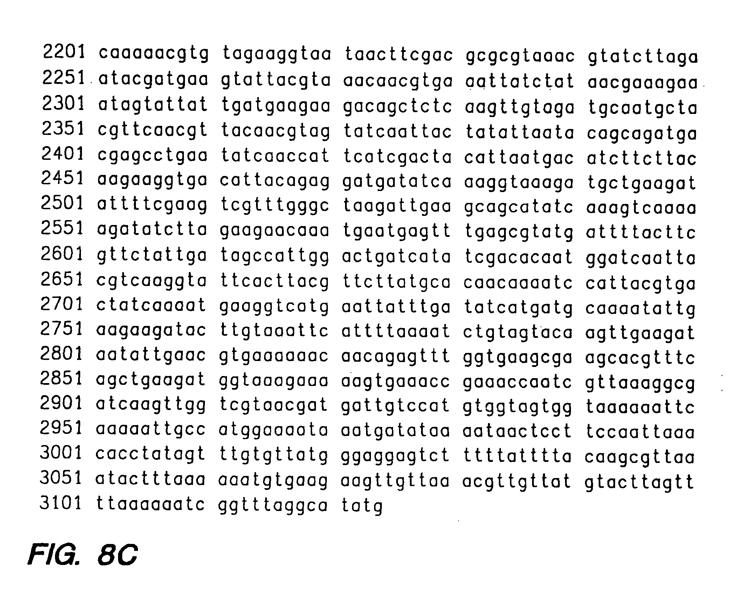

Figure 8 is the sequence of the *Staphylococcus aureus* secA gene [SEQ ID NO:8].

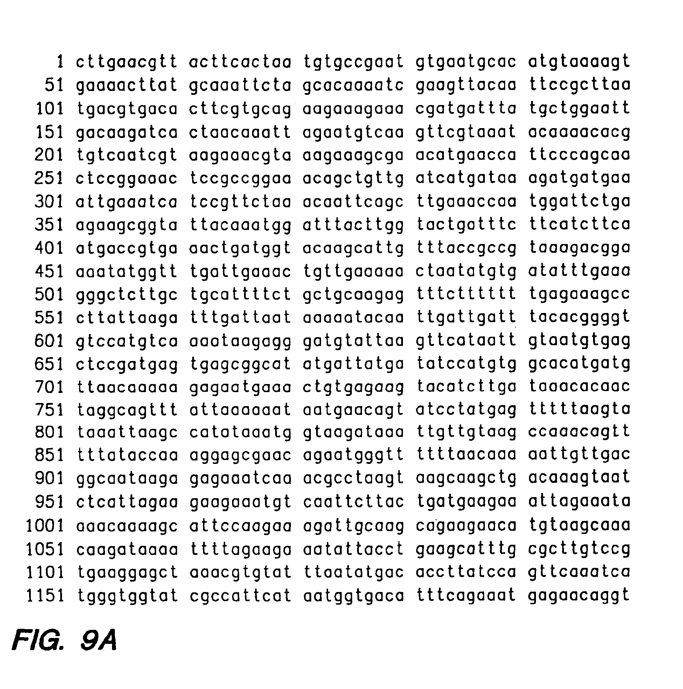

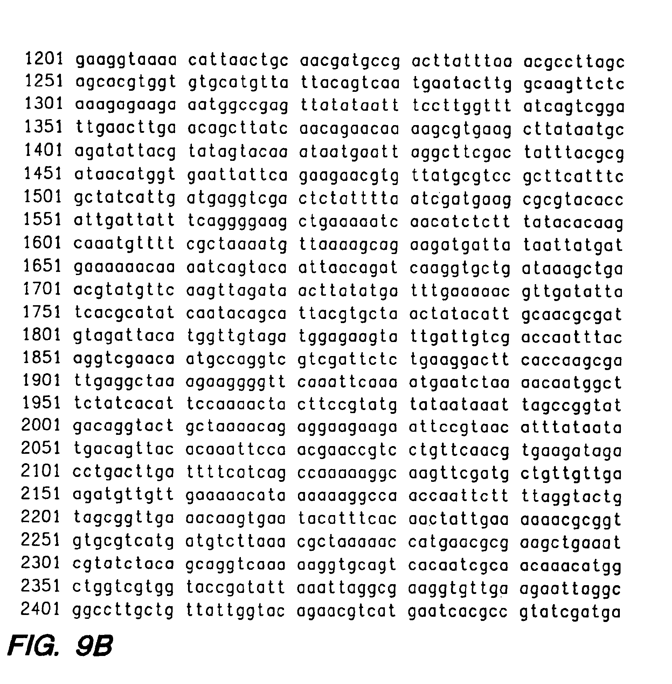

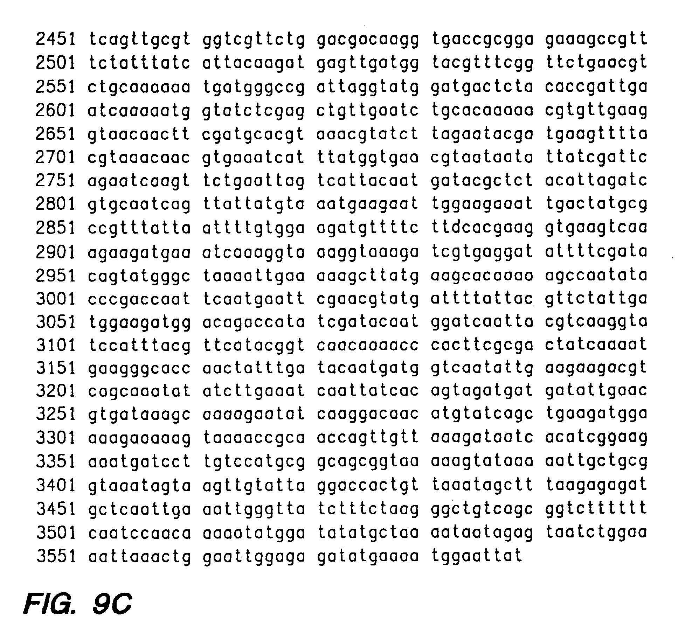

Figure 9 is the sequence of the *Staphylococcus carnosus* secA gene [SEQ ID NO:9].

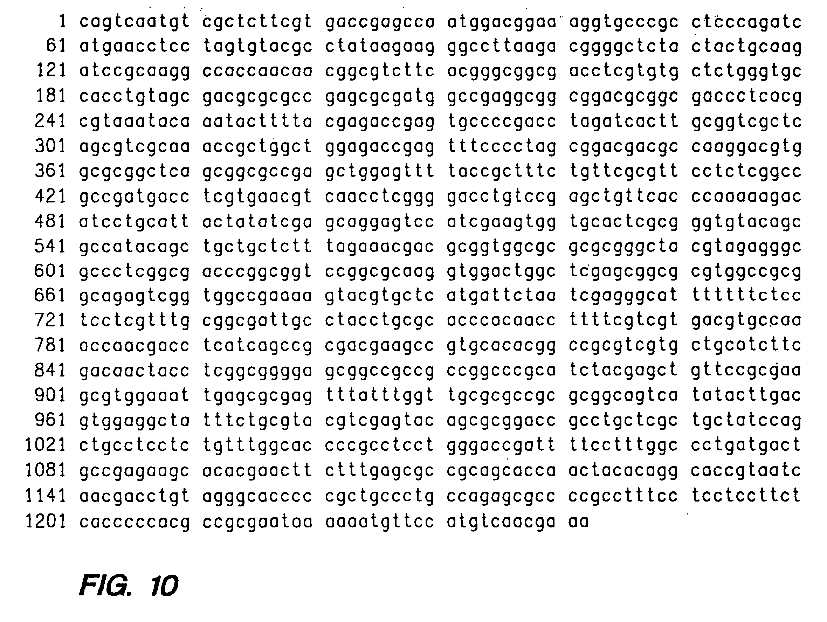

Figure 10 is the sequence of the bovine herpes virus ribonucleotide reductase small subunit gene [SEQ ID NO:10].

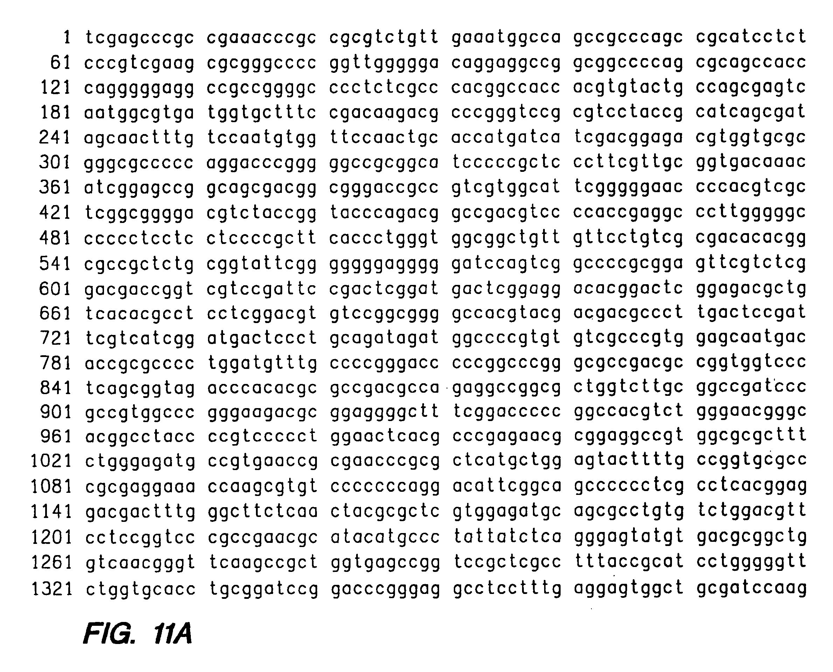

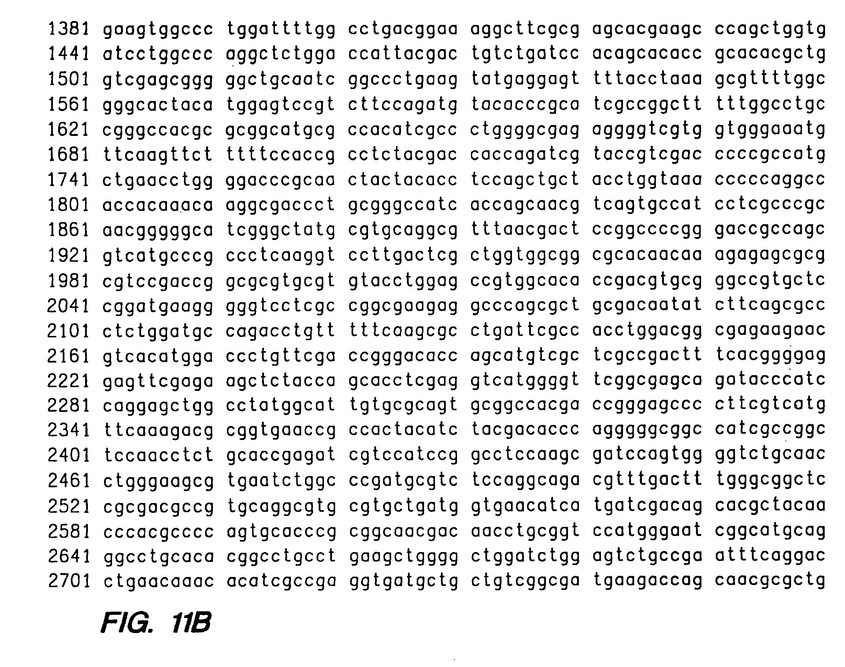

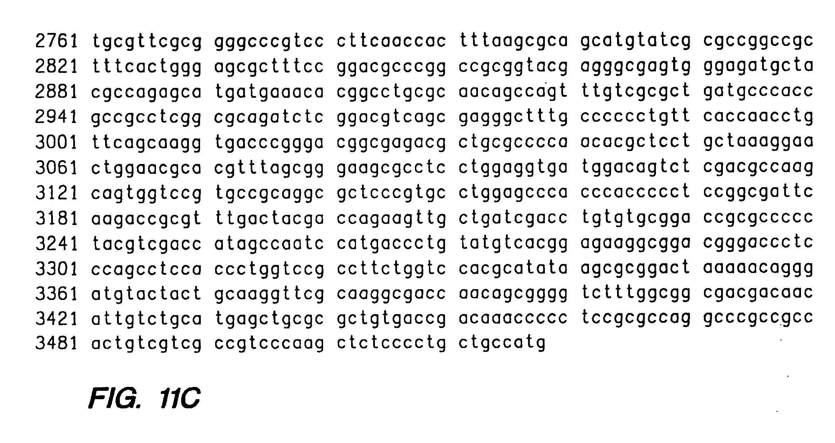

Figure 11 is the sequence of the Herpes simplex virus type 1 UL39 gene encoding ribonucleotide reductase 1 [SEQ ID NO:11].

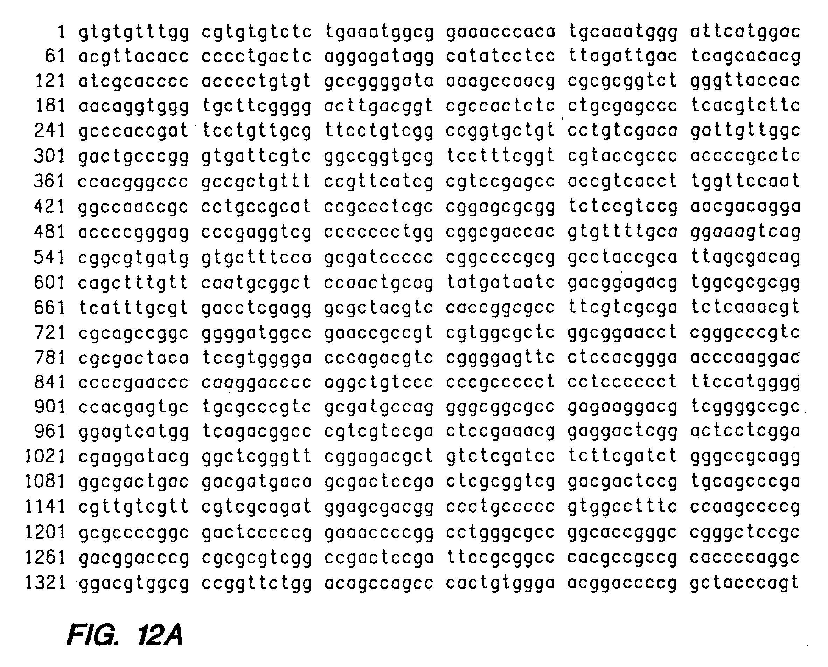

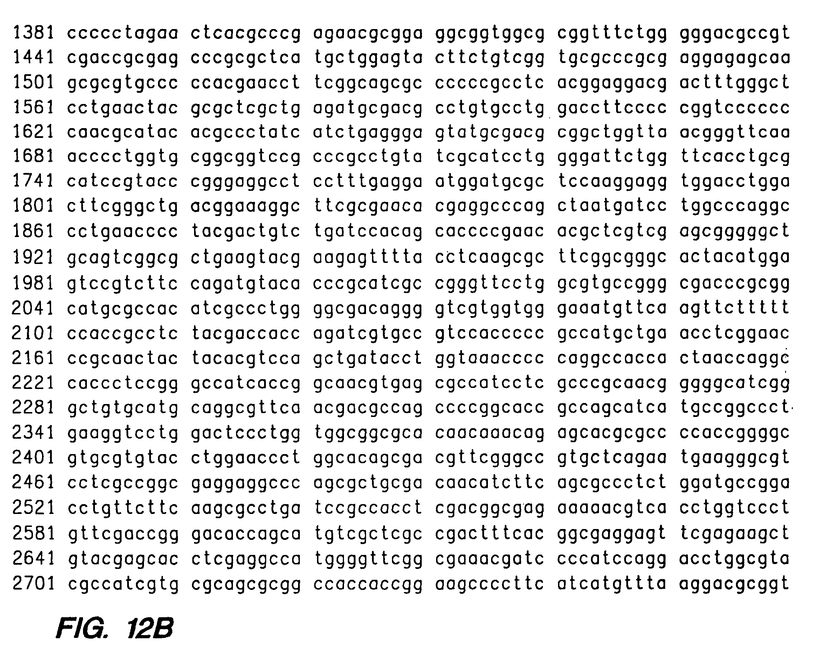

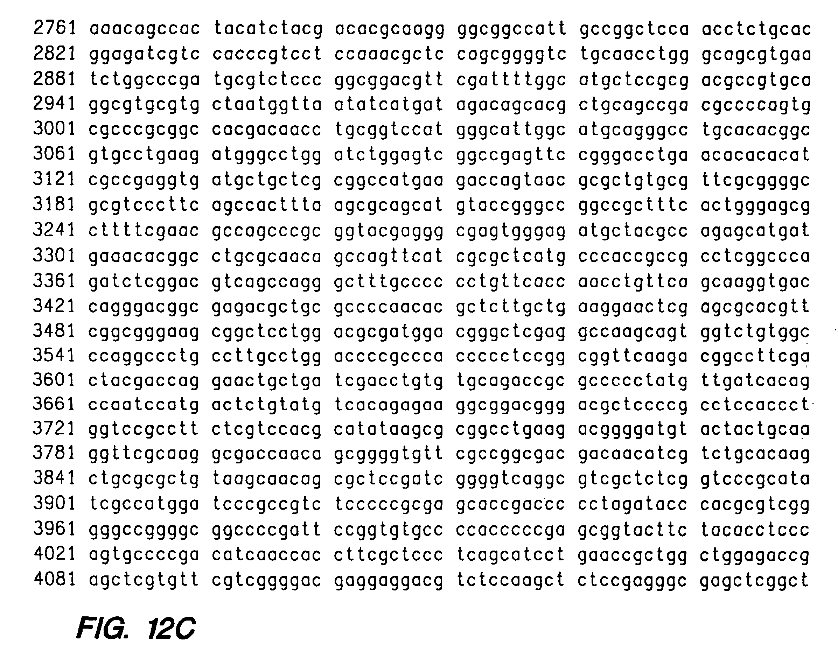

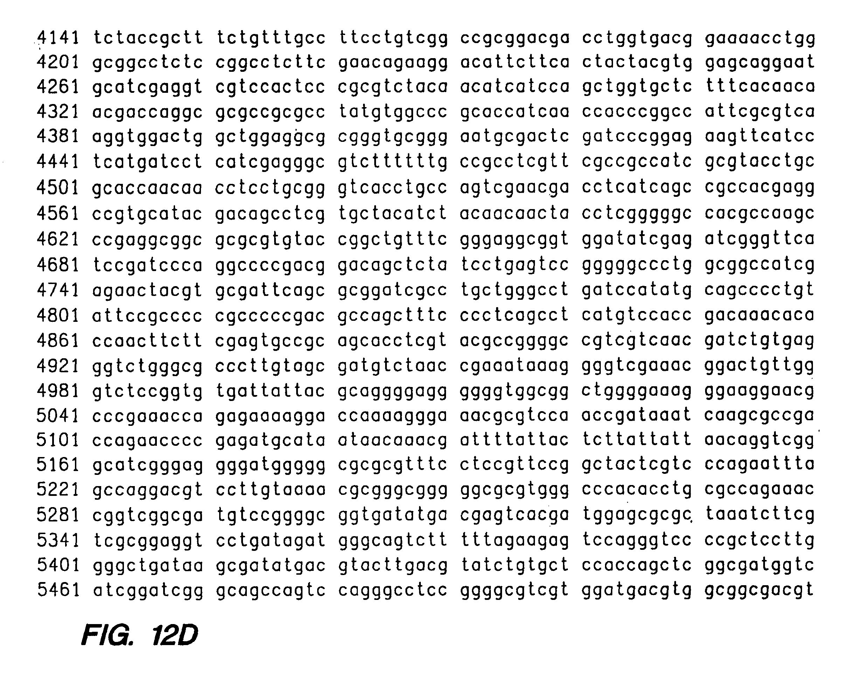

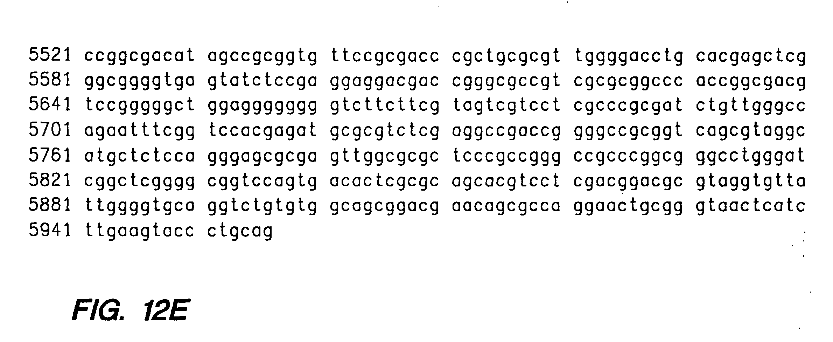

Figure 12 is the sequence of the Herpes simplex type 2 ribonucleotide reductase gene [SEQ ID NO:12]. The ribonucleotide reductase gene is encoded by nucleotides 419 to 3853 of SEQ ID NO:12.

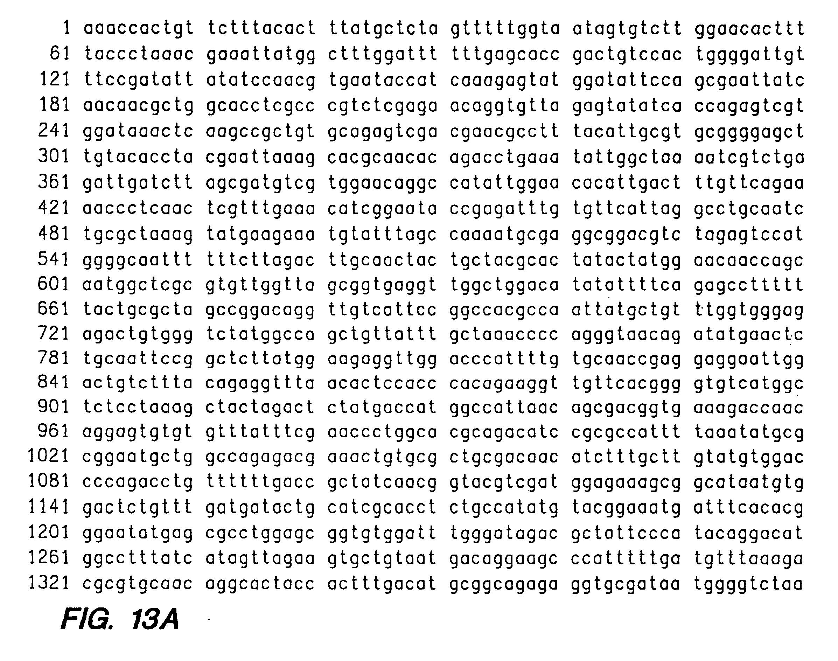

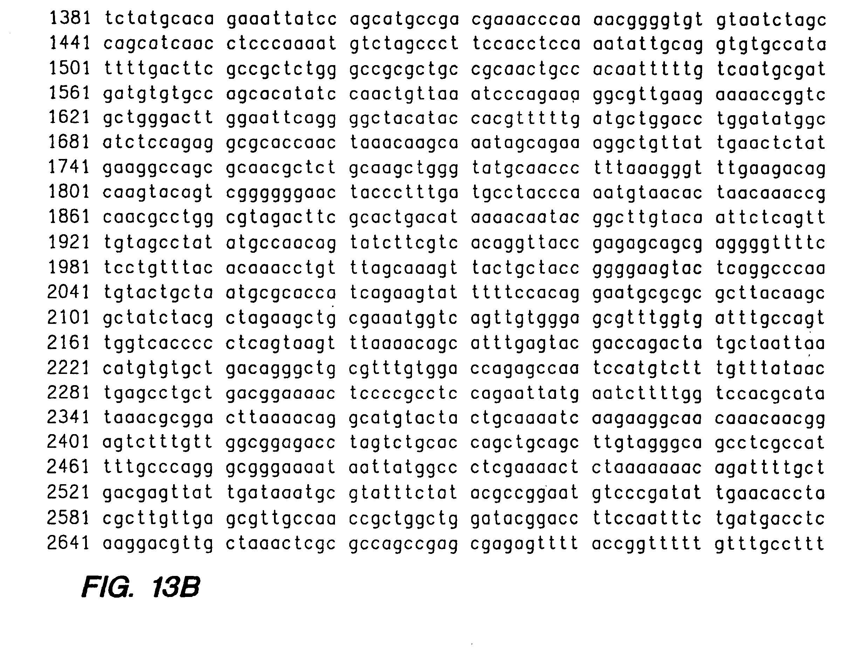

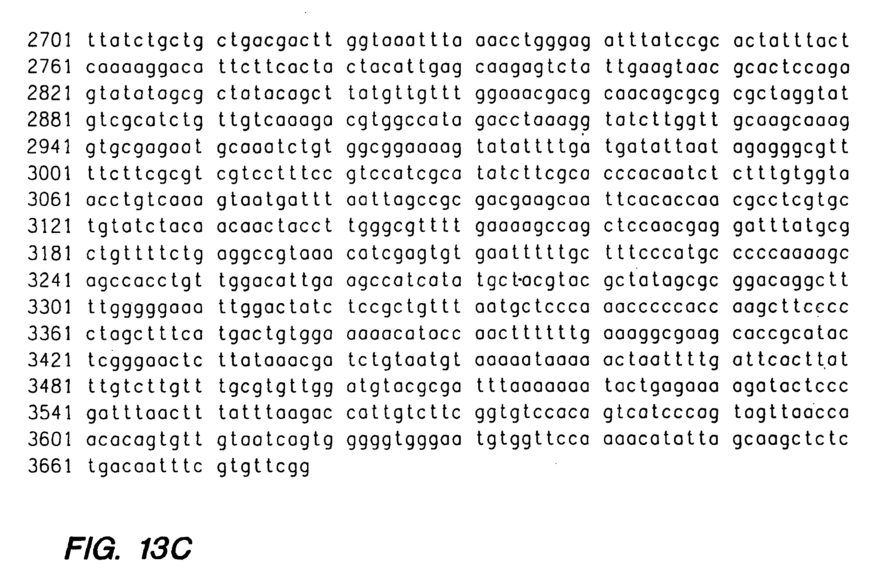

Figure 13 is the sequence of the equine herpes virus 4 ribonucleotide reductase large subunit and small subunit [SEQ ID NO:13]. The large subunit is encoded by nucleotides 77 to 2446 and the small subunit by nucleotides 2485-3447 of SEQ ID NO:13.

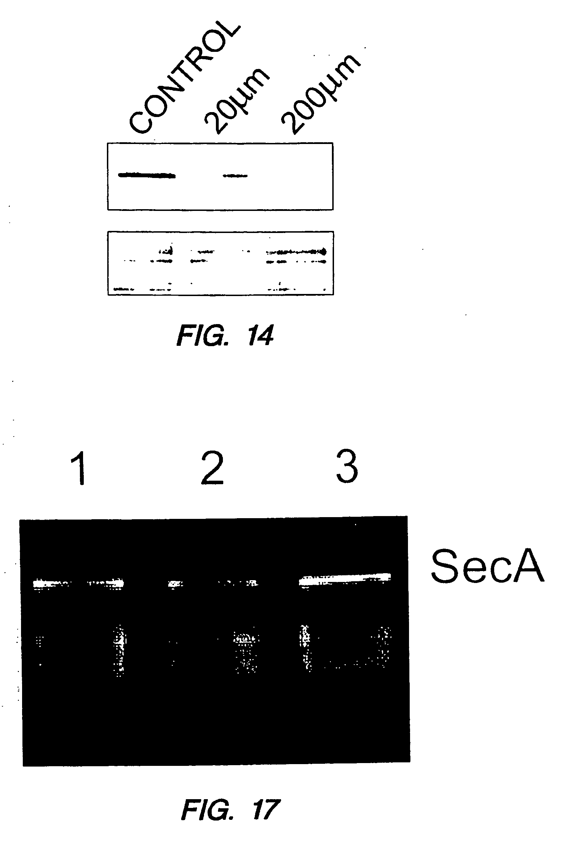

Figure 14 is a photograph of a Western blot of a polyacrylamide gel of the cellular protein from *E. coli* cells carrying a plasmid containing the mouse ribonucleotide reductase R2 gene after treatment with either 20μM or 200 μM of oligonucleotide AS-II-626-20.

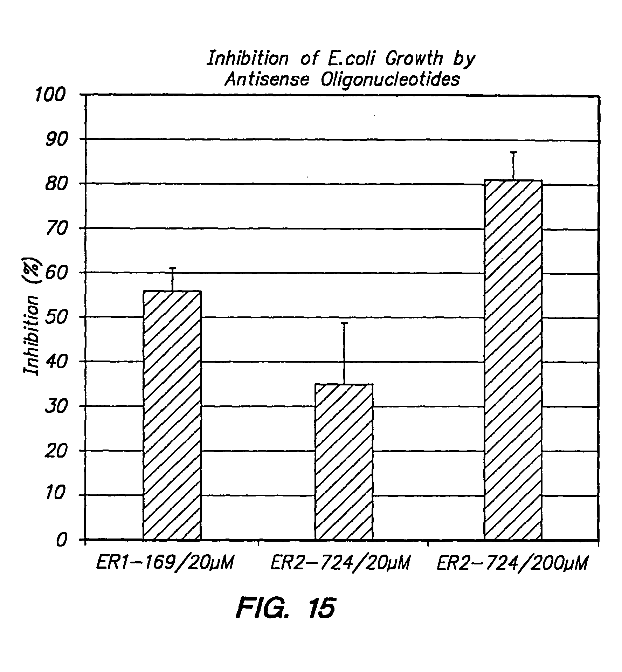

Figure 15 is a graph of the inhibition of *E. coli* growth after treatment of *E. coli* cells with ribonculeotide reductase antisense oligonucleotides.

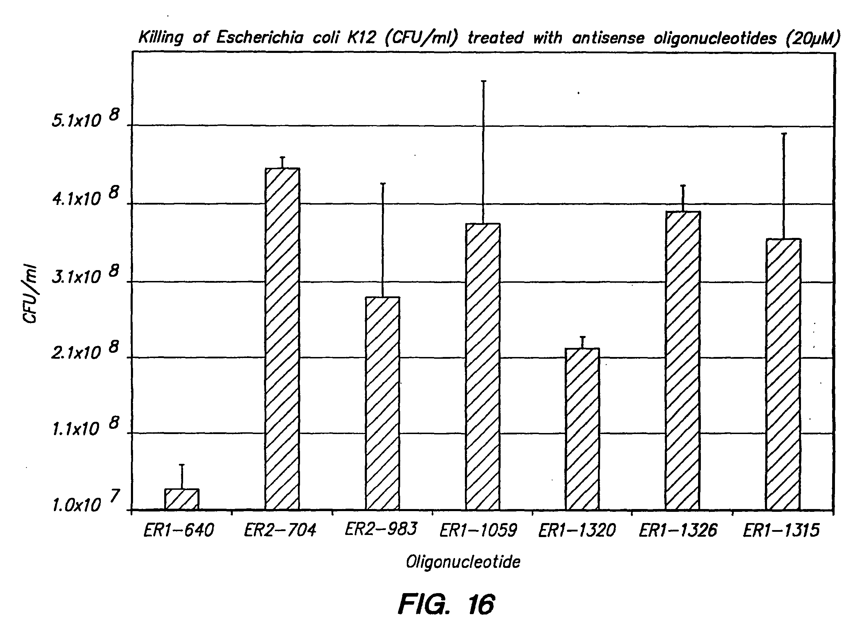

Figure 16 is a graph of the number of colony forming units/ml of *E. coli* cells after treatment with ribonucleotide reductase antisense oligonucleotides.

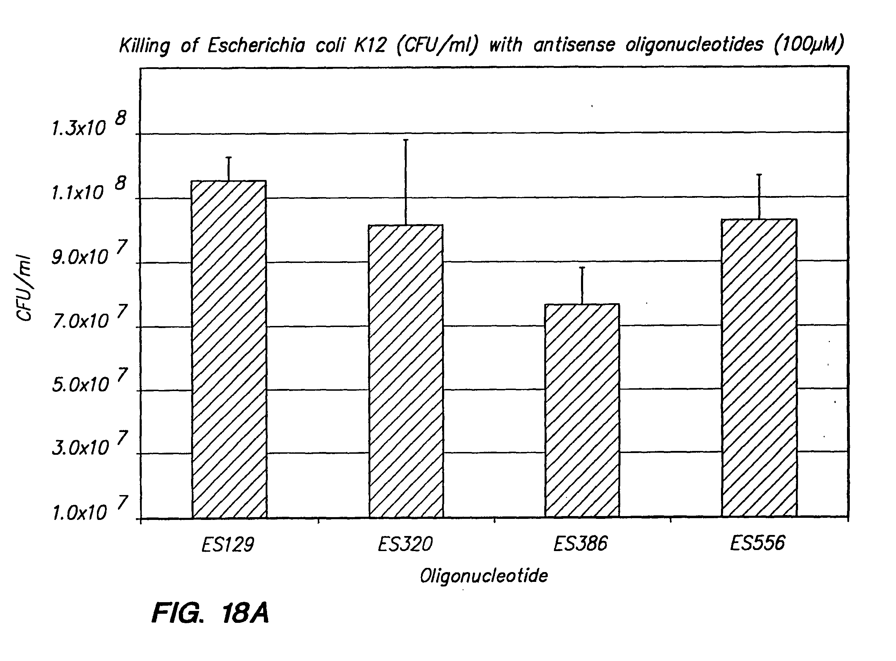

Figure 17 is a photograph of a Western blot of a polyacrylamide gel of cellular protein from *E. coli* cells after treatment with secA antisense oligonucleotides.

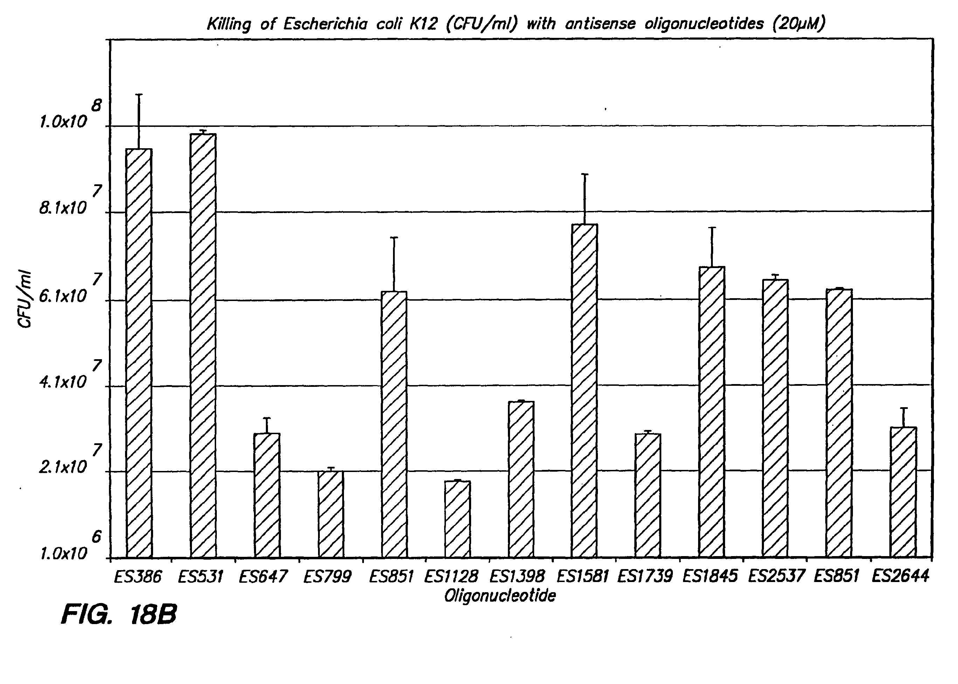

Figures 18a and 18b are graphs of the number of colony forming units/ml of *E. coli* cells after treatment with secA antisense oligonucleotides.

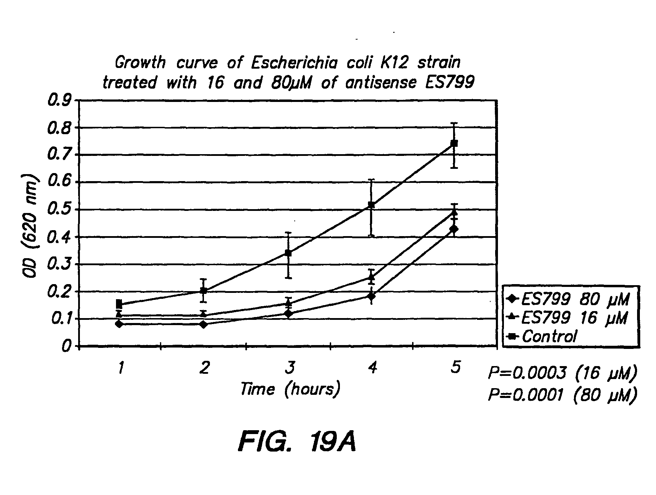

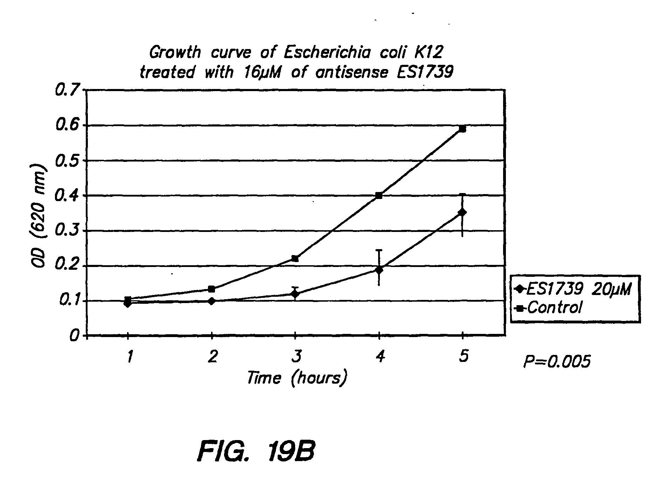

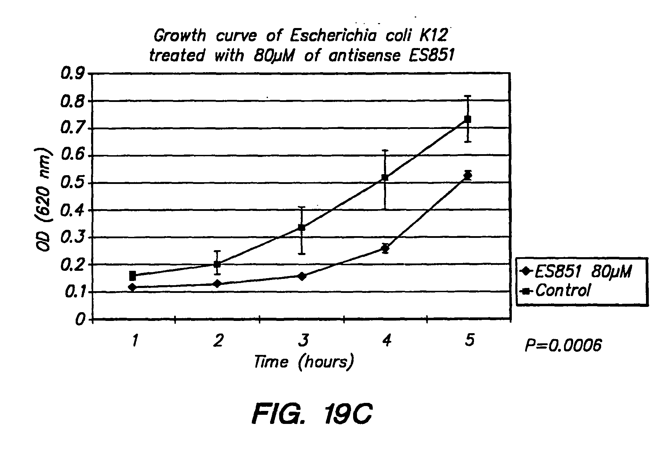

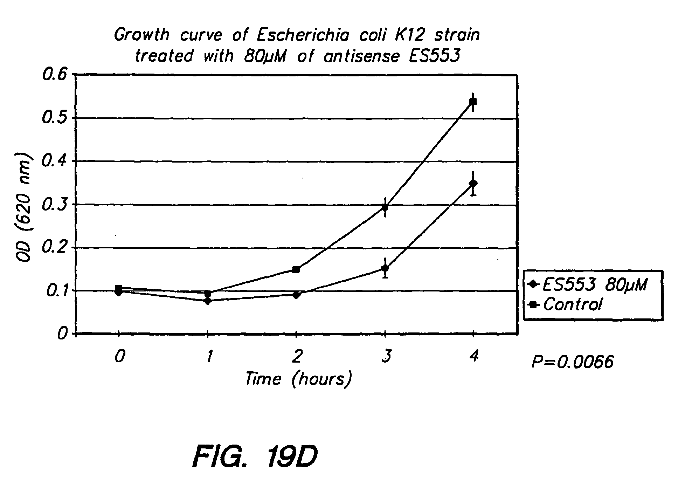

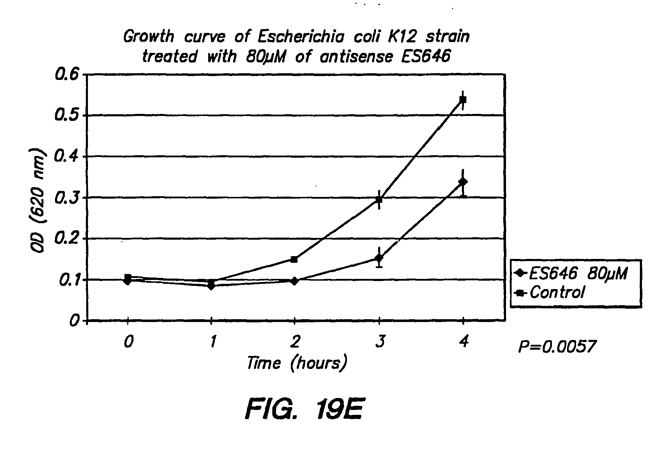

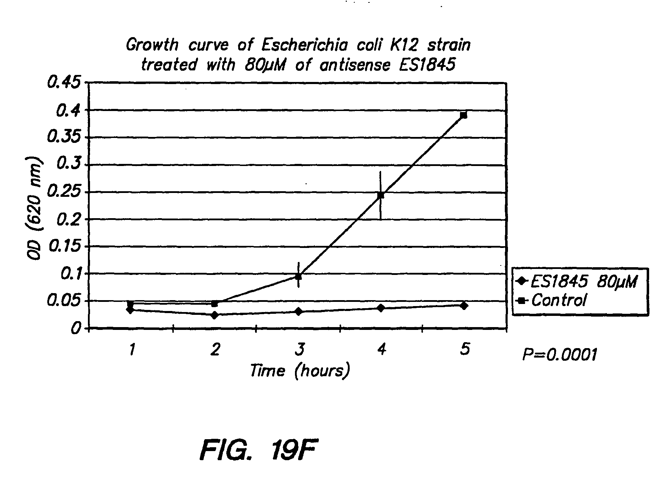

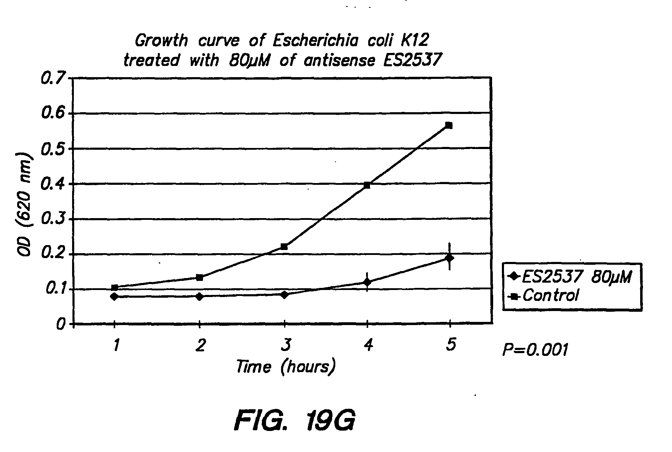

Figures 19a-g are graphs of growth curves of *E. coli* K12 after treatment with antisense oligonucleotides. Figure 19a shows the growth after treatment with 16 μM or 80 μM of antisense ES799 [SEQ ID NO:195]. Figure 19b shows the growth after treatment with 20 μM of antisense ES1739 [SEQ ID NO:229]. Figure 19c shows the growth after treatment with 80 μM of antisense ES851 [SEQ ID NO:197]. Figure 19d shows the growth after treatment with 80 μM of antisense ES553 [SEQ ID NO:188]. Figure 19e shows the growth after treatment with 80 μM of antisense ES646 [SEQ ID NO:191]. Figure 19f shows the growth after treatment with 80 μM of antisense ES1845 [SEQ ID NO:235]. Figure 19g shows the growth after treatment with 80 μM of antisense ES2537 [SEQ ID NO:254].

## DETAILED DESCRIPTION OF THE INVENTION

The present invention provides compounds that inhibit the growth of microbes by inhibiting the expression of a ribonucleotide reductase protein or the secA protein. Without being limited to any theory, the compounds inhibit the expression of the ribonucleotide reductase or the secA protein by inhibiting the transcription of the gene or the translation of the mRNA to protein. Such compounds include antisense oligonucleotides.

Definitions:

As used herein, the following terms have the following meanings:

The term "antisense oligonucleotide" as used herein means a nucleotide sequence that is complementary to the mRNA for the desired gene. Preferably, the antisense oligonucleotide is complementary to the mRNA for ribonucleotide reductase or secA.

The term "oligonucleotide" refers to an oligomer or polymer of nucleotide or nucleoside monomers consisting of naturally occurring bases, sugars, and inter-sugar (backbone) linkages. The term also includes modified or substituted oligomers comprising non-naturally occurring monomers or portions thereof, which function similarly. Such modified or substituted oligomers may be preferred over naturally occurring forms because of the properties such as enhanced cellular uptake, or increased stability in the presence of nucleases. The term also includes chimeric oligonucleotides which contain two or more chemically distinct regions. For example, chimeric oligonucleotides may contain at least one region of modified nucleotides that confer beneficial properties (e.g. increased nuclease resistance, increased uptake into cells) or two or more oligonucleotides of the invention may be joined to form a chimeric oligonucleotide.

The antisense oligonucleotides of the present invention may be ribonucleic or deoxyribonucleic acids and may contain naturally occurring or synthetic monomeric bases, including adenine, guanine, cytosine, thymine and uracil. The oligonucleotides may also contain modified bases such as xanthine, hypoxanthine, 2-aminoadenine, 6-methyl, 2-propyl and other alkyl adenines, 5-halo uracil, 5-halo cytosine, 6-aza uracil, 6-aza cytosine and 6-aza thymine, pseudo uracil, 4-thiouracil, 8-halo adenine, 8-aminoadenine, 8-thiol adenine, 8-thiolalkyl adenines, 8-hydroxyl adenine and other 8-substituted adenines, 8-halo guanines, 8-amino guanine, 8-thiol guanine, 8-thioalkyl guanines, 8-hydroxyl guanine and other 8-substituted guanines, other aza and deaza uracils, thymidines, cytosines or guanines, 5-trifluoromethyl uracil and 5-trifluoro cytosine.

The antisense oligonucleotides of the invention may also comprise modified phosphorus oxygen heteroatoms in the phosphate backbone, short chain alkyl or cycloalkyl intersugar linkages or short chain heteroatom or heterocyclic intersugar linkages. For example, the antisense oligonucleotides may contain methyl phosphonates, phosphorothioates, phosphorodithioates, phosphotriesters, and morpholino oligomers. In one embodiment of the invention, the antisense oligonucleotides comprise phosphorothioate bonds linking between the four to six 3'-terminus nucleotides. In another embodiment, the phosphorothioate bonds link all the nucleotides. The antisense oligonucleotides may also have sugar mimetics.

The antisense oligonucleotides of the invention may also comprise nucleotide analogues wherein the structure of the nucleotide is fundamentally altered. An example of such an oligonucleotide analogue is a peptide nucleic acid (PNA) wherein the deoxyribose (or ribose) phosphate backbone in DNA (or RNA) is replaced with a polyamide backbone which is similar to that found in peptides (Nielsen et al.[11]; Good and Nielsen[12]; Buchardt, deceased, et al.[13], U. S. Patent No. 5,766,855; Buchardt, deceased, et al.[14], U.S. Patent No. 5,719,262). PNA analogues have been shown to be resistant to degradation by enzymes and to have extended lives *in vivo and in vitro.* PNAs also bind more strongly to a complementary DNA sequence than to a naturally occurring nucleic acid molecule due to the lack of charge repulsion between the PNA strand and the DNA strand.

The oligonucleotides of the present invention may also include other nucleotides comprising polymer backbones, cyclic backbones, or acyclic backbones. For example, the nucleotides may comprise morpholino backbone structures (U.S. Patent No. 5,034,506[15]).

The oligonucleotides of the present invention are "nuclease resistant" when they have either been modified such that they are not susceptible to degradation by DNA and RNA nucleases or alternatively they have been placed in a delivery vehicle which in itself protects the oligonucleotide from DNA or RNA nucleases. Nuclease resistant oligonucleotides include, for example, methyl phosphonates, phosphorothioates, phosphorodithioates, phosphotriesters, and morpholino oligomers. Suitable delivery vehicles for conferring nuclease resistance include, for example liposomes.

The oligonucleotides of the present invention may also contain groups, such as groups for improving the pharmacokinetic properties of an oligonucleotides, or groups for improving the pharmacodynamic properties of an oligonucleotide. Preferably, the oligonucleotides do not contain reporter groups or labels, such as fluorescent dyes or radioactive labels.

The antisense oligonucleotides may be complementary to the complete ribonucleotide reductase or secA gene including the introns. Preferably, the antisense oligonucleotides are complimentary to the mRNA region from the ribonucleotide reductase gene or the secA gene.

The antisense oligonucleotides may be selected from the sequence complementary to the ribonucletide reductase or secA genes such that the sequence exhibits the least likelihood of showing duplex formation, hair-pin formation, and homooligomer/sequence repeats but has a high to moderate potential to bind to the ribonucleotides reductase gene or the secA gene sequence and contains a GC clamp. These properties may be determined using the computer modeling program OLIGO Primer Analysis Software, Version 5.0 (distributed by National Biosciences, Inc., Plymouth, MN). This computer program allows the determination of a qualitative estimation of these five parameters.

Alternatively, the antisense oligonucleotides may also be selected on the basis that the sequence is highly conserved for either the ribonucleotide reductase or the secA genes between two or more microbial species. These properties may be determined using the BLASTN program (Altschul, et al.[16]) of the University of Wisconsin Computer group (GCG) software (Devereux J. et al.[17]) with the National Center for Biotechnology Information (NCBI) databases.

The antisense oligonucleotides generally comprise from at least about 3 nucleotides or nucleotide analogs, preferably from about 3 to about 50 nucleotides or nucleotide analogs, more preferably, from about 7 to about 35 nucleotides or nucleotide analogs, most preferably from about 15 to about 25 nucleotide or nucleotide analogs.

Preferably, the antisense oligonucleotides comprise from 3 to about 50 nucleotides or nucleotide analogs, more preferably from 20 to about 50 nucleotides or nucleotide analogs and further comprise all or part of the sequences set forth in Tables 1, 2, 3, and 4 (below). Preferably, the oligonucleotides complementary to the ribonucleotide reductase gene comprise SEQ ID NOS.: 14 to 157 as shown in Tables 1 and 2. Preferably, the antisense oligonucleotides complementary to the secA gene comprise the SEQ ID NOS.: 158 to 265 as shown in Tables 3 and 4.

Table 1
Antisense oligonucleotides that target the *Escherichia coli* K12 ribonucleotide reductase large subunit (R1)

| SEQ ID No: | Name | Sequence 5'-3' | Tm (°C) | ΔG (kcal/mol) |
|---|---|---|---|---|
| 14 | ER1-16 | CCGTCGCGCTTTGTCACCAG | 61.1 | -43.0 |
| 15 | ER1-24 | CTGTGCTACCGTCGCGCTTT | 57.8 | -42.0 |
| 16 | ER1-33 | TGATGCGCTCTGTGCTACCG | 57.2 | -40.2 |
| 17 | ER1-44 | TTTGTCGAGATTGAT GCGCT | 53.3 | -38.7 |
| 18 | ER1-58 | AGAACGCGATGGATTTTGTC | 51.7 | -38.4 |
| 19 | ER1-71 | TGCCGCCCAATCCAGAACGC | 64.6 | -46.0 |
| 20 | ER1-79 | AGTCCTTCTGCCGCCCAATC | 57.7 | -42.2 |
| 21 | ER1-128 | AAACTGAATGTGGGAGCGCA | 55.5 | -39.8 |
| 22 | ER1-169 | ATAATGGTTTCGTGGATGTC | 55.5 | -35.4 |
| 23 | ER1-180 | CGGCAGCCTTGATAATGGTT | 54.2 | -40.6 |
| 24 | ER1-218 | ATACTGATAATCCGGCGCAT | 51.4 | -39.4 |
| 25 | ER1-252 | TACGCAGGTGGAAGATCGCC | 57.3 | -41.4 |

| SEQ ID No: | Name | Sequence 5'-3' | Tm (°C) | ΔG (kcal/mol) |
|---|---|---|---|---|
| 26 | ER1-294 | GGTCGTACAGCGCAGGCGGC | 64.4 | -45.9 |
| 27 | ER1-320 | GCCCATCTCGACCATTTTCA | 54.7 | -39.7 |
| 28 | ER1-330 | TATCGTATTTGCCCATCTCG | 50.4 | -38.1 |
| 29 | ER1-423 | CGGCAGCATAAGAGAAGGTC | 51.6 | -38.5 |
| 30 | ER1-439 | CCTTCCAGCTGCTTAACGGC | 56.4 | -41.9 |
| 31 | ER1-450 | CCAGATATTTGCCTTCCAGC | 51.5 | -38.8 |
| 32 | ER1-479 | ATAGATTTCGCCGGTCACGC | 56.4 | -41.8 |
| 33 | ER1-495 | GGAACTGGGCGCTCTCATAG | 53.9 | -39.7 |
| 34 | ER1-504 | GAATATAAAGGAACTGGGCG | 48.5 | -38.0 |
| 35 | ER1-518 | GCACGCGGCAACTAGAATAT | 52.2 | -39.4 |
| 36 | ER1-529 | TTCGAGAACAAGCACGCGGC | 60.8 | -43.3 |
| 37 | ER1-543 | TTTCACGCGGGTAGTTCGAG | 55.2 | -40.5 |
| 38 | ER1-566 | ACGCTTCACATATTGCAGGC | 52.2 | -38.7 |
| 39 | ER1-584 | GGAAACCGCGTCGTAAAAAC | 53.9 | -40.8 |
| 40 | ER1-592 | TTAAATGTGGAAACCGCGTC | 52.7 | -39.3 |
| 41 | ER1-617 | CATGATTGGCGTCGGCAGCG | 64.0 | -44.9 |
| 42 | ER1-628 | CGCACGCCGGACATGATTGG | 63.8 | -44.6 |
| 43 | ER1-640 | CGAGTCGGGGTACGCACGCC | 64.2 | -45.8 |
| 44 | ER1-667 | TCGATCAGTACGCAGGAGCT | 52.4 | -38.1 |
| 45 | ER1-680 | GCTGTCACCGCACTCGATCA | 56.9 | -39.1 |
| 46 | ER1-689 | GGAATCCAGGCTGTCACCGC | 59.0 | -41.9 |

| SEQ ID No: | Name | Sequence 5'-3' | Tm (°C) | ΔG (kcal/mol) |
|---|---|---|---|---|
| 47 | ER1-704 | GGAGGTGGCGTTGATGGAAT | 56.0 | -40.6 |
| 48 | ER1-716 | AACAATCGCGCTGGAGGTGG | 59.5 | -42.7 |
| 49 | ER1-778 | CTACCCAGCGCACGAATACG | 55.7 | -40.9 |
| 50 | ER1-817 | ATGCAGCCGGTATGGAACGC | 59.4 | -43.1 |
| 51 | ER1-829 | TTGTAGAACGGAATGCAGCC | 52.8 | -38.8 |
| 52 | ER1-846 | CCGCTGTCTGGAAATGTTTG | 53.1 | -38.6 |
| 53 | ER1-855 | AGGATTTCACCGCTGTCTGG | 54.0 | -39.2 |
| 54 | ER1-874 | CGCACACCGCCCTGAGAGCA | 63.9 | -44.0 |
| 55 | ER1-907 | CACATCGGGTAGAACAGCGT | 52.5 | -38.1 |
| 56 | ER1-925 | CTTTCCACTTCCAGATGCCA | 52.5 | -38.1 |
| 57 | ER1-964 | TTGCCTTCCACACCACGGTT | 57.5 | -40.8 |
| 58 | ER1-971 | CACGCGGTTGCCTTCCACAC | 60.8 | -42.5 |
| 59 | ER1-981 | CCATATGACGCACGCGGTTG | 59.4 | -42.1 |
| 60 | ER1-1034 | TTCACCTTTCAGCAGACGGG | 55.0 | -39.6 |
| 61 | ER1-1055 | CGGGCTGAACAGGGTGATAT | 53.8 | -39.6 |
| 62 | ER1-1059 | CGGACGGGCTGAACAGGGTG | 62.1 | -43.7 |
| 63 | ER1-1061 | GTCGGACGGGCTGAACAGGG | 61.2 | -43.4 |
| 64 | ER1-1106 | AAACTCTTCCTGATCGGCGA | 53.8 | -39.7 |
| 65 | ER1-1148 | GCGGATGCTGTCGTCTTTCT | 54.3 | -39.4 |
| 66 | ER1-1155 | GCTGCTTGCGGATGCTGTCG | 61.3 | -43.0 |
| 67 | ER1-1166 | GGCTTTCACACGCTGCTTGC | 58.2 | -41.4 |

| SEQ ID No: | Name | Sequence 5'-3' | Tm (°C) | ΔG (kcal/mol) |
|---|---|---|---|---|
| 68 | ER1-1173 | GCTCAACGGCTTTCACACGC | 58.0 | -41.3 |
| 69 | ER1-1212 | GACCGGTAGACGCACGTTCC | 56.7 | -40.8 |
| 70 | ER1-1255 | GGGCTATGGGTATTGCAGTG | 52.1 | -38.7 |
| 71 | ER1-1259 | AAACGGGCTATGGGTATTGC | 53.3 | -40.7 |
| 72 | ER1-1265 | CGGATCAAACGGGCTATGGG | 58.7 | -43.4 |
| 73 | ER1-1311 | GGGCTATCTCCAGGCACAGG | 55.9 | -40.7 |
| 74 | ER1-1315 | GGCAGGGCTATCTCCAGGCA | 58.7 | -42.5 |
| 75 | ER1-1320 | TGGTCGGCAGGGCTATCTCC | 58.6 | -42.4 |
| 76 | ER1-1326 | GCGGTTTGGTCGGCAGGGCT | 64.9 | -47.0 |
| 77 | ER1-1330 | TTCAGCGGTTTGGTCGGCAG | 60.5 | -43.1 |
| 78 | ER1-1336 | ACGTCGTTCAGCGGTTTGGT | 56.8 | -40.9 |
| 79 | ER1-1356 | TTTCACCGTTCTCGTCGTTG | 53.5 | -38.5 |
| 80 | ER1-1364 | CAGCGCGATTTCACCGTTCT | 57.5 | -41.7 |
| 81 | ER1-1370 | CGTACACAGCGCGATTTCAC | 54.2 | -38.9 |
| 82 | ER1-1379 | AGCAGACAGCGTACACAGCG | 54.0 | -38.2 |
| 83 | ER1-1388 | CAGGTTGAAAGCAGACAGCG | 53.4 | -38.4 |
| 84 | ER1-1397 | AATTGCGCCCAGGTTGAAAG | 56.5 | -41.9 |
| 85 | ER1-1407 | CCAGGTTATTAATTGCGCCC | 53.8 | -41.3 |
| 86 | ER1-1428 | TTGCCAGCTCTTCCAGTTCA | 53.3 | -38.2 |
| 87 | ER1-1438 | ACCGCCAGAATTGCCAGCTC | 58.8 | -42.5 |
| 88 | ER1-1451 | GTCAAGTGCACGAACCGCCA | 59.1 | -41.0 |

| SEQ ID No: | Name | Sequence 5'-3' | Tm (°C) | ΔG (kcal/mol) |
|---|---|---|---|---|
| 89 | ER1-1463 | ATCCAGCAGCGCGTCAAGTG | 58.5 | -41.2 |
| 90 | ER1-1468 | TGATAATCCAGCAGCGCGTC | 56.1 | -40.4 |
| 91 | ER1-1535 | GATCACACCAATACCCAGCG | 52.6 | -38.1 |
| 92 | ER1-1561 | TCGTTCGCCAGGTAGTAAGC | 52.2 | -39.0 |
| 93 | ER1-1570 | CGTTTACCGTCGTTCGCCAG | 57.9 | -42.2 |
| 94 | ER1-1584 | TGCCGTCGGAGTAGCGTTTA | 55.8 | -41.0 |
| 95 | ER1-1605 | TATGCGTCAGGTTGTTGGCG | 56.8 | -40.5 |
| 96 | ER1-1614 | CGAAGGTTTTATGCGTCAGG | 52.5 | -39.3 |
| 97 | ER1-1688 | GTTAAACCACGGGCACGCGC | 62.0 | -45.0 |
| 98 | ER1-1705 | TTCGCGTAAGTGGTTTCGTT | 52.6 | -39.3 |
| 99 | ER1-1731 | TATAGGTATCGATCGGCAGG | 49.5 | -38.0 |
| 100 | ER1-1777 | CAGTCGTAATGCAGCGGCTC | 55.8 | -40.2 |
| 101 | ER1-1789 | CGCAGAGCTTCCCAGTCGTA | 55.4 | -40.0 |
| 102 | ER1-1839 | TCAGAGCAGAAAGCGTGGAG | 53.0 | -38.1 |
| 103 | ER1-1849 | TCGGACGGCATCAGAGCAGA | 58.9 | -40.9 |
| 104 | ER1-1874 | GGCGTTAGAGATCTGCGAAG | 51.8 | -38.7 |
| 105 | ER1-1916 | TTTGATGCTGACGTAACCGC | 53.7 | -39.0 |
| 106 | ER1-1923 | TCGACGCTTTGATGCTGACG | 57.1 | -40.2 |
| 107 | ER1-1944 | CCTGGCGCAAAATACCGTCT | 56.5 | -42.0 |
| 108 | ER1-1957 | TAGTCCGGCACCACCTGGCG | 62.5 | -44.2 |
| 109 | ER1-1968 | GCAGGTGCTCGTAGTCCGGC | 59.3 | -42.4 |

| SEQ ID No: | Name | Sequence 5'-3' | Tm (°C) | ΔG (kcal/mol) |
|---|---|---|---|---|
| 110 | ER1-1974 | CGTCGTGCAGGTGCTCGTAG | 56.7 | -39.9 |
| 111 | ER1-1983 | GCTCATAGGCGTCGTGCAGG | 58.0 | -41.4 |
| 112 | ER1-1992 | CCCACAGCAGCTCATAGGCG | 58.0 | -41.5 |
| 113 | ER1-2000 | CGGCATTTCCCACAGCAGCT | 59.7 | -42.8 |
| 114 | ER1-2010 | CATCGTTACCCGGCATTTCC | 56.5 | -41.9 |
| 115 | ER1-2083 | GGATCGTAGTTGGTGTTGGC | 51.8 | -39.9 |
| 116 | ER1-2112 | TCGGCACTTTTCCTGACGGG | 59.5 | -42.8 |
| 117 | ER1-2145 | AGGCGGTGAGCAGGTCTTTC | 55.7 | -40.5 |
| 118 | ER1-2154 | CGAATTTGTAGGCGGTGAGC | 54.8 | -40.5 |
| 119 | ER1-2166 | GTGTTTTGACCCCGAATTTG | 51.9 | -38.6 |
| 120 | ER1-2211 | CGTCTTGTGCGTCTTCAGCG | 56.8 | -40.0 |
| 121 | ER1-2262 | TCTTACATGCGCCGCTTTCG | 58.6 | -42.8 |

Table 2
Antisense oligonucleotides that target the *Escherichia coli* K12 ribonucleotide reductase small subunit (R2)

| SEQ ID No: | Name | Sequence 5'-3' | Tm (°C) | ΔG (kcal/mol) |
|---|---|---|---|---|
| 122 | ER2-50 | CGGCTGACCAAAGAACATCG | 55.5 | -40.0 |
| 123 | ER2-60 | CCACGTTGACCGGCTGACCA | 61.2 | -42.2 |
| 124 | ER2-67 | TAGCGAGCCACGTTGACCGG | 60.6 | -43.2 |
| 125 | ER2-134 | CGGACGCCAGAAGAAAGAGA | 54.4 | -39.8 |
| 126 | ER2-144 | CAACTTCTTCCGGACGCCAG | 57.0 | -41.3 |

| SEQ ID No: | Name | Sequence 5'-3' | Tm (°C) | ΔG (kcal/mol) |
|---|---|---|---|---|
| 127 | ER2-168 | AATCTATACGGTCGCGGGAG | 53.4 | -40.5 |
| 128 | ER2-198 | TGTGTTTTTCGTGCTCCGGC | 58.3 | -41.6 |
| 129 | ER2-273 | GCAATAGCGCCACGTTCGGG | 62.1 | -45.2 |
| 130 | ER2-284 | AGAAATAAGCGGCAATAGCG | 51.8 | -40.3 |
| 131 | ER2-290 | CGGAATAGAAATAAGCGGCA | 52.4 | -40.3 |
| 132 | ER2-307 | ACCCAGGTTTCCAGTTCCGG | 57.4 | -42.0 |
| 133 | ER2-350 | ATAGGAACGGGAATGAATCG | 50.7 | -38.8 |
| 134 | ER2-441 | TCCCTTCCGCACGTTTCTGG | 59.5 | -42.8 |
| 135 | ER2-498 | CGCCCAGCAGATGCCAGTAG | 58.0 | -41.5 |
| 136 | ER2-505 | GTACCTTCGCCCAGCAGATG | 54.6 | -39.7 |
| 137 | ER2-544 | CGCAGGCTAACGGTCACAGT | 55.2 | -39.7 |
| 138 | ER2-557 | TTTCTTCAGCTCGCGCAGGC | 60.2 | -43.4 |
| 139 | ER2-640 | GCAAATGCGAAGGAACAAGC | 54.9 | -40.4 |
| 140 | ER2-655 | ATCAATTCGCGTTCTGCAAA | 53.4 | -39.3 |
| 141 | ER2-680 | GCGAATAATTTTGGCGTTGC | 54.9 | -41.6 |
| 142 | ER2-692 | GCGGGCAATCAGGCGAATAA | 59.5 | -44.0 |
| 143 | ER2-704 | CAGGGCTTCGTCGCGGGCAA | 66.8 | -47.8 |
| 144 | ER2-714 | CGGTCAGGTGCAGGGCTTCG | 62.3 | -44.0 |
| 145 | ER2-724 | TGCTGGGTGCCGGTCAGGTG | 63.6 | -43.5 |
| 146 | ER2-728 | CATATGCTGGGTGCCGGTCA | 58.8 | -41.4 |
| 147 | ER2-778 | GCAATTTCCGCCATCTCAGG | 56.8 | -41.5 |

| SEQ ID No: | Name | Sequence 5'-3' | Tm (°C) | ΔG (kcal/mol) |
|---|---|---|---|---|
| 148 | ER2-796 | TCCTGCTTACACTCTTCGGC | 52.1 | -38.3 |
| 149 | ER2-848 | ATCCGCCCAGTCTTTCTCCT | 54.2 | -40.4 |
| 150 | ER2-857 | GAACAGATAATCCGCCCAGT | 50.7 | -38.1 |
| 151 | ER2-976 | GGGTTGGAGCGCGTCTGGAA | 61.8 | -44.0 |
| 152 | ER2-983 | CGGGATCGGGTTGGAGCGCG | 68.1 | -49.1 |
| 153 | ER2-985 | CACGGGATCGGGTTGGAGCG | 64.0 | -45.6 |
| 154 | ER2-1045 | CTGACTTCCACTTCCTGCGG | 54.6 | -39.9 |
| 155 | ER2-1063 | TGCCCGACCAGATAAGAACT | 51.3 | -38.2 |
| 156 | ER2-1076 | TTCCGAGTCAATCTGCCCGA | 57.8 | -41.2 |
| 157 | ER2-1092 | AATCGTCGGTGTCCACTTCC | 53.6 | -38.8 |

Table 3
Antisense Sequences that Target *Escherichia coli SecA*

| SEQ ID No: | Name | Sequence 5 → 3' | Tm (°C) | ΔG kDa/mol |
|---|---|---|---|---|
| 158 | ES56 | GACCACTTTGCGCATCCGGC | 62.1 | -44.2 |
| 159 | ES62 | GATGTTGACCACTTTGCGCA | 54.3 | -38.3 |
| 160 | ES85 | ATCTCCGGTTCCATGGCATT | 55.5 | -40.8 |
| 161 | ES92 | TTTTTCCATCTCCGGTTCCA | 54.3 | -40.1 |
| 162 | ES116 | CCCTTTCAGTTCTTCGTCGG | 53.8 | -39.8 |
| 163 | ES124 | GCGGTTTTCCCTTTCAGTTC | 52.9 | -39.9 |
| 164 | ES129 | ACTCTGCGGTTTTCCCTTTC | 52.5 | -39.6 |
| 165 | ES153 | CGCCTTTTTCCAGACGTGCA | 58.4 | -41.9 |
| 166 | ES158 | CACTTCGCCTTTTTCCAGAC | 51.5 | -38.4 |
| 167 | ES165 | TTTCCAGCACTTCGCCTTTT | 54.1 | -40.5 |

| SEQ ID No: | Name | Sequence 5 → 3' | Tm (°C) | ΔG kDa/mol |
|---|---|---|---|---|
| 168 | ES170 | CAGATTTTCCAGCACTTCGC | 52.5 | -38.6 |
| 169 | ES206 | ACTTGCCTCACGTACCACGG | 54.9 | -39.5 |
| 170 | ES215 | GACGCGCTTACTTGCCTCAC | 55.0 | -40.1 |
| 171 | ES230 | GTGACGCATACCAAAGACGC | 53.1 | -38.5 |
| 172 | ES264 | TAAGAACCATACCGCCGAGT | 51.5 | -39.1 |
| 173 | ES286 | ATTTCGGCGATGCAGCGTTC | 59.7 | -43.4 |
| 174 | ES303 | TTCCTTCACCGGTACGCATT | 54.5 | -40.3 |
| 175 | ES307 | GTTTTTCCTTCACCGGTACG | 51.4 | -38.9 |
| 176 | ES320 | CGTTGCGGTCAGGGTTTTTC | 56.8 | -41.6 |
| 177 | ES336 | TCAGGTAAGCAGGCAGCGTT | 55.0 | -40.2 |
| 178 | ES351 | TACCGGTTAGTGCGTTCAGG | 52.8 | -39.2 |
| 179 | ES392 | TTGCGCCAGGTAGTCGTTGA | 56.5 | -40.4 |
| 180 | ES398 | GTCACGTTGCGCCAGGTAGT | 55.0 | -39.5 |
| 181 | ES418 | AGCGGACGGTTGTTTTCGGC | 60.8 | -44.5 |
| 182 | ES429 | GGAATTCAAACAGCGGACGG | 56.7 | -41.5 |
| 183 | ES436 | AGGCCAAGGAATTCAAACAG | 51.0 | -38.4 |
| 184 | ES448 | ATACCGACAGTCAGGCCAAG | 51.6 | -38.0 |
| 185 | ES485 | TTCGCGCTTTGCCGGTGCTG | 65.8 | -46.9 |
| 186 | ES531 | AGCCGTATTCGTTGTTCGTA | 50.1 | -37.9 |
| 187 | ES544 | CGCAGGTAGTCAAAGCCGTA | 53.1 | -39.5 |
| 188 | ES553 | ATGTTGTCGCGCAGGTAGTC | 52.6 | -38.1 |
| 189 | ES556 | GCCATGTTGTCGCGCAGGTA | 59.2 | -41.7 |
| 190 | ES617 | GTCCACTTCGTCCACCAGCG | 57.7 | -40.4 |
| 191 | ES646 | GGTGTACGCGCTTCATCGAT | 55.0 | -40.0 |
| 192 | ES647 | CGGTGTACGCGCTTCATCGA | 59.3 | -42.1 |
| 193 | ES695 | GCGTTTATACATTTCCGAGC | 49.5 | -38.4 |
| 194 | ES724 | CGGATCAGGTGCGGAATAAT | 53.9 | -40.4 |

| SEQ ID No: | Name | Sequence 5 → 3' | Tm (°C) | ΔG kDa/mol |
|---|---|---|---|---|
| 195 | ES799 | TTCACCTGGCGAGATTTTTC | 51.8 | -38.6 |
| 196 | ES824 | CAGCACCAGACCACGTTCGG | 58.6 | -40.7 |
| 197 | ES851 | GCCCTCTTTCACCAGCAGTT | 53.3 | -39.1 |
| 198 | ES866 | CCCTTCATCCATGATGCCCT | 55.9 | -40.6 |
| 199 | ES889 | TTGGCCGGAGAGTACAGAGA | 52.2 | -38.1 |
| 200 | ES898 | AGCATGATGTTGGCCGGAGA | 57.6 | -40.9 |
| 201 | ES922 | AGCGCCGCCGTTACGTGGTG | 64.6 | -46.5 |
| 202 | ES950 | GTCACGGGTAAACAGCGCAT | 54.9 | -40.0 |
| 203 | ES1068 | CACCTTCTTTCGCTTCCACA | 52.8 | -38.4 |
| 204 | ES1097 | CAGCGTTTGGTTTTCGTTCT | 52.1 | -38.9 |
| 205 | ES1109 | GGTGATCGAAGCCAGCGTTT | 56.5 | -41.2 |
| 206 | ES1128 | GACGGAAGTAGTTCTGGAAG | 45.5 | -35.0 |
| 207 | ES1147 | CCCGCCAGTTTTTCATACAG | 52.3 | -39.2 |
| 208 | ES1152 | TCATCCCCGCCAGTTTTTCA | 57.5 | -41.6 |
| 209 | ES1218 | GAACAACGACGGTATCCAGC | 52.0 | -38.2 |
| 210 | ES1328 | GCCTTTCGCAGTACGTTCTT | 51.4 | -38.9 |
| 211 | ES1350 | TAGTACCCACCAGCACCGGC | 57.1 | -41.4 |
| 212 | ES1398 | CGGCTTTGGTCAGTTCGTTT | 54.3 | -40.1 |
| 213 | ES1410 | TGTGCTTAATACCGGCTTTG | 50.8 | -38.6 |
| 214 | ES1439 | GTTGGCGTGGAATTTGGCGT | 59.3 | -43.0 |
| 215 | ES1462 | GCCTGAGCAACAATCGCCGC | 62.4 | -44.5 |
| 216 | ES1515 | CTGTACCACGACCCGCCATA | 55.6 | -40.3 |
| 217 | ES1518 | TATCTGTACCACGACCCGCC | 54.7 | -40.0 |
| 218 | ES1545 | CTGCCTGCCAGCTACCACCG | 60.2 | -42.9 |
| 219 | ES1563 | TTTCCAGCGCGGCAACTTCT | 59.4 | -43.4 |
| 220 | ES1581 | TTTGCTCTGCGGTCGGATTT | 57.0 | -41.8 |
| 221 | ES1589 | TTTTTCAATTTGCTCTGCGG | 53.2 | -39.8 |

| SEQ ID No: | Name | Sequence 5 → 3' | Tm (°C) | ΔG kDa/mol |
|---|---|---|---|---|
| 222 | ES1624 | ACCGCATCGTGACGTACCTG | 55.7 | -39.6 |
| 223 | ES1629 | CCAGTACCGCATCGTGACGT | 55.7 | -39.6 |
| 224 | ES1633 | GCTTCCAGTACCGCATCGTG | 55.5 | -40.0 |
| 225 | ES1655 | ACCGATGATATGCAGGCCAC | 54.6 | -39.6 |
| 226 | ES1712 | ACGACCAGAACGACCGCGCA | 63.3 | -44.1 |
| 227 | ES1718 | CCCCTGACGACCAGAACGAC | 56.6 | -40.1 |
| 228 | ES1722 | CATCCCCCTGACGACCAGAA | 56.9 | -40.4 |
| 229 | ES1739 | GAAACGGGAAGAACCAGCAT | 53.1 | -39.5 |
| 230 | ES1748 | CGACAGGTAGAAACGGGAAG | 51.4 | -38.6 |
| 231 | ES1781 | GGAAGCAAAAATACGCATCA | 50.6 | -38.2 |
| 232 | ES1785 | GGTCGGAAGCAAAAATACGC | 53.9 | -40.9 |
| 233 | ES1794 | CGGATACTCGGTCGGAAGCA | 57.3 | -41.7 |
| 234 | ES1814 | ACCCAGTTTACGCATCATGC | 52.5 | -38.5 |
| 235 | ES1845 | ACGGGTGTTCAATGGCTTCG | 57.1 | -41.2 |
| 236 | ES1861 | ATCGCTTTAGTCACCCACGG | 54.1 | -40.0 |
| 237 | ES1888 | CTTTCAACTTTACGCTGGGC | 51.9 | -39.3 |
| 238 | ES1892 | ACGGCTTTCAACTTTACGCT | 51.1 | -39.2 |
| 239 | ES2007 | TGGTTTCGCTCACATCGCTG | 57.0 | -40.0 |
| 240 | ES2054 | GTAGGCATCAATGGTCGCTT | 51.7 | -38.5 |
| 241 | ES2084 | CCACATTTCTTCCAGCGACT | 51.7 | -38.0 |
| 242 | ES2087 | ATCCCACATTTCTTCCAGCG | 53.9 | -39.7 |
| 243 | ES2191 | TCACGCAGCGTCTCTTCATG | 54.7 | -38.2 |
| 244 | ES2275 | CCTTTCTCGAAGTGACGCAT | 51.9 | -38.2 |
| 245 | ES2306 | CCACAGGGAGTCAAGCGTTT | 54.1 | -39.3 |
| 246 | ES2325 | TCGCTGCCAGGTGCTCTTTC | 57.7 | -41.1 |
| 247 | ES2330 | GTCCATCGCTGCCAGGTGCT | 59.7 | -41.9 |
| 248 | ES2339 | ACGCAGATAGTCCATCGCTG | 52.7 | -38.4 |

| SEQ ID No: | Name | Sequence 5 → 3' | Tm (°C) | ΔG kDa/mol |
|---|---|---|---|---|
| 249 | ES2381 | CTTCGGATCTTTCTGTGCGT | 51.9 | -38.2 |
| 250 | ES2395 | CGTTTGTATTCCTGCTTCGG | 52.5 | -39.4 |
| 251 | ES2422 | ATCGCTGCAAACATGGAGAA | 53.1 | -38.5 |
| 252 | ES2520 | CCATACGACGCTGTTGTTCC | 52.9 | -38.5 |
| 253 | ES2525 | GGCTTCCATACGACGCTGTT | 54.2 | -40.0 |
| 254 | ES2537 | CGCTAAACGCTCGGCTTCCA | 59.9 | -44.1 |
| 255 | ES2555 | GCTAAGCTGCTGCATTTGCG | 56.2 | -41.3 |
| 256 | ES2619 | CTACTTTGCGCTCTCCGGTT | 53.8 | -40.4 |
| 257 | ES2626 | TTACGTCCTACTTTGCGCTC | 50.0 | -38.0 |
| 258 | ES2646 | AACCGCACGGGCAAGGATCG | 63.6 | -45.9 |
| 259 | ES2651 | ACCAGAACCGCACGGGCAAG | 61.7 | -44.0 |
| 260 | ES2656 | TTTTTACCAGAACCGCACGG | 55.1 | -41.0 |

Table 4

Antisense Sequences that Target *E. coli SecA* based on Conserved Sequences

| SEQ ID No: | Name | Sequence 5 → 3' | Tm (°C) | ΔG kDa/mol |
|---|---|---|---|---|
| 261 | ES386 | CAGGTAGTCGTTGACGGTAA | 47.7 | -35.7 |
| 262 | ES388 | CAGGTAGTCGTTGACGGT | 45.0 | -32.9 |
| 263 | ES1126 | CGGAAGTAGTTCTGGAAGGT | 47.6 | -36.5 |
| 264 | ES1702 | CGACCGCGCAACTGGTTATC | 57.8 | -41.9 |
| 265 | ES2644 | CCGCACGGGCAAGGATCGTT | 63.6 | -45.9 |

In Tables 1, 2, 3, and 4, the "Tm" is the melting temperature of an oligonucleotide duplex calculated according to the nearest-neighbor thermodynamic values. At this temperature 50% of nucleic acid molecules are in duplex and 50% are denatured. The "ΔG" is the free energy of the oligonucleotide, which is a measurement of an oligonucleotide duplex stability.

The following sequences have been determined to be conserved among species:

ES386 [SEQ ID NO:261] is conserved among *Escherichia coli* and *Mycobacterium tuberculosis;*

ES388 [SEQ ID NO:262] is conserved among *Escherichia coli; Mycobacterium tuberculosis;* and *Mycobacterium bovis;*

ES553 [SEQ ID NO:188] is conserved among *Escherichia coli, Mycobacterium tuberculosis, Mycobacterium bovis, Streptomyces coelicolor;* and *Streptomyces lividans*;

ES556 [SEQ ID NO:189] is conserved among *Escherichia coli, Mycobacterium tuberculosis, Mycobacterium bovis, Streptomyces coelicolor;* and *Streptomyces lividans*; and *Synechoccus sp. ;* and

ES646 [SEQ ID NO:191] is conserved among *Escherichia coli* and *Staphylococcus carnosus*;

ES1126 [SEQ ID NO:263] is conserved among *Escherichia coli* and *Rhodobacter capsulatus* SecA genes.

ES2644 [SEQ ID NO:265] is conserved among *Escherichia coli* SecA gene, MutA (A:T to C:G transversion), and tyrosine-specific transport protein (tyrP) gene.

The term "alkyl" refers to monovalent alkyl groups preferably having from 1 to 20 carbon atoms and more preferably 1 to 6 carbon atoms. This term is exemplified by groups such as methyl, ethyl, *n*-propyl, iso-propyl, *n*-butyl, iso-butyl, *n*-hexyl, and the like.

The term "aryl" refers to an unsaturated aromatic carbocyclic group of from 6 to 14 carbon atoms having a single ring (e.g., phenyl) or multiple condensed (fused) rings (e.g., naphthyl or anthryl). Preferred aryls include phenyl, naphthyl and the like.

The term "cycloalkyl" refers to cyclic alkyl groups of from 3 to 20 carbon atoms having a single cyclic ring or multiple condensed rings. Such cycloalkyl groups include, by way of example, single ring structures such as cyclopropyl, cyclobutyl, cyclopentyl, cyclooctyl, and the like, or multiple ring structures such as adamantanyl, and the like.

The term "halo" or "halogen" refers to fluoro, chloro, bromo and iodo and preferably is either fluoro or chloro.

The term "thiol" refers to the group -SH.

As to any of the above groups which contain one or more substituents, it is understood, of course, that such groups do not contain any substitution or substitution patterns which are sterically impractical and/or synthetically non-feasible. In addition, the compounds of this invention include all stereochemical isomers arising from the substitution of these compounds.

The term "pharmaceutically acceptable salt" refers to salts which retain the biological effectiveness and properties of the antisense oligonucleotides of this invention and which are not biologically or otherwise undesirable. In many cases, the antisense oligonucleotides of this invention are capable of forming acid and/or base salts by virtue of the presence of amino and/or carboxyl groups or groups similar thereto.

Pharmaceutically acceptable base addition salts can be prepared from inorganic and organic bases. Salts derived from inorganic bases, include by way of example only, sodium, potassium, lithium, ammonium, calcium and magnesium salts. Salts derived from organic bases include, but are not limited to, salts of primary, secondary and tertiary amines, such as alkyl amines, dialkyl amines, trialkyl amines, substituted alkyl amines, di(substituted alkyl) amines, tri(substituted alkyl) amines, alkenyl amines, dialkenyl amines, trialkenyl amines, substituted alkenyl amines, di(substituted alkenyl) amines, tri(substituted alkenyl) amines, cycloalkyl amines, di(cycloalkyl) amines, tri(cycloalkyl) amines, substituted cycloalkyl amines, disubstituted cycloalkyl amine, trisubstituted cycloalkyl amines, cycloalkenyl amines, di(cycloalkenyl) amines, tri(cycloalkenyl) amines, substituted cycloalkenyl amines, disubstituted cycloalkenyl amine, trisubstituted cycloalkenyl amines, aryl amines, diaryl amines, triaryl amines, heteroaryl amines, diheteroaryl amines, triheteroaryl amines, heterocyclic amines, diheterocyclic amines, triheterocyclic amines, mixed di- and tri-amines where at least two of the substituents on the amine are different and are selected from the group consisting of alkyl, substituted alkyl, alkenyl, substituted alkenyl, cycloalkyl, substituted cycloalkyl, cycloalkenyl, substituted cycloalkenyl, aryl, heteroaryl, heterocyclic, and the like. Also included are amines where the two or three substituents, together with the amino nitrogen, form a heterocyclic or heteroaryl group.

Examples of suitable amines include, by way of example only, isopropylamine, trimethyl amine, diethyl amine, tri(*iso*-propyl) amine, tri(*n*-propyl) amine, ethanolamine, 2-dimethylaminoethanol, tromethamine, lysine, arginine, histidine, caffeine, procaine, hydrabamine, choline, betaine, ethylenediamine, glucosamine, N-alkylglucamines, theobromine, purines, piperazine, piperidine, morpholine, N-ethylpiperidine, and the like. It should also be understood that other carboxylic acid derivatives would be useful in the practice of this invention, for example, carboxylic acid amides, including carboxamides, lower alkyl carboxamides, dialkyl carboxamides, and the like.

Pharmaceutically acceptable acid addition salts may be prepared from inorganic and organic acids. Salts derived from inorganic acids include hydrochloric acid, hydrobromic acid, sulfuric acid, nitric acid, phosphoric acid, and the like. Salts derived from organic acids include acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, *p*-toluene-sulfonic acid, salicylic acid, and the like.

The term "ribonucleotide reductase gene" or the "ribonucleoside diphosphate reductase gene" refers to any gene which encodes a protein that either reduces the four main ribonucleotides to the corresponding deoxyribonucleotides involved in DNA synthesis or encodes a subunit of a multimeric enzyme which reduces the four main ribonucleotides to the corresponding deoxyribonucleotides. Without being limiting, examples of ribonucleotide reductase genes from bacteria include the *E. coli* nrdA, nrdB and nrd D genes; the *S. typhimurium* nrdE and nrdF genes; and the *Lactococcus lactis* nrdEF gene. Examples of the ribonucleotide reductase genes from viruses include the herpes simplex type 1 and 2 ribonucleotide reductases and the bovine and equine herpes simplex ribonucleotide reductases.

The term "secA" refers to an oligonucleotide sequence which encodes a protein having similar properties as those expressed by the *E. coli* secA gene. Without being limiting, examples of secA genes from bacteria include the *Mycobacterium bovis* secA gene; the *Mycobacterium tuberculosis* secA gene, the *Staphylococcus aureus* secA gene and the *Staphylococcus carnosus* secA gene.

The term "microorganism" means a bacteria, fungi or virus having either a ribonucleotide reductase or secA gene. Specifically excluded from this definition is the malerial parasite, plasmodium.

The term "bacteria" refers to any bacteria encoding either a ribonucleotide reductase gene or a secA gene, including *Escherichi coli, Mycobacterium tuberculosis, Mycobacterium bovis, Mycobacterium smegmatis, Salmonella typhimurium, Thermoplasma acidophilum, Pyrococcus furiosus, Bacillus subtilis, Bacillus firmus, Lactococcus lactis, Staphylococcus aureus, Staphylococcus camosus, Listeria monocytogenes, Borrelia burgdorferi, P. sativum, S. griseus,* and *Synechoccus sp.*

The term "virus" refers to any virus having a ribonucleotide reductase gene. Preferably the virus will be a DNA virus. Examples of suitable viruses include various herpes viruses (such as herpes simplex types 1 and 2, varicella-herpes zoster, cytomegalovirus and Epstein-Barr virus) and the various hepatitis viruses.

The term "complementary to" means that the antisense oligonucleotide sequence is capable of binding to the target sequence, ie the ribonucleotide reductase gene or the secA gene. Preferably the antisense oligonucleotide sequence has at least about 75 % identity with the target sequence, preferably at least about 90% identity and most preferably at least about 95 % identity with the target sequence allowing for gaps or mismatches of several bases. Identitiy can be determined, for example, by using the BLASTN program of the University of Wisconsin Computer Group (GCG) software.

The term "inhibiting growth" means a reduction in the growth of the bacteria or viruses of at least 25 % , more preferably of at least 50 % and most preferably of at least 75 %. The reduction in growth can be determined for bacteria by a measuring the optical density of a liquid bacteria culture with a spectrophotometer or by counting the number of colony forming units/ml (CFU/ml) upon plating on culture plates. The reduction in growth can be determined for viruses by measuring the number of plaque forming units/ml upon plating on susceptible cells.

## Preparation of the Antisense Oligonucleotides

The antisense oligonucleotides of the present invention may be prepared by conventional and well-known techniques. For example, the oligonucleotides may be prepared using solid-phase synthesis and in particular using commercially available equipment such as the equipment available from Applied Biosystems Canada Inc., Mississauga, Canada. The oligonucleotides may also be prepared by enzymatic digestion of the naturally occurring ribonucleotide reductase or secA gene by methods known in the art.

## Isolation and Purification of the Antisense Oligonucleotides

Isolation and purification of the antisense oligonucleotides described herein can be effected, if desired, by any suitable separation or purification such as, for example, filtration, extraction, crystallization, column chromatography, thin-layer chromatography, thick-layer chromatography, preparative low or high-pressure liquid chromatography or a combination of these procedures. However, other equivalent separation or isolation procedures could, of course, also be used.

The invention contemplates a method of evaluating if an antisense oligonucleotide inhibits the growth of a microbe having a ribonucleotide reductase or secA gene. The method comprises selecting the microbe/microorganism having a ribonucleotide reductase or secA gene, administering the antisense oligonucleotide; and comparing the growth of the treated microbe with the growth of an untreated microorganism.

In order for the antisense oligonucleotide to effectively interrupt the expression of the ribonucleotide reductase or secA gene, the antisense oligonucleotide enters the microorganism's cell, in the case of fungal or bacterial cells or enter the mammalian cell having the virus target.

Although oligonucleotides are taken up by bacterial cells, some modification of the oligonucleotides may help facilitate or regulate said uptake. thus, a carier molecule, for example an amino acid, can be linked to the oligonucleotide. for example, bacteria have multiple transport systems for the recognition and uptake of molecules of leucine. The addition of this amino acid to the oligonucleotide may facilitate the uptake of the oligonucleotide in the bacteria and not substantially interfere with the activity of the antisense oligonucleotide in the bacterial cell.

Other methods are contemplated for facilitating the uptake of the antisense oligonucleotide into bacteria. For example, the addition of other amino acids or peptides or primary amines to the 3' or 5' termini of the antisense oligonucleotide may enable utilization of specific transport systems. Addition of lactose to the oligonucleotide by a covalent linkage may also be used to enable transport of the antisense oligonucleotide by lactose permease. Other sugar transport systems are also known to be functional in bacteria and can be utilized in this invention.

With regard to inhibiting the expression of ribonucleotide reductase in DNA viruses, the antisense oligonucleotide is preferably introduced into the cell infected with the DNA virus. The antisense oligonucleotides may be delivered using vectors or liposomes.

An expression vector comprising the antisense oligonucleotide sequence may be constructed having regard to the sequence of the oligonucleotide and using procedures known in the art. The vectors may be selected from plasmids or benign viral vectors depending on the eukaryotic cell and the DNA virus. Phagemids are a specific example of beneficial vectors because they can be used either as plasmids or a bacteriophage vectors. Examples of other vectors include viruses such as bacteriiophages, baculoviruses and retroviruses, DNA viruses, liposomes and other recombination vectors.

Vectors can be constructed by those skilled in the art to contain all the expression elements required to achieve the desired transcription of the antisense oligonucleotide sequences. Therefore, the invention provides vectors comprising a transcription control sequence operatively linked to a sequence which encodes an antisense oligonucleotide. Suitable transcription and translation elements may be derived from a variety of sources, including bacterial, fungal, viral, mammalian or insect genes. Selection of appropriate elements is dependent on the host cell chosen.

Reporter genes may be included in the vector. Suitable reporter genes include β-galactosidase (e.g. lacZ), chloramphenicol, acetyl-transferase, firefly luciferase, or an immunoglobulin or portion thereof. Transcription of the antisense oligonucleotide may be monitored by monitoring for the expression of the reporter gene.

The vectors can be introduced into cells or tissues by any one of a variety of known methods within the art. Such methods can be found generally described in Sambrook et al.[18]; Ausubel et al.[19]; Chang et al.[20]; Vega et al.[21]; and Vectors: A Survey of Molecular Cloning Vectors and Their Uses[22] and include, for example, stable or transient transfection, lipofection, electroporation and infection with recombinant viral vectors.

Introduction of nucleic acids by infection offers several advantages. Higher efficiency and specificity for tissue type can be obtained. Viruses typically infect and propagate in specific cell types. Thus, the virus' specificity may be used to target the vector to specific cell types *in vivo* or within a tissue or mixed culture of cells. Viral vectors can also be modified with specific receptors or ligands to alter target specificity through receptor mediated events.

## Pharmaceutical Formulations

When employed as pharmaceuticals, the antisense oligonucleotides are usually administered in the form of pharmaceutical compositions. These compounds can be administered by a variety of routes including oral, rectal, transdermal, subcutaneous, intravenous, intramuscular, and intranasal. These compounds are effective as both injectable and oral compositions. Such compositions are prepared in a manner well known in the pharmaceutical art and comprise at least one active compound.

This invention also includes pharmaceutical compositions which contain, as the active ingredient, one or more of the antisense oligonucleotides associated with pharmaceutically acceptable carriers. In making the compositions of this invention, the active ingredient is usually mixed with an excipient, diluted by an excipient or enclosed within such a carrier which can be in the form of a capsule, sachet, paper or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders.

In preparing a formulation, it may be necessary to mill the active compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, e.g. about 40 mesh.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The compositions of the invention can be formulated so as to provide quick, sustained or delayed release of the active ingredient after administration to the patient by employing procedures known in the art.

The compositions are preferably formulated in a unit dosage form, each dosage containing from about 5 to about 100 mg, more usually about 10 to about 30 mg, of the active ingredient. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Preferably, the antisense oligonucleotide is employed at no more than about 20 weight percent of the pharmaceutical composition, more preferably no more than about 15 weight percent, with the balance being pharmaceutically inert carrier(s).

The antisense oligonucleotide is effective over a wide dosage range and is generally administered in a pharmaceutically effective amount. It, will be understood, however, that the amount of the antisense oligonucleotide actually administered will be determined by a physician, in the light of the relevant circumstances, including the condition to be treated, the chosen route of administration, the actual compound administered, the age, weight, and response of the individual patient, the severity of the patient's symptoms, and the like.

For preparing solid compositions such as tablets, the principal active ingredient/antisense oligonucleotide is mixed with a pharmaceutical excipient to form a solid preformulation composition containing a homogeneous mixture of a compound of the present invention. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation is then subdivided into unit dosage forms of the type described above containing from, for example, 0.1 to about 500 mg of the active ingredient of the present invention.

The tablets or pills of the present invention may be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the novel compositions of the present invention may be incorporated for administration orally or by injection include aqueous solutions, suitably flavored syrups, aqueous or oil suspensions, and flavored emulsions with edible oils such as corn oil, cottonseed oil, sesame oil, coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described *supra.* Preferably the compositions are administered by the oral or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a face mask tent, or intermittent positive pressure breathing machine. Solution, suspension, or powder compositions may be administered, preferably orally or nasally, from devices which deliver the formulation in an appropriate manner.

The following formulation examples illustrate representative pharmaceutical compositions of the present invention.

Formulation Example 1

Hard gelatin capsules containing the following ingredients are prepared:

| Ingredient | Quantity (mg/capsule) |
|---|---|
| Active Ingredient | 30.0 |
| Starch | 305.0 |
| Magnesium stearate | 5.0 |

The above ingredients are mixed and filled into hard gelatin capsules in 340 mg quantities.

Formulation Example 2

A tablet formula is prepared using the ingredients below:

| Ingredient | Quantity (mg/tablet) |
|---|---|
| Active Ingredient | 25.0 |
| Cellulose, microcrystalline | 200.0 |
| Colloidal silicon dioxide | 10.0 |

(continued)

| Ingredient | Quantity (mg/tablet) |
|---|---|
| Stearic acid | 5.0 |

The components are blended and compressed to form tablets, each weighing 240 mg.

Formulation Example 3

A dry powder inhaler formulation is prepared containing the following components:

| Ingredient | Weight % |
|---|---|
| Active Ingredient | 5 |
| Lactose | 95 |

The active ingredient is mixed with the lactose and the mixture is added to a dry powder inhaling appliance.

Formulation Example 4

Tablets, each containing 30 mg of active ingredient, are prepared as follows:

| Ingredient | Quantity (mg/tablet) |
|---|---|
| Active Ingredient | 30.0 mg |
| Starch | 45.0 mg |
| Microcrystalline cellulose | 35.0 mg |
| Polyvinylpyrrolidone (as 10% solution in sterile water) | 4.0 mg |
| Sodium carboxymethyl starch | 4.5 mg |
| Magnesium stearate | 0.5 mg |
| Talc | 1.0 mg |
| Total | 120 mg |

The active ingredient, starch and cellulose are passed through a No. 20 mesh U.S. sieve and mixed thoroughly. The solution of polyvinylpyrrolidone is mixed with the resultant powders, which are then passed through a 16 mesh U. S. sieve. The granules so produced are dried at 50° to 60°C and passed through a 16 mesh U.S. sieve. The sodium carboxymethyl starch, magnesium stearate, and talc, previously passed through a No. 30 mesh U.S. sieve, are then added to the granules which, after mixing, are compressed on a tablet machine to yield tablets each weighing 120 mg.

Formulation Example 5

Capsules, each containing 40 mg of medicament are made as follows:

| Ingredient | Quantity (mg/capsule) |
|---|---|
| Active Ingredient | 40.0 mg |
| Starch | 109.0 mg |
| Magnesium stearate | 1.0 mg |
| Total | 150.0 mg |

The active ingredient, starch, and magnesium stearate are blended, passed through a No. 20 mesh U.S. sieve, and filled into hard gelatin capsules in 150 mg quantities.

Formulation Example 6

Suppositories, each containing 25 mg of active ingredient are made as follows:

| Ingredient | Amount |
| --- | --- |
| Active Ingredient | 25 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

Formulation Example 7

Suspensions, each containing 50 mg of medicament per 5.0 mL dose are made as follows:

| Ingredient | Amount |
| --- | --- |
| Active Ingredient | 50.0 mg |
| Xanthan gum | 4.0 mg |
| Sodium carboxymethyl cellulose (11 %) | |
| Microcrystalline cellulose (89%) | 50.0 mg |
| Sucrose | 1.75 g |
| Sodium benzoate | 10.0 mg |
| Flavor and Color | q.v. |
| Purified water to | 5.0 mL |

The active ingredient, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

Formulation Example 8

| Ingredient | Quantity (mg/capsule) |
| --- | --- |
| Active Ingredient | 15.0 mg |
| Starch | 407.0 mg |
| Magnesium stearate | 3.0 mg |
| Total | 425.0 mg |

The active ingredient, starch, and magnesium stearate are blended, passed through a No. 20 mesh U.S. sieve, and filled into hard gelatin capsules in 425.0 mg quantities.

Formulation Example 9

A formulation may be prepared as follows:

| Ingredient | Quantity |
| --- | --- |
| Active Ingredient | 5.0 mg |
| Com Oil | 1.0 mL |

Formulation Example 10

A topical formulation may be prepared as follows:

| Ingredient | Quantity |
|---|---|
| Active Ingredient | 1-10 g |
| Emulsifying Wax | 30 g |
| Liquid Paraffin | 20 g |
| White Soft Paraffin | to 100 g |

The white soft paraffin is heated until molten. The liquid paraffin and emulsifying wax are incorporated and stirred until dissolved. The active ingredient is added and stirring is continued until dispersed. The mixture is then cooled until solid.

Another preferred formulation employed in the methods of the present invention employs transdermal delivery devices ("patches"). Such transdermal patches may be used to provide continuous or discontinuous infusion of the antisense oligonucleotides of the present invention in controlled amounts. The construction and use of transdermal patches for the delivery of pharmaceutical agents is well known in the art. See, for example, U.S. Patent 5,023,252[23], herein incorporated by reference. Such patches may be constructed for continuous, pulsatile, or on demand delivery of pharmaceutical agents.

Another preferred method of delivery involves "shotgun" delivery of the naked antisense oligonucleotides across the dermal layer. The delivery of "naked" antisense oligonucleotides is well known in the art. See, for example, Felgner et al.,U.S. Patent No. 5,580,859[24]. It is contemplated that the antisense oligonucleotides may be packaged in a lipid vesicle before "shotgun" delivery of the antisense oligonucleotide.

Frequently, it will be desirable or necessary to introduce the pharmaceutical composition to the brain, either directly or indirectly. Direct techniques usually involve placement of a drug delivery catheter into the host's ventricular system to bypass the blood-brain barrier. One such implantable delivery system used for the transport of biological factors to specific anatomical regions of the body is described in U.S. Patent 5,011,472[25] which is herein incorporated by reference.

Indirect techniques, which are generally preferred, usually involve formulating the compositions to provide for drug latentiation by the conversion of hydrophilic drugs into lipid-soluble drugs. Latentiation is generally achieved through blocking of the hydroxy, carbonyl, sulfate, and primary amine groups present on the drug to render the drug more lipid soluble and amenable to transportation across the blood-brain barrier. Alternatively, the delivery of hydrophilic drugs may be enhanced by intra-arterial infusion of hypertonic solutions which can transiently open the blood-brain barrier.

Other suitable formulations for use in the present invention can be found in *Remington's Pharmaceutical Sciences*[26].

The antisense oligonucleotides or the pharmaceutical composition comprising the antisense oligonucleotides may be packaged into convenient kits providing the necessary materials packaged into suitable containers.

Utility

The antisense oligonucleotides of the present invention may be used for a variety of purposes. They may be used to inhibit the expression of the ribonucleotide reductase gene in a microorganism, resulting in the inhibition of growth of that microorganism. They may be used to inhibit the expression of the secA gene in a microorganism, resulting in the inhibition of growth of that microorganism. The oligonucleotides may be used as hybridization probes to detect the presence of the ribonucleotide reductase gene or the secA gene in the microorganism. When so used the oligonucleotides may be labeled with a suitable detectable group (a radioisotope, a ligand, another member of a specific binding pair, for example, biotin). The oligonucleotides may also be used to determine the presence of a particular microorganism in a biological sample. Finally, the oligonucleotides may be used as molecular wight markers.

In order to further illustrate the present invention and advantages thereof, the following specific examples are given but are not meant to limit the scope of the claims in any way.

## EXAMPLES

In the examples below, all temperatures are in degrees Celsius (unless otherwise indicated) and all percentages are weight percentages (also unless otherwise indicated).

In the examples below, the following abbreviations have the following meanings. If an abbreviation is not defined, it has its generally accepted meaning:

| | |
|---|---|
| μM = | micromolar |
| mM = | millimolar |
| M = | molar |
| ml = | milliliter |
| μl = | microliter |
| mg = | milligram |
| μg = | microgram |
| IPTG = | isopropyl-β-D-thiogalactoside |
| PAGE = | polyacrylamide gel electrophoresis |
| PVDF = | polyvinylidene difluoride |
| rpm = | revolutions per minute |
| OD = | optical density |
| CFU = | colony forming units |
| ΔG = | free energy, a measurement of oligonucleotide duplex stability |
| kcal = | kilocalories |

General Methods in Molecular Biology:

Standard molecular biology techniques known in the art and not specifically described were generally followed as in Sambrook et al.[18]; Ausubel et al.[19]; and Perbal[27].

The antisense oligonucleotides in Tables 1, 2 and 3 were selected from the sequence complementary to the ribonucletide reductase or secA genes of *E. coli* such that the sequence exhibited the least likelihood of showing one or more of duplex formation, hair-pin formation, and homooligomer/sequence repeats but had a high to moderate potential to bind to the ribonucleotide reductase gene or the secA gene sequence. These properties were determined using the computer modeling program OLIGO Primer Analysis Software, Version 5.0 (distributed by National Biosciences, Inc., Plymouth, MN).

The antisense oligonucleotides in Table 4 were selected on the basis that the sequence is highly conserved for the secA genes between two or more microbial species. This property was determined using the BLASTN program (Altschul, et al.[16]) of the University of Wisconsin Computer group (GCG) software (Devereux J. et al.[17]) with the National Center for Biotechnology Information (NCBI) databases

Phosphorothioate oligonucleotides comprising the desired sequences were specially ordered either from Boston BioSystems, Bedford MA; Canadian Life Technologies, Burlington, Canada; Dalton Chemical Laboratories, Inc., North York, Canada; Hybridon, Inc., Milford Ma; Oligos Etc., or Oligos Therapeutics, Inc., Wilsonvill OR; or TriLink Bio Technologies, San Diego, CA. Antisense oligonucleotides may also be made by methods known in the art.

Polymerase chain reaction (PCR) was carried out generally as in *PCR Protocols: A Guide To Methods And Applications*[28].

Example 1: Inhibition of mouse ribonucleotide reductase small subunit (R2) expression in *Escherichia coli by antisense oligonucleotide AS-II-626-20*

Competent BL21 (DE3) cells carrying a plasmid containing the mouse ribonucleotide reductase R2 gene were used. (Mann et al.[34]) The antisense oligonucleotide, AS-II-626-20, GGCTAAATCGCTCCACCAAG [SEQ ID NO:266] is specifically complementary to the mouse ribonucleotide reductase R2 gene. Approximately $10^{10}$ bacteria/ml were electroporated using a Cell Porator (Gibco BRL, Burlington, Canada) in micro electro-chambers (0.4 cm between the electrodes) at a pulse of 2.4 kV, 4 kΩ with either 20 μM or 200 μM of antisense oligonucleotide AS-II-626-20, following methods described by the manufacturer (Dower W.J.[29] ; Neuman et; and Taketo, A.[31]). Control populations were subjected to electroporation but without the antisense oligonucleotide AS-II-626-20.

The bacterial cells were then transferred to Luria-Bertani broth (Miller J.H.[32]) containing 50 μg/ml of ampicillin and 0.4 mM of isopropyl β-D-thiogalactoside (IPTG) (expression inducer) (Horwitz J.P.[33]) to grow at 30°C on a shaker at 250 rotations per minute (rpm) for 5 hours.

The cells were harvested by centrifugation and treated with 2 x sample loading buffer (100 mM Tris[hydroxymethyl'aminomethane, pH 6.8, 200 mM dithiothrietol, 4% sodium dodecyl sulfate, 20% glycerol and 0.015% bromophenol blue) and sonicated (Olsvik, et al.[35]) for 15 seconds. The supernatants were resolved by polyacrylamide gel electrophoresis (PAGE) (Laemmli U.K.[36]).

The ribonucleotide reductase R2 expression was detected by Western blot. The protein gel was blotted onto polyvinylidene difuoride (PVDF) protein sequencing membrane. (Choy et al.[37]). The presence of the mouse ribonucleotide reductase was detected with a rabbit anti-mouse R2 subunit antibody (Chan et al.[39]). The presence of the antibody bound to the ribonucleotide reducatase was detected using a second goat anti-rabbit immunoglobulin linked with horseradish peroxidase (Amersham Life Sciences, Oakville Canada).

The upper panel of Figure 14 is a photograph of the Western Blot results. The lower panel of Figure 14 is a photograph of the membrane stained with India ink to indicate the level of protein loaded in each lane.

It is clear that administration of either 20 µM or 200 µM AS-II-626-20 resulted in a marked reduction of mouse ribonucleotide reductase gene expression in the *E. coli* cells.

Example 2: Inhibition of bacteria *Escherichia coli* K12 growth by antisense oligonucleotides ER1-169 and ER2-724 targeting *E. coli* ribonucleotide reductase large subunit (R1) and small subunit (R2)

*E. coli* cells were electroporated by the method set forth in Example 1 with ER1-169 [SEQ ID NO:22] or ER2-724 [SEQ ID NO:145] at the concentrations shown in Figure 15, while the control cells received oligonucleotide AS-II-626-20 [SEQ ID NO: ] (targeting mouse ribonucleotide reductase small subunit).

The *E. coli* cells were then transferred to fresh Luria-Bertani broth (Miller J.H.[32]) to grow at 30°C on a shaker at 250 rpm for 3 hours. The flasks for the test and the control each contained the same number of bacteria per ml at the start of the experiment. The optical density at 590 nm ($OD_{590}$) of the cultures was measured at the start and at the end of the 3 hours. The inhibition of *E. coli* growth was calculated by comparing the increase in $OD_{590}$ values at the start and the end of the 3 hours of the oligonucleotide-treated cultures to the increase of the control cultures at the start and at the end of the 3 hours. (Carpentier P.L.[40])

The results indicate that ER1-169 [SEQ ID NO:22] and ER2-724 [SEQ ID NO:145] inhibited the growth of *E. coli.*

Example 3: Killing of *Escherichia coli* K12 by antisense oligonucleotides targeting the ribonucleotide reductase large subunit (R1) or the small subunit (R2)

*E. coli* cells (approximately 2 x $10^9$ were incubated with 20 µM of each of the phosphorothioate oligonucleotides set forth in Figure 12 on ice for 45 minutes. A control without oligonucleotides was also incubated for each experiment. Cells were heat shocked by placing them in a 42°C bath for 45 seconds. (Sambrook J. et al.[18])

Luria-Bertani (LB) broth (Miller J.H.[32] ) was added and the samples were incubated at room temperature for 30 minutes. Dilutions of treated and untreated bacteria were incubated overnight at 37°C on culture plates containing LB medium, and the number of colonies was counted.

The number of killed bacteria was calculated by subtracting the surviving colony forming units (CFU/ml) of the oligonucleotide-treated bacteria from the CFU/ml of the control. Figure 16 shows the number of bacteria killed by treatment with the antisense sequences: ER1-640 [SEQ ID NO:43]; ER1-1059 [SEQ ID NO:62]; ER1-1320 [SEQ ID NO:75]; ER1-1315 [SEQ ID NO:74]; ER1-1326 [SEQ ID NO:76]; ER2-704 [SEQ ID NO:143] and ER2-983 [SEQ ID NO:152].

The results from Figure 16 show that antisense oligonucleotides complementary to either the R1 or R2 subunit of ribonucleotide reductase are effective as anti-bacterial agents.

Example 4: Inhibition of the secA protein expression in Escherichia coli following treatment with antisense phosphorothioate oligonucleotides

*E. coli* cells were heat shock transformed by the method set forth in Example 3 above with the 80 µM of each of the antisense phosphorothioate oligonucleotides set forth in Figure 17.

Luria-Bertani broth was then added to the treated *E. coli* cells and they were allowed to grow at 30°C on a shaker at 250 rpm for 3 hours.

Approximately the same quantity of treated and untreated bacteria, based on optical density, were washed in phosphate buffered saline, suspended in 2X Laemmli sample buffer (Laemmli U.K.[36]), heated for 5 minutes at 95°C and subjected to SDS-PAGE (sodium dodecyl sulfate-polyacrylamide gel electrophoresis).

The gel was blotted onto polyvinylidene difluoride protein sequencing membrane by the methods set forth in Example 1. A rabbit polyclonal SecA antiserum (der Blaauwen et al.[6]) was used to detect the expression of the *E. coli* secA gene. The presence of bound rabbit antibody was detected using a goat anti-rabbit immunoglobulin (Amersham Life Sciences, Oakville, Canada).

Figure 17 is a photograph of the Western Blot of *E. coli* cells treated with oligonucleotides ES799 [SEQ ID NO:195] (lane 1); ES1845 [SEQ ID NO:235] (lane 2); and the control (lane 3). When compared to the control, lane 3, the ES799 [SEQ ID NO:195] and ES1845 [SEQ ID NO:235] oligonucleotides clearly decreased the SecA protein levels in the treated *E. coli* cells. The top band in the Figure 17 represents SecA. Non-specific background bands appear below the SecA protein band.

It has been found that the antisense oligonucleotides are effective inhibitors of SecA expression in *E. coli.*

Example 5: Killing of Escherichia coli K12 by antisense secA oligonucleolides

*E. coli* cells were heat shock transformed by the method described in Example 3 above with either 100 $\mu$M or 20 $\mu$M of the antisense phosphorothioate oligonucleotides set forth in Figures 18a and 18b

Luria-Bertani (LB) broth (Miller J.H.[32]) was added and the bacterial samples were incubated at room temperature for 30 minutes. Dilutions of treated and untreated bacteria were incubated overnight at 37°C on culture plates containing LB medium, and the number of colonies was counted.

The number of killed bacteria was calculated by subtracting the surviving colony forming units (CFU/ml) of the oligonucleotide-treated bacteria from the CFU/ml of the control. Figures 18a and 18b show the number of bacteria killed by treatment with the various antisense sequences. Accordingly, antisense oligonucleotides complementary to the secA gene act to inhibit the growth of *E. coli.*

Example 6: Effect of antisense oligonucleotides on Escherichia coli K12 growth

E. coli cells were heat shock transformed by the method described in Example 3 with either 16 yM, 20 M or 80 yM of each of the antisense phosphorothioate oligonucleotides set forth in Figures 19a-g.

Equal numbers of the treated E. coli cells were then transferred to flasks containing fresh Luria-Bertani broth to grow at 30"C on a shaker at 250 rpm. The number of bacteria per flask was determined by the turbidity of the cultures at OD620 taken each hour (Carpentier P.L.40).

Figures 19a-g show the rate of growth of the E. coli in each of the flasks after treatment with the various oligonucleotides. When growth curves of the treated and untreated cultures were statistically analyzed, the growth of the antisense treated cultures was found to be significantly inhibited when compared to the control cultures.

The statistical p values are found in the Figures.

The invention includes the subject matter of the following paragraphs

§1. An antisense oligonucleotide which is nuclease resistant and comprises from about 3 to about 50 nucleotides, which nucleotides are complementary to the ribonucleotide reductase gene or the secA gene of a microorganism.

§2. The oligonucleotide of §1 comprising one or more phosphorothioate intemucleotide linkages.

§3. An antisense oligonucleotide comprising from about 3 to about 50 nucleotides which is capable of binding to the ribonucleotide reductase gene or the secA gene of a microorganism, wherein the oligonucleotide comprises all or part of a sequence selected from the group consisting ofSEQ ID NO:22; SEQ IDNO:43; SEQ IDNO:62; SEQ IDNO:74; SEQ IDNO:75; SEQ ID NO:76; SEQ ID NO: 143; SEQ IDNO:145; SEQ ID NO:152; SEQ IDNO:164; SEQ ID NO: 176; SEQ ID NO:186; SEQ IDNO:188; SEQ ID NO:189; SEQ ID NO:191; SEQ ID NO:192; SEQ ID NO:195; SEQ IDNO:197; SEQ IDNO:206; SEQ IDNO:212; SEQ ID <RTI NO:220; SEQ IDNO:229; SEQ IDNO: 235; SEQ IDNO:254; SEQ IDNO:261; SEQ ID NO:262; SEQ IDNO:263; SEQ ID NO:264; and SEQ ID NO:265.

§4. A pharmaceutical composition comprising a pharmaceutically acceptable excipient and an effective amount of an oligonucleotide which is nuclease resistant and comprises from about 3 to about 50 nucleotides, which nucleotides are complementary to the ribonucleotide reductase gene or the secA gene of a microorganism.

§5. The pharmaceutical composition comprising a pharmaceutically acceptable excipient and an effective amount of an oligonucleotide comprising from about 3 to about 50 nucleotides which is capable of binding to the ribonucleotide reductase gene or the secA gene of a microorganism, wherein the oligonucleotide comprises all or part of a sequence selected from the group consisting of SEQ IDNO :22; SEQ IDNO:43; SEQ IDNO:62; SEQ ID NO: 74; SEQ ID NO:75; SEQ ID NO:76; SEQ IDNO: 143; SEQ IDNO:145; SEQ IDNO:152; SEQ ID NO: 164; SEQ IDNO:176; SEQ ID NO:186; SEQ IDNO:188; SEQ IDNO:189; SEQ ID NO: 191; SEQ ID <RTI NO:192; SEQ IDNO: 195; SEQ IDNO:197; SEQ ID NO:206; SEQ ID NO:212; SEQ ID NO:220;SEQ ID NO:229; SEQ ID NO:235; SEQ ID NO:254; SEQ IDNO:261; SEQ ID NO:262; SEQ ID NO:263; SEQ ID NO:264; andSEQ ID NO:265.

§6. A method of inhibiting the expression of a ribonucleotide reductase gene in a microorganism having a ribonucleotide reductase gene, comprising administering to said microorganism or to a cell infected with said microorganism an effective amount of an antisense oligonucleotide comprising from at least about 3 nucleotides which are complementary to the ribonucleotide reductase gene of the microorganism under conditions such that the expression of the ribonucleotide reductase gene is inhibited.

§7. The method according to §6, wherein said microorganism is a bacterial cell.

§8. The method according to §6, wherein said microorganism is a virus.

§9. The method according to §6 wherein the antisense oligonucleotide comprises a sequence selected from the group consisting of SEQ ID NO:22; SEQ ID NO:43; SEQ ID NO:62; SEQ IDNO:74; SEQ ID NO:75; SEQ ID NO: 76; SEQ IDNO: 143; SEQ IDNO:145; and SEQ IDNO:152.

§10. A method of inhibiting the expression of the secA gene in a microorganism having a secA gene, comprising administering to said microorganism an effective amount of an antisense oligonucleotide comprising from at least about 3 nucleotides which are complementary to the secA gene of the microorganism under conditions such that the secA gene is inhibited.

§11. The method according to §10, wherein said microorganism is a bacterial cell.

§ 12. The method according to §11 wherein the antisense oligonucleotide comprises a sequence selected from the group consisting of SEQ ID NO:164; SEQ IDNO:176; SEQ IDNO:186; SEQ IDNO:188; SEQ IDNO:189; SEQ IDNO:191; SEQ IDNO:192; SEQ ID NO:195; SEQ IDNO:197; SEQ IDNO:206; SEQ ID NO:212; SEQ IDNO:220; SEQ IDNO:229; SEQ IDNO:235; SEQ ID NO:254; SEQ ID NO:261; SEQ ID NO:262; SEQ ID NO:263; SEQ ID NO:264; and SEQ ID NO:265.

§13. A method of inhibiting the growth of a microorganism having a ribonucleotide reductase gene or a secA gene, which method comprises identifying the microorganism and administering to said microorganism an effective amount of an antisense oligonucleotide comprising from at least about 3 nucleotides which are complementary to either the ribonucleotide reductase gene or the secA gene of the microorganism under conditions whereby the growth of the microorganism is inhibited.

§ 14. The method according to §13, wherein said microorganism is a bacterial cell.

§15. The method according to § 13, wherein said microorganism is a virus.

§16. The method according to §13 wherein the antisense oligonucleotide comprises a sequence selected from the group consisting ofSEQ ID NO:22; SEQ ID NO:43; SEQ ID NO:62; SEQ ID NO:74; SEQ ID NO:75; SEQ ID NO:76; SEQ ID NO:143; SEQ ID NO:145; SEQ ID NO:152; SEQ ID NO:164; SEQ ID NO:176; SEQ IDNO:186; SEQ IDNO:188; SEQ IDNO:189; SEQ IDNO:191; SEQ ID NO:192; SEQ IDNO:195; SEQ IDNO:197; SEQ ID NO: 206; SEQ ID NO:212; SEQ ID NO-220; SEQ ID NO:229; SEQ ID NO:235; SEQ ID NO:254; SEQ ID NO:261; SEQ ID NO-262; SEQ ID NO:263; SEQ ID NO:264; and SEQ ID NO:265.

§ 17. A method for treating a mammalian pathologic condition mediated by microorganisms, which method comprises identifying a mammal having a pathologic condition mediated by microorganisms having a ribonucleotide reductase gene or a secA gene and administering to said mammal an effective amount of an antisense oligonucleotide comprising at least about 3 nucleotides which are complementary to either the ribonucleotide reductase gene or the secA gene of the microorganism under conditions such that the growth of the microorganism is inhibited.

**Claims**

1. An antisense oligonucleotide comprising from 15 to 50 nucleotides complementary to a mRNA encoding a ribonucleotide reductase or a secA protein of a bacterium, wherein the oligonucleotide has reduced internal duplex formation, reduced hair-pin formation and reduced homooligomer/sequence repeats and comprises a sequence of at least 15 nucleotides selected from any one of SEQ ID NOs: 14 - 265, and wherein the oligonucleotide is nuclease resistant and inhibits the growth of the bacterium.

2. The antisense oligonucleotide according to Claim 1, wherein said antisense oligonucleotide comprises from 15 to 35 nucleotides.

3. The antisense oligonucleotide according to Claim 1 or 2, wherein the antisense oligonucleotide comprises a sequence selected from the group consisting of SEQ ID NO:14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264 and 265.

4. The antisense oligonucleotide according to Claim 3, wherein the antisense oligonucleotide comprises a sequence selected from the group consisting of SEQ ID NO:22, 43, 62, 74, 75, 76, 143, 145, 152, 164, 176, 186, 188, 189, 191, 192, 195, 197, 206, 212, 220, 229, 235, 254, 261, 262, 263, 264 and 265.

5. The antisense oligonucleotide according to Claim 3 or 4, wherein the antisense oligonucleotide comprises a sequence selected from the group consisting of SEQ ID NO:22, 43, 62, 74, 75, 76, 143, 145 and 152.

**6.** The antisense oligonucleotide according to Claim 3, wherein the antisense oligonucleotide comprises a sequence selected from the group consisting of SEQ ID NO:164, 176, 186, 188, 189, 191, 192, 195, 197, 206, 212, 220, 229, 235, 254, 261, 262, 263, 264 and 265.

**7.** The antisense oligonucleotide according to any one of Claims 1 to 6, wherein said oligonucleotide comprises one or more phosphorothioate intemucleotide linkages.

**8.** The antisense oligonucleotide according to any one of Claims 1 to 6, wherein said antisense oligonucleotide is within a vector.

**9.** A recombinant vector comprising a sequence encoding the antisense oligonucleotide according to any one of Claims 1 to 6.

**10.** A pharmaceutical composition comprising a pharmaceutically acceptable excipient and the antisense oligonucleotide according to any one of Claims 1 to 7.

**11.** A pharmaceutical composition comprising a pharmaceutically acceptable excipient and an effective amount of the vector according to Claim 9.

**12.** An *in vitro* method of inhibiting the growth of a bacterium having a ribonucleotide reductase gene or a secA gene, which method comprises the steps of:

(a) identifying the bacterium; and
(b) administering to said bacterium the antisense oligonucleotide according to any one of Claims 1 to 7 under conditions whereby the growth of the bacterium is inhibited.

**13.** The method according to Claim 12, wherein said bacterium is *Escherichia coli.*

**14.** The antisense oligonucleotide as defined by any one of Claims 1 to 7, for use as a medicament in a mammal.

**15.** The antisense oligonucleotide as defined by any one of Claims 1 to 7, for use as a medicament in a human.

**16.** The antisense oligonucleotide according to any one of Claims 1 to 7, for use in inhibiting growth of a bacterium in a mammal.

**17.** The antisense oligonucleotide according to Claim 16, wherein said mammal has a pathological condition mediated by the bacterium.

**18.** The antisense oligonucleotide according to Claim 16, wherein said bacterium is *Escherichia coli.*

**19.** The antisense oligonucleotide according to any one of Claims 16 to 18, wherein said mammal is a human.

**20.** Use of an antisense oligonucleotide as defined by any one of Claims 1 to 8 for the manufacture of a medicament for inhibiting growth of a bacterium in a mammal.

**21.** Use of the vector according to Claim 9 for the manufacture of a medicament for inhibiting growth of a bacterium in a mammal.

**22.** Use according to Claim 20 or 21, wherein said mammal has a pathological condition mediated by the bacterium.

**23.** Use according to any one of Claims 20 to 22, wherein said bacterium is *Escherichia coli.*

**24.** Use according to any one of Claims 20 to 23, wherein said mammal is a human.

**25.** An antisense oligonucleotide comprising from 15 to 50 nucleotides complementary to a mRNA encoding a ribonucleotide reductase or a secA protein of a bacterium for use in inhibiting growth of a bacterium in a mammal, wherein the oligonucleotide has reduced internal duplex formation, reduced hair-pin formation and reduced homooligomer/sequence repeats and comprises a sequence of at least 15 nucleotides selected from any one of SEQ ID NOs: 14 - 265.

**26.** The antisense oligonucleotide according to Claim 25, wherein said antisense oligonucleotide comprises from 15 to 35 nucleotides.

**27.** The antisense oligonucleotide according to Claim 25 or 26, wherein the antisense oligonucleotide comprises a sequence selected from the group consisting of SEQ ID NO:14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157,158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264 and 265.

**28.** The antisense oligonucleotide according to Claim 27, wherein the antisense oligonucleotide comprises a sequence selected from the group consisting of SEQ ID NO:22, 43, 62, 74, 75, 76, 143, 145, 152, 164, 176, 186, 188, 189, 191, 192, 195, 197, 206, 212, 220, 229, 235, 254, 261, 262, 263, 264 and 265.

**29.** The antisense oligonucleotide according to Claim 27, wherein the antisense oligonucleotide comprises a sequence selected from the group consisting of SEQ ID NO:22, 43, 62, 74, 75, 76, 143, 145 and 152.

**30.** The antisense oligonucleotide according to Claim 27, wherein the antisense oligonucleotide comprises a sequence selected from the group consisting of SEQ ID NO:164, 176, 186, 188, 189, 191, 192, 195, 197, 206, 212, 220, 229, 235, 254, 261, 262, 263, 264 and 265.

**31.** The antisense oligonucleotide according to any one of Claims 25 to 30, wherein said oligonucleotide comprises one or more phosphorothioate intemucleotide linkages.

**32.** The antisense oligonucleotide according to any one of Claims 25 to 30, wherein the antisense oligonucleotide sequence is within a vector.

**33.** The antisense oligonucleotide according to any one of Claims 25 to 32, wherein said mammal has a pathological condition mediated by the bacterium.

**34.** The antisense oligonucleotide according to Claim 33, wherein said bacterium is *Escherichia coli.*

**35.** The antisense oligonucleotide according to any one of Claims 25 to 34, wherein said mammal is a human.

**36.** The recombinant vector according to Claim 9, for use in inhibiting growth of a bacterium in a mammal.

**37.** The recombinant vector according to Claim 36, wherein said mammal has a pathological condition mediated by the bacterium.

**38.** The recombinant vector according to Claim 37, wherein said bacterium is *Escherichia coli*.

**39.** The recombinant vector according to any one of Claims 36 to 38, wherein said mammal is a human.

```
   1 atgaatcaga atctgctggt gacaaagcgc gacggtagca cagagcgcat caatctcgac
  61 aaaatccatc gcgttctgga ttgggcggca gaaggactgc ataacgtttc gatttcccag
 121 gtcgagctgc gctcccacat tcagttttat gacggtatca agacctctga catccacgaa
 181 accattatca aggctgccgc agacctgatc tcccgtgatg cgccggatta tcagtatctc
 241 gccgcgcgcc tggcgatctt ccacctgcgt aaaaaagcct acggccagtt tgagccgcct
 301 gcgctgtacg accacgtggt gaaaatggtc gagatgggca aatacgataa tcatctgctg
 361 gaagactaca cggaagaaga gttcaagcag atggacacct ttatcgatca cgaccgtgat
 421 atgaccttct cttatgctgc cgttaagcag ctggaaggca aatatctggt acagaaccgc
 481 gtgaccggcg aaatctatga gagcgcccag ttcctttata ttctagttgc cgcgtgcttg
 541 ttctcgaact acccgcgtga aacgcgcctg caatatgtga agcgttttta cgacgcggtt
 601 tccacattta aaatttcgct gccgacgcca atcatgtccg gcgtgcgtac cccgactcgt
 661 cagttcagct cctgcgtact gatcgagtgc ggtgacagcc tggattccat caacgccacc
 721 tccagcgcga ttgttaaata cgtttcccag cgtgccggga tcggcatcaa cgccgggcgt
 781 attcgtgcgc tgggtagccc gattcgcggt ggtgaagcgt tccataccgg ctgcattccg
 841 ttctacaaac atttccagac agcggtgaaa tcctgctctc agggcggtgt gcgcggcggt
 901 gcggcaacgc tgttctaccc gatgtggcat ctggaagtgg aaagcctgct ggtgttgaaa
 961 aacaaccgtg gtgtggaagg caaccgcgtg cgtcatatgg actacggggt acaaatcaac
1021 aaactgatgt atacccgtct gctgaaaggt gaagatatca ccctgttcag cccgtccgac
1081 gtaccggggc tgtacgacgc gttcttcgcc gatcaggaag agtttgaacg tctgtatacc
1141 aaatatgaga aagacgacag catccgcaag cagcgtgtga aagccgttga gctgttctcg
1201 ctgatgatgc aggaacgtgc gtctaccggt cgtatctata ttcagaacgt tgaccactgc
1261 aatacccata gcccgtttga tccggccatc gcgccagtgc gtcagtctaa cctgtgcctg
1321 gagatagccc tgccgaccaa accgctgaac gacgtcaacg acgagaacgg tgaaatcgcg
```

*FIG. 1A*

| | |
|---|---|
| 1381 | ctgtgtacgc tgtctgcttt caacctgggc gcaattaata acctggatga actggaagag |
| 1441 | ctggcaattc tggcggttcg tgcacttgac gcgctgctgg attatcagga ttacccgatc |
| 1501 | ccggccgcca aacgtggagc gatgggtcgt cgtacgctgg gtattggtgt gatcaacttc |
| 1561 | gcttactacc tggcgaacga cggtaaacgc tactccgacg gcagcgccaa caacctgacg |
| 1621 | cataaaacct tcgaagccat tcagtattac ctgctgaaag cctctaatga gctggcgaaa |
| 1681 | gagcaaggcg cgtgcccgtg gtttaacgaa accacttacg cgaaagggat cctgccgatc |
| 1741 | gatacctata agaaagatct ggataccatc gctaatgagc cgctgcatta cgactgggaa |
| 1801 | gctctgcgtg agtcaatcaa aacgcacggt ctgcgtaact ccacgctttc tgctctgatg |
| 1861 | ccgtccgaga cttcttcgca gatctctaac gccactaacg gtattgaacc gccgcgcggt |
| 1921 | tacgtcagca tcaaagcgtc gaaagacggt attttgcgcc aggtggtgcc ggactacgag |
| 1981 | cacctgcacg acgcctatga gctgctgtgg gaaatgccgg gtaacgatgg ttatctgcaa |
| 2041 | ctggtgggta tcatgcagaa atttatcgat cagtcgatct ctgccaacac caactacgat |
| 2101 | ccgtcacgct tcccgtcagg aaaagtgccg atgcagcagt tgctgaaaga cctgctcacc |
| 2161 | gcctacaaat tcggggtcaa aacactgtat tatcagaaca cccgtgacgg cgctgaagac |
| 2221 | gcacaagacg atctggtgcc gtcaatccag gacgatggct gcgaaagcgg cgcatgtaag |
| 2281 | atctga |

*FIG. 1B*

```
7381 ctggtgccgt caatccagga cgatggctgc gaaagcggcg catgtaagat ctgatattga
7441 gatgccggat gcggcgtaaa cgccttatcc ggcctacggc tcggtttgta ggcctgataa
7501 gacgcgccag cgtcgcatca ggctccgggt gccggatgca gcgtgaacgc cttatccggc
7561 ctacggctcg gatttgtagg cctgataaga cgcgccagcg tcgcatcagg cacaggatgc
7621 ggcgtaaaat gccttatccg gcattaaact cccaacagga cacactcatg gcatatacca
7681 ccttttcaca gacgaaaaat gatcagctca aagaaccgat gttctttggt cagccggtca
7741 acgtggctcg ctacgatcag caaaaatatg acatcttcga aaagctgatc gaaaagcagc
7801 tctctttctt ctggcgtccg gaagaagttg acgtctcccg cgaccgtata gattaccagg
7861 cgctgccgga gcacgaaaaa cacatcttta tcagcaacct gaaatatcag acgctgctgg
7921 attccattca gggtcgtagc ccgaacgtgg cgctattgcc gcttatttct attccggaac
7981 tggaaacctg ggtcgaaacc tgggcgttct cagaaacgat tcattcccgt tcctatactc
8041 atatcattcg taatatcgtt aacgatccgt ctgttgtgtt tgacgatatc gtcaccaacg
8101 agcagatcca gaaacgtgcg gaagggatct ccagctatta cgatgagctg atcgaaatga
8161 ccagctactg gcatctgctg ggcgaaggta cccacaccgt taacggtaaa actgtgaccg
8221 ttagcctgcg cgagctgaag aaaaaactgt atctctgcct gatgagcgtt aacgcgctgg
8281 aagcgattcg tttctacgtc agctttgctt gttccttcgc atttgcagaa cgcgaattga
8341 tggaaggcaa cgccaaaatt attcgcctga ttgcccgcga cgaagccctg cacctgaccg
```

*FIG. 2A*

8401 gcacccagca tatgctgaat ctgctgcgca gcggcgcgga cgatcctgag atggcggaaa
8461 ttgccgaaga gtgtaagcag gagtgctatg acctgtttgt tcaggcagct caacaggaga
8521 aagactgggc ggattatctg ttccgcgacg gttcgatgat tggtctgaat aaagacattc
8581 tctgccagta cgttgaatac atcaccaata tccgtatgca ggcagtcggt ttggatctgc
8641 cgttccagac gcgctccaac ccgatcccgt ggatcaacac ttggctggtg tctgataacg
8701 tgcaggttgc tccgcaggaa gtggaagtca gttcttatct ggtcgggcag attgactcgg
8761 aagtggacac cgacgatttg agtaacttcc agctctgatg gcccgcgtta ccctgcgcat
8821 cactggcaca caactgctgt gccaggatga acacccttcc cttctggcgg cgctggaatc
8881 ccacaatgtg gcggttgagt accagtgtcg cgaaggttac tgcggctcct gtcgcacacg

*FIG. 2B*

```
301  gtgaacgtcg atctggtgcc ggatgcagcg gatacgctcc gggcgcaagg atttcgtcaa
361  ttaccggtgg tgatggcggg cgatttgagc tggtctggct tccgcccgga catgattaac
421  cgtctgcacc cgacacccca cgcggcaaac gcatgagcgc gctcgtctac ttctccagca
481  gctctgaaaa tacgcaccgc tttatgcagc gtctggggct gcctgccacg cgtattccgc
541  tcaatgagcg ggagcgaatt caggtagacg aaccgtacat tctggttgtg ccgtcatacg
601  gcggcggcgg gatggccggt gcggtgccgc gacaggtgat ccgcttttta aatgatgaac
661  acaaccgggc gcgcattcgc ggcgttatcg cctccggtaa tcgcaatttc ggcgatgcct
721  ggggatgcgc tggcgatgtg atagcacaaa aatgcggcgt cccctggctg taccgctttg
781  agctcatggg cacacaacgc gacatcgata atgtccgaaa aggagtaaat gaattttggc
841  aacaactacc ccggagcgcg taatgcagga aaccatggat taccacgccc tgaacgcgat
901  gctgaatctt tacgataaag caggccatat tcagttcgac aaggaccagc aggcgatcga
961  cgccttcttt gccacccacg tccgcccgca ttccgtgacg tttgccagcc agcatgaacg
1021 tctggggacg ctggttcggg aagggtatta cgatgacgcc gtcctcgcgc gttacgaccg
1081 cgccttcgtc cttcgcctgt tcgagcacgc ccatgccagc ggctttcgct tccagacgtt
1141 tcttggcgcc tggaagttct ataccagtta cacgctgaaa accttcgacg gcaaacgtta
1201 tctggaacac tttgaagatc gggtgacaat ggtggcgttg acgctggcgc agggtgacga
1261 aacgctggcc acccaactga ccgatgaaat gctttctggt cgctttcagc ccgctacccc
1321 gactttttta aattgcggca aacagcagcg tggggaactg gtctcctgct tcctgctccg
1381 tatcgaagac aacatggagt cgatcgggcg ggcggtgaat tcggcgctgc aactctccaa
1441 acgcggcggc ggcgtcgcgt ttttactctc caatctgcgc gaggcgggcg cgccgatcaa
1501 acgcattgag aatcagtctt ccggcgtgat cccggtgatg aaaatgctgg aagacgcgtt
1561 ttcgtatgcc aaccaacttg gcgcgcgcca gggggccggc gcggtttatc tccatgcgca
1621 ccatccggat attctgcgtt ttctggatac caaacgagaa aacgctgacg aaaaaatccg
```

*FIG. 3A*

```
1681 gatcaaaacg ctctctctcg gcgtggtgat cccggatatc accttccggc tggcgaaaga
1741 aaacgcgcaa atggcgctct tttcgcccta tgacatacaa cgacgctacg gcaaaccgtt
1801 tggcgatatc gccattagcg aacggtacga tgaattaatt gccgatccgc acgtgcgcaa
1861 aacctatatt aacgcccgtg actttttttca aacactggcg gagattcagt tcgaatccgg
1921 gtatccctac atcatgtttg aagatacggt aaaccgcgcg aatcccattg ctggtcgcat
1981 taatatgagc aacctgtgct cagaaatttt acaggtcaat agcgcttccc gttacgacga
2041 taaccttgac tatacccaca tcgggcatga catctcctgc aatctcggct cgctgaatat
2101 cgctcacgtc atggattcac cggacattgg ccgtaccgta gaaaccgcta ttcgcggcct
2161 gacggcggtg tcggacatga gccatatacg cagcgtgccc tcaatagccg ccggtaatgc
2221 cgcctctcat gccatcggtc tgggccagat gaatctgcat ggctatctgg cgagggaagg
2281 tattgcctac ggttcgccgg aggcgttgga tttcaccaat ctctattttt acaccattac
2341 ctggcatgcc gtgcatactt caatgcggct agcccgcgaa cgcggcaaaa ccttcgccgg
2401 atttgcgcag tcgcgctatg ccagcggcga ctattttacg cagtatttac aggacgactg
2461 gcaaccgaaa acagcgaaag tcagggcgct atttgcccgc agcggcatta cgctgcccac
2521 acgagaaatg tggctaaagc tgcgcgacga tgtgatgcgc tatggcatct ataaccaaaa
2581 tttgcaggcg gtgccgccga ccggttcgat ttcttacatt aatcatgcga cctccagcat
2641 tcatccgatt gtggccaaaa ttgagattcg caaagagggc aaaaccgggc gtgtgtatta
2701 ccccgcgccg tttatgacca atgaaaacct ggacatgtat caggatgctt acgatatcgg
2761 tccggaaaaa attattgata cctatgccga ggccacgcgc cacgtcgatc aagggctgtc
2821 gctcaccctg tttttccccg ataccgccac gacccgcgat atcaacaagg cgcagatcta
2881 tgcctggcga aaaggtatta agtccctgta ttacatccgg cttcgccagt tggcgctgga
2941 aggtactgaa attgaaggct gcgtatcctg cgcgctataa ggaaagccat atgaaattat
3001 ctcgtattag cgccatcaac tggaacaaga tccaggacga caaagatctg gaggtatgga
```

*FIG. 3B*

```
3061 accggctgac cagtaacttc tggctgccgg aaaaagtgcc gttatcgaat gatattccgg
3121 cctggcagac gctgagcgcc gccgaacagc agctcaccat tcgcgtgttt acgggactta
3181 cgctgctcga cactatccag aacatcgcag gcgcgccgtc gttaatggca gatgccatca
3241 cgccgcatga agaggcagtg ctgtcgaaca tcagctttat ggaagcggta cacgcccgct
3301 cttacagttc tattttctcc acgctgtgcc agacgaaaga ggttgatgcc gcctacgcct
3361 ggagcgaaga aaacccaccg cttcagcgta aggcgcagat tattttagct cattacgtca
3421 gcgatgaacc gctaaagaaa aagattgcca gcgtcttttt agagtctttt ctgttctatt
3481 ccggcttctg gttgccgatg tatttctcca gccgcggtaa gctcacgaac actgccgacc
3541 tgattcgttt aatcattcgc gatgaagcgg ttcacggtta ttatattggc tataagtatc
3601 agatagcgct acaaaaacta tcggcaatcg agcgtgaaga gttaaagctt ttcgcgctgg
3661 atttgttgat ggaactgtac gacaacgaaa tccgctacac agaagcgtta tatgcggaaa
3721 ccggctgggt taacgacgtc aaagccttct tgtgctacaa cgccaataaa gccttaatga
3781 acctgggtta tgaggcgtta tttccgccgg agatggcaga cgtgaatccc gcaatccttg
3841 ccgcgctctc gccgaatgcc gacgaaaacc atgatttctt ttccggctca ggttcatctt
3901 atgtgatggg gaaaacagtc gaaaccgaag acgaagactg gaatttttaa ccttacgggc
3961 atgggaaata acgttacatt tcccatgcct ttatttcaag caatagggag tcaaatcgcg
4021 caaatattac aacatgtcct acactcaata cgagtgacat tattcacctg gattccccca
4081 attcaggtgg atttttgctg gttgttccaa aaaatatctc ttcctcccca ttcgcgttca
4141 gcccttatat catgggaaat cacagccgat agcacctcgc aatattcatg ccagaagcaa
4201 attcagggtt gtctcagatt ctgagtatgt tagggtagaa aaaggtaact atttctatca
4261 ggtaacatat cgacataagt aaataacagg aatcattcta ttgcatggca attaaattag
4321 aagtgaagaa tctgtataaa atatttggag agcatccgca gcgtgccttc aaatatattg
4381 aaaagggact atcgaaagag caaatactgg aaaaaacggg gctatcgctt ggcgttaaag
```

*FIG. 3C*

```
4441 acgccagtct ggccattgaa gaaggcgaga tatttgtcat catgggatta tccggctcgg
4501 gtaaatccac aatggtacgc cttctcaatc gcctgattga acccacccgc ggacaggtac
4561 tgattgacgg cgttgatatt gccaaaatat cagacgctga gcttcgcgag gtgcgcagga
4621 aaaagattgc gatggtcttc cagtcatttg cgctcatgcc gcatatgacc gtgctggata
4681 atacggcatt cggtatggaa ttagcgggca tcgcggcgca agagcgtcgc gaaaaagcgc
4741 tggacgcctt gcgtcaggtg ggcttgagga attacgctca cgcctacccg gatgaacttt
4801 ccggtgggat gcgtcagcgt gttgggcttg cccgcgcgct ggcaatcaac cctgatatct
4861 tattaatgga tgaagcgttt tccgccctcg atcc
```

*FIG. 3D*

```
   1 gaattcttat tttccctagc tttggattta ttctcacttc ctatgatctt ttattctcga
  61 ttattatttt tgctttggca attattatca tttttcgaca taaaacaaac ctcaaaagaa
 121 tcaaaaatca ttgtgaatcc cttgtcccct ttggtttaaa cttatcgaga caaaaagaaa
 181 aatagcacaa tatattgtgt tgtttttctt tttttacata atttaacact atatctagta
 241 tctttaattt gactagatat tttttttacg ctaaataaga ctataaaaac tcgagaaaaa
 301 gtcaaggact ttttactccc gtctaaaaaa tatattggcc caaaaggaga tttaaaatgg
 361 ttacagttta ttctaaaaac aattgtatgc aatgcaaaat ggtcaaaaaa tggctttctg
 421 aacacgaaat tgcatttaac gaaatcaata ttgatgaaca gcctgaattt gtcgaaaaag
 481 taattgaaat gggttttcga gctgctcctg taatcacaaa agatgatttc gccttttctg
 541 gtttccgtcc ttctgaatta gcaaagttgg cttaatatga aacttgctta tttcagtgtg
 601 actggacaaa cgcgtcgttt tgtttctaaa acagacttgc cgaatgtcga aattacacct
 661 gacgatgatt tagagatgga cgagcctttc cttttgataa ctccctctta tgctgaagaa
 721 tcaccaaccg tttctaaatc aatagacgtt atggactcgg tttttgactt tatggcttat
 781 aatgataatt ataaacattg tcgtggaatt atcggcactg gaaatcgtaa ttttgctggc
 841 atctatattt ttaccgctaa agaagtttca gcaaaatatc aaattccact tttatatgat
 901 tttgagttta atggtacgcc agctgatgtt gctgctgttg aaaaactcgc tgcacagctt
 961 gatcaaggag cgaaagtcac ctttaaaaat ccgctgtgat tttttatggc ttcaccctat
1021 ttgagtgaag ctt
```

*FIG. 4*

```
   1 cagctgtact ggcataacga catttatact gtcgtataaa attcgactgg
  51 caaatctggc actctctccg gccaggtgaa ccagtcgttt ttttttgaat
 101 tttataagag ctataaaaaa cggtgcgaac gctgttttct taagcacttt
 151 tccgcacaac ttatcttcat tcgtgctgtg gactgcaggc tttaatgata
 201 agatttgtgc gctaaatacg tttgaatatg atcgggatgg caataacgtg
 251 agtggaatac tgacgcgctg gcgacagttt ggtaaacgct acttctggcc
 301 gcatctctta ttagggatgg ttgcggcgag tttaggtttg cctgcgctca
 351 gcaacgccgc cgaaccaaac gcgcccgcaa aagcgacaac ccgcaaccac
 401 gagccttcag ccaaagttaa ctttggtcaa ttggccttgc tggaagcgaa
 451 cacacgccgc ccgaattcga actattccgt tgattactgg catcaacatg
 501 ccattcgcac ggtaatccgt catctttctt tcgcaatggc accgcaaaca
 551 ctgcccgttg ctgaagaatc tttgcctctt caggcgcaac atcttgcatt
 601 actggatacg ctcagcgcgc tgctgaccca ggaaggcacg ccgtctgaaa
 651 agggttatcg cattgattat gcgcatttta ccccacaagc aaaattcagc
 701 acgcccgtct ggataagcca ggcgcaaggc atccgtgctg gccctcaacg
 751 cctcacctaa caacaataaa cctttacttc attttattaa ctccgcaacg
 801 cggggcgttt gagattttat tatgctaatc aaattgttaa ctaaagtttt
 851 cggtagtcgt aacgatcgca ccctgcgccg gatgcgcaaa gtggtcaaca
 901 tcatcaatgc catggaaccg gagatggaaa aactctccga cgaagaactg
 951 aaagggaaaa ccgcagagtt tcgtgcacgt ctggaaaaag gcgaagtgct
1001 ggaaaatctg atcccggaag ctttcgccgt ggtacgtgag gcaagtaagc
1051 gcgtctttgg tatgcgtcac ttcgacgttc agttactcgg cggtatggtt
1101 cttaacgaac gctgcatcgc cgaaatgcgt accggtgaag gaaaaaccct
```

*FIG. 5A*

1151 gaccgcaacc ctccctcctt acctgaacgc actaaccggt aaaggcgtgc
1201 acgtagttac cgtcaacgac tacctggcgc aacgtgacgc cgaaaacaac
1251 cgtccgctgt ttgaattcct tggcctgact gtcggtatca acctgccggg
1301 catgccagca ccggcaaagc gcgaagctta cgcagctgac atcacttacg
1351 gtaccaacaa cgaatacggc tttgactacc tgcgcgacaa catggcgttc
1401 agccctgaag aacgtgtaca gcgtaaactg cactatgcgc tggtggacga
1451 agtggactcc atcctgatcg atgaagcgcg tacaccgctg atcatttccg
1501 gcccggcaga agacagctcg gaaatgtata aacgcgtgaa taaaattatt
1551 ccgcacctga tccgtcagga aaaagaagac tccgaaacct tccagggcga
1601 aggccacttc tcggtggacg aaaaatctcg ccaggtgaac ctgaccgaac
1651 gtggtctggt gctgattgaa gaactgctgg tgaaagaggg catcatggat
1701 gaaggggagt ctctgtactc tccggccaac atcatgctga tgcaccacgt
1751 aacggcggcg ctgcgcgctc atgcgctgtt tacccgtgac gtcgactaca
1801 tcgttaaaga tggtgaagtt atcatcgttg acgaacacac cggtcgtacc
1851 atgcagggcc gtcgctggtc cgatggtctg caccaggctg tggaagcgaa
1901 agaaggtgtg cagatccaga acgaaaacca aacgctggct tcgatcacct
1951 tccagaacta cttccgtctg tatgaaaaac tggcggggat gaccggtact
2001 gctgataccg aagctttcga atttagctca atctacaagc tggataccgt
2051 cgttgttccg accaaccgtc caatgattcg taaagatctg ccggacctgg
2101 tctacatgac tgaagcggaa aaaattcagg cgatcattga agatatcaaa
2151 gaacgtactg cgaaaggcca gccggtgctg gtgggtacta tctccatcga
2201 aaaatcggag ctggtgtcaa acgaactgac caaagccggt attaagcaca
2251 acgtcctgaa cgccaaattc cacgccaacg aagcggcgat tgttgctcag

*FIG. 5B*

2301 gcaggttatc cggctgcggt gactatcgcg accaatatgg cgggtcgtgg
2351 tacagatatt gtgctcggtg gtagctggca ggcagaagtt gccgcgctgg
2401 aaaatccgac cgcagagcaa attgaaaaaa ttaaagccga ctggcaggta
2451 cgtcacgatg cggtactgga agcaggtggc ctgcatatca tcggtaccga
2501 gcgtcacgaa tcccgtcgta tcgataacca gttgcgcggt cgttctggtc
2551 gtcaggggga tgctggttct tcccgtttct acctgtcgat ggaagatgcg
2601 ctgatgcgta tttttgcttc cgaccgagta tccggcatga tgcgtaaact
2651 gggtatgaag ccaggcgaag ccattgaaca cccgtgggtg actaaagcga
2701 ttgccaacgc ccagcgtaaa gttgaaagcc gtaacttcga cattcgtaag
2751 caactgctgg aatatgatga cgtggctaac gatcagcgtc gcgccattta
2801 ctcccagcgt aacgaactgt tggatgtcag cgatgtgagc gaaaccatta
2851 acagcattcg tgaagatgtg ttcaaagcga ccattgatgc ctacattcca
2901 ccacagtcgc tggaagaaat gtgggatatt ccggggctgc aggaacgtct
2951 gaagaacgat ttcgacctcg atttgccaat tgccgagtgg ctggataaag
3001 aaccagaact gcatgaagag acgctgcgtg acggcattct ggcgcagtcc
3051 atcgaagtgt atcagcgtaa agaagaagtg gttggtgctg agatgatgcg
3101 tcacttcgag aaaggcgtca tgctgcaaac gcttgactcc ctgtggaaag
3151 agcacctggc agcgatggac tatctgcgtc agggtatcca cctgcgtggc
3201 tacgcacaga aagatccgaa gcaggaatac aaacgtgaat cgttctccat
3251 gtttgcagcg atgctggagt cgttgaaata tgaagttatc agtacgctga
3301 gcaaagttca ggtacgtatg cctgaagagg ttgaggagct ggaacaacag
3351 cgtcgtatgg aagccgagcg tttagcgcaa atgcagcagc ttagccatca
3401 ggatgacgac tctgcagccg cagctgcact ggcggcgcaa accggagagc

*FIG. 5C*

3451 gcaaagtagg acgtaacgat ccttgcccgt gcggttctgg taaaaaatac
3501 aagcagtgcc atggccgcct gcaataaaag ctaactgttg aagtaaaagg
3551 cgcaggattc tgcgcctttt ttataggttt aagacaatga aaaagctgca
3601 aattgcggta ggtattattc gcaacgagaa caatgaaatc tttataacgc
3651 gtcgcgcagc agatgcgcac atggcgaata aactggagtt tcccggcggt
3701 aaaattgaaa tgggtgaaac gccggaacag gcggtggtgc gtgaacttca
3751 ggaagaagtc gggattaccc cccaacattt ttcgctattt gaaaaactgg
3801 aatatgaatt c

*FIG. 5D*

```
   1 gatctacggc agaactcgtc gcttggagcg ttcgaccgac catctacctg
  51 ttcgacgtcg aactcgacca ctgaacgtaa tcgccgccag cgcaagtcct
 101 gtcagcgcgt ggagatcacc gcgcgtgggc gagggccggt ggtgcgaggt
 151 gaggcctgcg ccgacagctt ctatgccgcg cttgaatcag cggtcgtcaa
 201 actggagagc gtgcgccgcg gtaaggatcg ccgcaaggtg cactacggcg
 251 acaaaacccc ggtttcgctg gccgaggcga ccgcggtggt gccagcgccg
 301 gagaacggct tcaacaccag accagccgag gcacacgatc acgacggtgc
 351 cgtcgtcgag cgggagcctg ggcggatcgt tcgcaccaaa gaacacccgg
 401 ccaagccgat gtcggtcgat gacgcgctct accagatgga gctggttgga
 451 cacgacttct tcttgttcta cgacaaggac accgaacggc cgtcggtggt
 501 ctaccgccgg cacgcctacg actacggctt gatccgtctg gcgtgatcgg
 551 cggcgcgcgc cgctcgtcac ctaccatggg agtcgcctta tctaaagact
 601 cctacacatg cggggacata gctgtgctgt cgaagttgct gcgccttggc
 651 gaaggtcgca tggtcaagcg cctcaagaag gtggcggact atgtcggcac
 701 tttgtccgac gatgtcgaga aactcaccga cgccgagctg agggcgaaaa
 751 ccgacgagtt caagcggcgg ctggccgacc agaaaaaccc agaaaccctc
 801 gacgacctgt tgcccgaggc cttcgccgtg gcccgcgagg ccgcctggcg
 851 ggtgctggac cagcggccgt tcgacgtgca ggtgatgggt gcggccgccc
 901 tgcacctggg caacgttgcc gagatgaaga ccggtgaagg caagaccctg
 951 acctgtgtgt tgcccgctta cctcaatgcg ctggccggca acggcgtgca
1001 catcgtcacc gtcaacgact acctggctaa acgcgacagt gagtggatgg
1051 gccgcgtgca ccgcttcctc gggcttcagg tcggggtgat tttcgccacc
1101 atgacacccg atgaacgccg ggtggcctat aacgccgaca tcacctacgg
```

*FIG. 6A*

```
1151 caccaataac gagtttgggt tcgactacct gcgcgacaac atggcgcact
1201 cactggatga tctggtgcag cgcgggcacc attacgccat tgtcgacgag
1251 gtcgattcca tcctgatcga cgaggcccgc accccgctga tcatctccgg
1301 tcccgccgac ggcctccaac tggtacaccg agttcgccgg ttggcgccgc
1351 tgatggaaaa ggacgtccac tacgaggtcg atctacgcaa acgcaccgtc
1401 ggcgtgcacg agaagggtgt ggaattcgtc gaagaccagc tcggcatcga
1451 caacctgtac gaggccgcca actcgccgtt ggtcagctat ctcaacaacg
1501 ctctgaaggc caaagagctg ttcagccgcg acaaggacta catcgtccgc
1551 gatggtgagg tgctcatcgt cgacgagttc accggccggg tgctgatcgg
1601 ccgccgctac aacgagggca tcgaccaggc catcgaggcc aaggagcacg
1651 tcgagatcaa ggccgagaac cagacgctgg ccaccatcac gctgcagaac
1701 tacttccggc tctacgacaa gctcgccggc atgaccggca ccgcccagac
1751 ggaggcggcc gagctgcacg agatctacaa gctgggcgtg gtcagcatcc
1801 cgaccaacat gccgatgatc cgtgaagacc agtccgacct gatctacaag
1851 accgaggagg ccaagtacat cgcggtggtc gacgacgtcg ccgagcgcta
1901 cgcgaaggga cagccggtgc tgatcggcac caccagcgtg gagcgctcgg
1951 agtatctgtc gcggcagttc accaagcggc gcatcccgca caatgtgctc
2001 aacgccaagt accacgagca agaggcgacc atcatcgcgg tggcgggccg
2051 ccgcggcggc gtcaccgtcg ccaccaacat ggccggtcgc ggcaccgaca
2101 ttgtgctggg cggcaacgtc gactttctca ccgatcagcg gctgcgcgaa
2151 cggcctggat ccggtggaga cgcccgagga gtacgaggcg gcctggcact
2201 ccgaactgcc catcgtcaaa gaggaagcca gcaaggaggc caaggaagta
2251 atcgaggccg gcggctgtac gtgctgggca ccgagcggcc acgagtcgcg
```

*FIG. 6B*

```
2301 gcggatcgac aaccagttgc gtggccggtc cggccgccag gggaccccgg
2351 ggagtcgcgc ttctatttgt cgctgggtga cgagctgatg cgccgcttca
2401 atggcgcggc cttggagacc ttgttgacca ggctgaacct gcccgacgac
2451 gtgccgatcg aagccaagat ggtcacccgg gccatcaaga gcgcccagac
2501 ccaggtcgag cagcagaact ttgaggtccg caagaacgtc ctcaaatacg
2551 acgaggtgat gaaccagcag cgcaaggtca tctacgccga gcgccggcgc
2601 atcctcgaag gcgaaaacct caaggaccag gcgctggaca tggtccgcga
2651 tgtcatcacc gcctacgtcg acggcgcgac cggcgaaggc tatgccgaag
2701 attgggatct ggacgcgttg tggacggcac tcaaaaccct ctatccggag
2751 gggatcaccg ccgactcgct gacccgcaag gaccacgaat tcgagcgcga
2801 cgatctcacc cgcgaggagt tgctggaggc actactcaag gacgccgaac
2851 gtgcctatgc cgcacgggaa gccgaactcg aggaaatcgc cggcgagggt
2901 gcgatgcgcc agctggaacg caacgtgctg ctcaacgtca tagaccgtaa
2951 gtggcgtgaa cacctctacg agatggacta cctcaaggag ggtatcgggc
3001 tgcgcgcgat ggcgcacggc gatccgttgg tcgagtacca gcgtgagggc
3051 tacgacatgt tcatggccat gctcgacggc atgaaagagg aatcggtcgg
3101 cttcctgttc aacgtcaccg tggaggcggt ccccgccccg ccggttgccc
3151 cggctgccga acccgcagag cttgccgaat tcgccgccgc ggccgcagcc
3201 gcgggcagca acgcagcgcg gtcgatggtg gcgcgcgcga aagagctcca
3251 agtgcattac gcgccaaggg tgttgccagc gagtcgcccg ctttgaccta
3301 ttccggtccc gcggaggatg gctcggctca ggtgcagcgc aacggcggtg
3351 gagcccacaa gacgccggcc ggagtgccgg ccggtgctag ccggcgcgag
3401 cggcgcgaac gcgcccgccg acaaggccgc ggcgccaagc cgccgaaatc
```

*FIG. 6C*

```
3451 ggtcaagaag cgttagcgcg taggttgcag atgggtgtat cggtttctca
3501 gttcccagaa gtcacttccc ggcacacccc ggccccggcg cgcatgcaca
3551 tttcgttgca cggcgggcaa ggggttcgct aatctcaccc gttcgtcgac
3601 cttcgtcggc gtcggttctg ctggtagcgg ggttcggcgc tttcctggcg
3651 tttctcgact cgacaatcgt caacatcgcg ttcccggata tccagcgttc
3701 cttcccgtcc tacgacatcg ggagcctgtc ctggattctg aacggctata
3751 acatgctctt cgccgccttc atggttgcgg ccggcaggtt ggccgatttg
3801 ctgggccgca gacgacattc ctgtccggtg tgctggtgtt caccattgcg
3851 tccgggctgt gcgccgtcgc cggcagtgtc gagcagttgg tggcgttccg
3901 ggtgctgcag ggcatcgggg ctgcgatact cgtgcctcgt tcgctcgcac
3951 tggtcgttga gggcttcgac cgggccgccg cgcgcacgct atcggcctgt
4001 ggggtgcggc ggcagcgatc cactagttct agagcggcgc accgc
```

*FIG. 6D*

```
  1 tcaaacacca gaccagaagg aggcaacacg atcacggacg gtgccgttcg
 51 tcgagcggga gcctggggcg gatcgttcgc accaaagaac aacccggcca
101 cgccgatgtc ggtcgatgac gcgctctacc agatggagct ggttggacac
151 gacttcttct tgttctacga caaggacacc gaacggccgt cggtggtcta
201 ccgccggcac gcctacgact acggcttgat ccgtctggcg tcatcggcgg
251 cgcgcgccgc gtcgtcacct accatgggag tcgccttatc taaagactcc
301 tacacatgcg gggacatagc tgtgctgtcg aagttgctgc gccttggcga
351 aggtcgcatg gtcaagcgcc tcaagaaggt ggccgactat gtcggcactt
401 tgtccgacga tgtcgagaaa ctcaccgacg ccgagctgag ggcgaaaacc
451 gacgagttca agcaggctgg ccgaccagaa aaacccagaa accctcgacg
501 acctgttgcc cgaggccttc accgtgcccc gcgagacccg cctgccgggt
551 gctggaccaa cgaccgttcg acgtgcaggt gatgggtacg accgccctgc
601 acctgggcga cgttgccgag atgtagaccg gtgaaggcaa gaccctgacc
651 tgtgttttac ccgcttacct caatgccctg gccgccaacg gcgtgcacgt
701 agttaccgtc aacgactacc tggctaaacg cgacagtgag tggatgggcc
751 gcgtgcaccg cttcctcggg cttcaggtcg gggtgatttt ggccaccatg
801 acacccgatg aacgccgggt ggcctataac gccgacatca cctacggcac
851 caataacgag tttgggttcg actacctgcg cgacaacatg gcgcactcac
901 tggatgatct ggtgcagcgc gggcaccatt acgccattgt cgacgaaggt
951 cgattccatc ctgatcgacg agggcggggc cccccccca tctccgcccg
```

*FIG. 7A*

```
1001 gggcgcccgc ctccaactgg ttcaccgagt tcgcccggtt ggcgtgccgc
1051 ggctggtttt ggacgtccac tacgaggtcg atctacgcaa acgcaccgtc
1101 ggcgtgcacg agaagggtgt ggaattcgtc gaagaccagc tcggcatcga
1151 caacctgtac gagaccgcca actcgccgtt ggtcagctat ctcaacaacg
1201 ctctgaaggc caaagagctg ttcagccgcg acaaggacta catcgtccgc
1251 gatggtgagg tgctcatcgt cgacgagttc accggccggg tgctgatcgg
1301 ccgccgctac aacgagggca tgcaccaggc catcgaggcc aaggagcacg
1351 tcgagatcaa ggccgagaac cagacgctgg ccaccatcac gctgcagaac
1401 tacttccggc tctaggagaa gctcgccggg atg
```

*FIG. 7B*

```
   1 tggcttgatt caaactagtg aacaataaat taagtttaaa gcacttgtgt
  51 ttttgcacaa gtttttttat actccaaaag caaattatga ctatttcata
 101 gttcgataat gtaatttgtt gaatgaaaca tagtgactat gctaatgtta
 151 atggatgtat atatttgaat gttaagttaa taatagtatg tcagtctatt
 201 gtatagtccg agtcgaaaat cgtaaaatat ttataatata atttattagg
 251 aagtataatt gcgtattgag aatatattta ttagtgataa acttgttgac
 301 aacagaatgt gaatgaagta tgtcataaat atatttatat tgattctaca
 351 aatgagtaaa taagtataat tttctaacta taaatgataa gatatattgt
 401 tgtaggccaa acagtttttt agctaaagga gcgaacgaaa tgggattttt
 451 atcaaaaatt cttgatggca ataataaaga aattaaacag ttaggtaaac
 501 ttgctgataa agtaatcgct ttagaagaaa aaacggcaat tttaactgat
 551 gaagaaattc gtaataaaac gaaacaattc caaacagaat tagctgacat
 601 tgataatgtc aaaaagcaaa atgattattt acataaaatt ttaccagaag
 651 catatgcact tgttagagaa ggctctaaac gtgtattcaa tatgacacca
 701 tataaagttc aaattatggg tggtattgca attcataaag gtgatatcgc
 751 tgagatgaga acaggtgaag gtaaaacatt aacagcgaca atgccaacat
 801 acttaaatgc attagctggt agaggtgttc acgttattac agtcaatgaa
 851 tacttatcaa gtgttcaaag tgaagaaatg gctgagttat ataacttctt
 901 aggtttgact gtcggattaa acttaaacag taagacgaca gaggaaaaac
 951 gtgaagcata cgcacaagac attacttaca gtactaataa tgagctaggt
1001 tttgattact tacgagataa catggtgaat tattctgaag atagggtaat
1051 gcgtccatta cattttgcaa tcattgatga ggtggactca attttaatcg
```

*FIG. 8A*

```
1101 acgaggcacg tacgccatta attatttctg gtgaagctga aaagtcaacg
1151 tcactttata cacaagcaaa tgtttttgcg aaaatgttaa aacaggacga
1201 tgattataaa tacgatgaaa aaacgaaagc tgtacattta acagaacaag
1251 gtgcggataa agctgaacgt atgttcaaag ttgaaaactt atatgatgta
1301 caaaatgttg atgttattag tcatatcaac acagctttac gtgcgcacgt
1351 tacattacaa cgtgacgtag actatatggt tgttgatggc gaagtattaa
1401 ttgtcgatca atttacagga cgtacaatgc caggccgtcg tttctcggaa
1451 ggtttacacc aagctattga agcgaaggaa ggcgttcaaa ttcaaaatga
1501 atctaaaact atggcgtcta ttacattcca aaactatttc agaatgtaca
1551 ataaacttgc gggtatgaca ggtacagcta aaactgaaga agaagaattt
1601 agaaatattt ataacatgac agtaactcaa attccgacaa ataaacctgt
1651 gcaacgtaac gataagtctg atttaattta cattagccaa aaaggtaaat
1701 ttgatgcagt agtagaagat gttgttgaaa aacacaaggc agggcaacca
1751 gtgctattag gtactgttgc agttgagact tctgaatata tttcaaattt
1801 acttaaaaaa cgtggtatcc gtcatgatgt gttaaatgcg aaaaatcatg
1851 aacgtgaagc tgaaattgtt gcaggcgctg gacaaaaagg tgccgttact
1901 attgccacta acatggctgg tcggggtaca gatatcaaat taggtgaagg
1951 cgtagaggaa ttaggcggtt tagcagtaat aggtacagag cgacatgaat
2001 ctcgtcgtat tgatgaccag ttacgtggtc gttctggacg tcaaggtgat
2051 aaaggggata gtcgcttcta tttatcatta caagatgaat taatgattcg
2101 ttttggttct gaacgtttac agaaaatgat gagccgacta ggtttagatg
2151 actctacacc aattgaatca aaaatggtat caagagctgt tgaatcagca
```

*FIG. 8B*

2201 caaaaacgtg tagaaggtaa taacttcgac gcgcgtaaac gtatcttaga
2251 atacgatgaa gtattacgta aacaacgtga aattatctat aacgaaagaa
2301 atagtattat tgatgaagaa gacagctctc aagttgtaga tgcaatgcta
2351 cgttcaacgt tacaacgtag tatcaattac tatattaata cagcagatga
2401 cgagcctgaa tatcaaccat tcatcgacta cattaatgac atcttcttac
2451 aagaaggtga cattacagag gatgatatca aaggtaaaga tgctgaagat
2501 attttcgaag tcgtttgggc taagattgaa gcagcatatc aaagtcaaaa
2551 agatatctta gaagaacaaa tgaatgagtt tgagcgtatg attttacttc
2601 gttctattga tagccattgg actgatcata tcgacacaat ggatcaatta
2651 cgtcaaggta ttcacttacg ttcttatgca caacaaaatc cattacgtga
2701 ctatcaaaat gaaggtcatg aattatttga tatcatgatg caaaatattg
2751 aagaagatac ttgtaaattc attttaaaat ctgtagtaca agttgaagat
2801 aatattgaac gtgaaaaaac aacagagttt ggtgaagcga agcacgtttc
2851 agctgaagat ggtaaagaaa aagtgaaacc gaaaccaatc gttaaaggcg
2901 atcaagttgg tcgtaacgat gattgtccat gtggtagtgg taaaaaattc
2951 aaaaattgcc atggaaaata aatgatataa aataactcct tccaattaaa
3001 cacctatagt ttgtgttatg ggaggagtct ttttatttta caagcgttaa
3051 atactttaaa aaatgtgaag aagttgttaa acgttgttat gtacttagtt
3101 ttaaaaaatc ggtttaggca tatg

FIG. 8C

```
   1 cttgaacgtt acttcactaa tgtgccgaat gtgaatgcac atgtaaaagt
  51 gaaaacttat gcaaattcta gcacaaaatc gaagttacaa ttccgcttaa
 101 tgacgtgaca cttcgtgcag aagaaagaaa cgatgattta tgctggaatt
 151 gacaagatca ctaacaaatt agaatgtcaa gttcgtaaat acaaaacacg
 201 tgtcaatcgt aagaaacgta aagaaagcga acatgaacca ttcccagcaa
 251 ctccggaaac tccgccggaa acagctgttg atcatgataa agatgatgaa
 301 attgaaatca tccgttctaa acaattcagc ttgaaaccaa tggattctga
 351 agaagcggta ttacaaatgg atttacttgg tactgatttc ttcatcttca
 401 atgaccgtga aactgatggt acaagcattg tttaccgccg taaagacgga
 451 aaatatggtt tgattgaaac tgttgaaaaa ctaatatgtg atatttgaaa
 501 gggctcttgc tgcattttct gctgcaagag tttctttttt tgagaaagcc
 551 cttattaaga tttgattaat aaaaatacaa ttgattgatt tacacggggt
 601 gtccatgtca aaataagagg gatgtattaa gttcataatt gtaatgtgag
 651 ctccgatgag tgagcggcat atgattatga tatccatgtg gcacatgatg
 701 ttaacaaaaa gagaatgaaa ctgtgagaag tacatcttga taaacacaac
 751 taggcagttt attaaaaaat aatgaacagt atcctatgag tttttaagta
 801 taaattaagc catataaatg gtaagataaa ttgttgtaag ccaaacagtt
 851 tttataccaa aggagcgaac agaatgggtt ttttaacaaa aattgttgac
 901 ggcaataaga gagaaatcaa acgcctaagt aagcaagctg acaaagtaat
 951 ctcattagaa gaagaaatgt caattcttac tgatgaagaa attagaaata
1001 aaacaaaagc attccaagaa agattgcaag cagaagaaca tgtaagcaaa
1051 caagataaaa ttttagaaga aatattacct gaagcatttg cgcttgtccg
1101 tgaaggagct aaacgtgtat ttaatatgac accttatcca gttcaaatca
1151 tgggtggtat cgccattcat aatggtgaca tttcagaaat gagaacaggt
```

*FIG. 9A*

```
1201 gaaggtaaaa cattaactgc aacgatgccg acttatttaa acgccttagc
1251 agcacgtggt gtgcatgtta ttacagtcaa tgaatacttg gcaagttctc
1301 aaagagaaga aatggccgag ttatataatt tccttggttt atcagtcgga
1351 ttgaacttga acagcttatc aacagaacaa aagcgtgaag cttataatgc
1401 agatattacg tatagtacaa ataatgaatt aggcttcgac tatttacgcg
1451 ataacatggt gaattattca gaagaacgtg ttatgcgtcc gcttcatttc
1501 gctatcattg atgaggtcga ctctatttta atcgatgaag cgcgtacacc
1551 attgattatt tcaggggaag ctgaaaaatc aacatctctt tatacacaag
1601 caaatgtttt cgctaaaatg ttaaaagcag aagatgatta taattatgat
1651 gaaaaaacaa aatcagtaca attaacagat caaggtgctg ataaagctga
1701 acgtatgttc aagttagata acttatatga tttgaaaaac gttgatatta
1751 tcacgcatat caatacagca ttacgtgcta actatacatt gcaacgcgat
1801 gtagattaca tggttgtaga tggagaagta ttgattgtcg accaatttac
1851 aggtcgaaca atgccaggtc gtcgattctc tgaaggactt caccaagcga
1901 ttgaggctaa agaaggggtt caaattcaaa atgaatctaa aacaatggct
1951 tctatcacat tccaaaacta cttccgtatg tataataaat tagccggtat
2001 gacaggtact gctaaaacag aggaagaaga attccgtaac atttataata
2051 tgacagttac acaaattcca acgaaccgtc ctgttcaacg tgaagataga
2101 cctgacttga ttttcatcag ccaaaaaggc aagttcgatg ctgttgttga
2151 agatgttgtt gaaaaacata aaaaaggcca accaattctt ttaggtactg
2201 tagcggttga aacaagtgaa tacatttcac aactattgaa aaaacgcggt
2251 gtgcgtcatg atgtcttaaa cgctaaaaac catgaacgcg aagctgaaat
2301 cgtatctaca gcaggtcaaa aaggtgcagt cacaatcgca acaaacatgg
2351 ctggtcgtgg taccgatatt aaattaggcg aaggtgttga agaattaggc
2401 ggccttgctg ttattggtac agaacgtcat gaatcacgcc gtatcgatga
```

*FIG. 9B*

```
2451 tcagttgcgt ggtcgttctg gacgacaagg tgaccgcgga gaaagccgtt
2501 tctatttatc attacaagat gagttgatgg tacgtttcgg ttctgaacgt
2551 ctgcaaaaaa tgatgggccg attaggtatg gatgactcta caccgattga
2601 atcaaaaatg gtatctcgag ctgttgaatc tgcacaaaaa cgtgttgaag
2651 gtaacaactt cgatgcacgt aaacgtatct tagaatacga tgaagtttta
2701 cgtaaacaac gtgaaatcat ttatggtgaa cgtaataata ttatcgattc
2751 agaatcaagt tctgaattag tcattacaat gatacgctct acattagatc
2801 gtgcaatcag ttattatgta aatgaagaat tggaagaaat tgactatgcg
2851 ccgtttatta attttgtgga agatgttttc ttdcacgaag gtgaagtcaa
2901 agaagatgaa atcaaaggta aaggtaaaga tcgtgaggat attttcgata
2951 cagtatgggc taaaattgaa aaagcttatg aagcacaaaa agccaatata
3001 cccgaccaat tcaatgaatt cgaacgtatg attttattac gttctattga
3051 tggaagatgg acagaccata tcgatacaat ggatcaatta cgtcaaggta
3101 tccatttacg ttcatacggt caacaaaacc cacttcgcga ctatcaaaat
3151 gaagggcacc aactatttga tacaatgatg gtcaatattg aagaagacgt
3201 cagcaaatat atcttgaaat caattatcac agtagatgat gatattgaac
3251 gtgataaagc aaaagaatat caaggacaac atgtatcagc tgaagatgga
3301 aaagaaaaag taaaaccgca accagttgtt aaagataatc acatcggaag
3351 aaatgatcct tgtccatgcg gcagcggtaa aaagtataaa aattgctgcg
3401 gtaaatagta agttgtatta ggaccactgt taaatagctt taagagagat
3451 gctcaattga aattgggtta tctttctaag ggctgtcagc ggtctttttt
3501 caatccaaca aaaatatgga tatatgctaa aataatagag taatctggaa
3551 aattaaactg gaattggaga gatatgaaaa tggaattat
```

*FIG. 9C*

```
   1 cagtcaatgt cgctcttcgt gaccgagcca atggacggaa aggtgcccgc ctcccagatc
  61 atgaacctcc tagtgtacgc ctataagaag ggccttaaga cggggctcta ctactgcaag
 121 atccgcaagg ccaccaacaa cggcgtcttc acgggcggcg acctcgtgtg ctctgggtgc
 181 cacctgtagc gacgcgcgcc gagcgcgatg gccgaggcgg cggacgcggc gaccctcacg
 241 cgtaaataca aatactttta cgagaccgag tgccccgacc tagatcactt gcggtcgctc
 301 agcgtcgcaa accgctggct ggagaccgag tttcccctag cggacgacgc caaggacgtg
 361 gcgcggctca gcggcgccga gctggagttt taccgctttc tgttcgcgtt cctctcggcc
 421 gccgatgacc tcgtgaacgt caacctcggg gacctgtccg agctgttcac ccaaaaagac
 481 atcctgcatt actatatcga gcaggagtcc atcgaagtgg tgcactcgcg ggtgtacagc
 541 gccatacagc tgctgctctt tagaaacgac gcggtggcgc gcgcgggcta cgtagagggc
 601 gccctcggcg acccggcggt ccggcgcaag gtggactggc tcgagcggcg cgtggccgcg
 661 gcagagtcgg tggccgaaaa gtacgtgctc atgattctaa tcgagggcat tttttctcc
 721 tcctcgtttg cggcgattgc ctacctgcgc acccacaacc ttttcgtcgt gacgtgccaa
 781 accaacgacc tcatcagccg cgacgaagcc gtgcacacgg ccgcgtcgtg ctgcatcttc
 841 gacaactacc tcggcgggga gcggccgccg ccggcccgca tctacgagct gttccgcgaa
 901 gcgtggaaat tgagcgcgag tttatttggt tgcgcgccgc gcggcagtca tatacttgac
 961 gtggaggcta tttctgcgta cgtcgagtac agcgcggacc gcctgctcgc tgctatccag
1021 ctgcctcctc tgtttggcac cccgcctcct gggaccgatt ttcctttggc cctgatgact
1081 gccgagaagc acacgaactt ctttgagcgc cgcagcacca actacacagg caccgtaatc
1141 aacgacctgt agggcacccc cgctgccctg ccagagcgcc ccgcctttcc tcctccttct
1201 cacccccacg ccgcgaataa aaatgttcc atgtcaacga aa
```

*FIG. 10*

```
   1 tcgagcccgc cgaaacccgc cgcgtctgtt gaaatggcca gccgcccagc cgcatcctct
  61 cccgtcgaag cgcgggcccc ggttggggga caggaggccg gcggccccag cgcagccacc
 121 caggggggagg ccgccggggc ccctctcgcc cacggccacc acgtgtactg ccagcgagtc
 181 aatggcgtga tggtgctttc cgacaagacg cccgggtccg cgtcctaccg catcagcgat
 241 agcaactttg tccaatgtgg ttccaactgc accatgatca tcgacggaga cgtggtgcgc
 301 gggcgccccc aggacccggg ggccgcggca tcccccgctc ccttcgttgc ggtgacaaac
 361 atcggagccg gcagcgacgg cgggaccgcc gtcgtggcat tcgggggaac cccacgtcgc
 421 tcggcgggga cgtctaccgg tacccagacg gccgacgtcc ccaccgaggc ccttgggggc
 481 ccccctcctc ctccccgctt caccctgggt ggcggctgtt gttcctgtcg cgacacacgg
 541 cgccgctctg cggtattcgg gggggagggg gatccagtcg gccccgcgga gttcgtctcg
 601 gacgaccggt cgtccgattc cgactcggat gactcggagg acacggactc ggagacgctg
 661 tcacacgcct cctcggacgt gtccggcggg gccacgtacg acgacgccct tgactccgat
 721 tcgtcatcgg atgactccct gcagatagat ggccccgtgt gtcgcccgtg gagcaatgac
 781 accgcgcccc tggatgtttg ccccgggacc cccggcccgg gcgccgacgc cggtggtccc
 841 tcagcggtag acccacacgc gccgacgcca gaggccggcg ctggtcttgc ggccgatccc
 901 gccgtggccc gggaagacgc ggaggggctt tcggaccccc ggccacgtct gggaacgggc
 961 acggcctacc ccgtccccct ggaactcacg cccgagaacg cggaggccgt ggcgcgcttt
1021 ctgggagatg ccgtgaaccg cgaacccgcg ctcatgctgg agtacttttg ccggtgcgcc
1081 cgcgaggaaa ccaagcgtgt cccccccagg acattcggca gcccccctcg cctcacggag
1141 gacgactttg ggcttctcaa ctacgcgctc gtggagatgc agcgcctgtg tctggacgtt
1201 cctccggtcc cgccgaacgc atacatgccc tattatctca gggagtatgt gacgcggctg
1261 gtcaacgggt tcaagccgct ggtgagccgg tccgctcgcc tttaccgcat cctggggggtt
1321 ctggtgcacc tgcggatccg gacccgggag gcctcctttg aggagtggct gcgatccaag
```

*FIG. 11A*

```
1381 gaagtggccc tggattttgg cctgacggaa aggcttcgcg agcacgaagc ccagctggtg
1441 atcctggccc aggctctgga ccattacgac tgtctgatcc acagcacacc gcacacgctg
1501 gtcgagcggg ggctgcaatc ggccctgaag tatgaggagt tttacctaaa gcgttttggc
1561 gggcactaca tggagtccgt cttccagatg tacacccgca tcgccggctt tttggcctgc
1621 cgggccacgc gcggcatgcg ccacatcgcc ctggggcgag aggggtcgtg gtgggaaatg
1681 ttcaagttct ttttccaccg cctctacgac caccagatcg taccgtcgac ccccgccatg
1741 ctgaacctgg ggacccgcaa ctactacacc tccagctgct acctggtaaa cccccaggcc
1801 accacaaaca aggcgaccct gcgggccatc accagcaacg tcagtgccat cctcgcccgc
1861 aacgggggca tcgggctatg cgtgcaggcg tttaacgact ccggccccgg gaccgccagc
1921 gtcatgcccg ccctcaaggt ccttgactcg ctggtggcgg cgcacaacaa agagagcgcg
1981 cgtccgaccg gcgcgtgcgt gtacctggag ccgtggcaca ccgacgtgcg ggccgtgctc
2041 cggatgaagg gggtcctcgc cggcgaagag gcccagcgct gcgacaatat cttcagcgcc
2101 ctctggatgc cagacctgtt tttcaagcgc ctgattcgcc acctggacgg cgagaagaac
2161 gtcacatgga ccctgttcga ccgggacacc agcatgtcgc tcgccgactt tcacggggag
2221 gagttcgaga agctctacca gcacctcgag gtcatggggt tcggcgagca gatacccatc
2281 caggagctgg cctatggcat tgtgcgcagt gcggccacga ccgggagccc cttcgtcatg
2341 ttcaaagacg cggtgaaccg ccactacatc tacgacaccc agggggcggc catcgccggc
2401 tccaacctct gcaccgagat cgtccatccg gcctccaagc gatccagtgg ggtctgcaac
2461 ctgggaagcg tgaatctggc ccgatgcgtc tccaggcaga cgtttgactt tgggcggctc
2521 cgcgacgccg tgcaggcgtg cgtgctgatg gtgaacatca tgatcgacag cacgctacaa
2581 cccacgcccc agtgcacccg cggcaacgac aacctgcggt ccatgggaat cggcatgcag
2641 ggcctgcaca cggcctgcct gaagctgggg ctggatctgg agtctgccga atttcaggac
2701 ctgaacaaac acatcgccga ggtgatgctg ctgtcggcga tgaagaccag caacgcgctg
```

*FIG. 11B*

```
2761 tgcgttcgcg gggcccgtcc cttcaaccac tttaagcgca gcatgtatcg cgccggccgc
2821 tttcactggg agcgctttcc ggacgcccgg ccgcggtacg agggcgagtg ggagatgcta
2881 cgccagagca tgatgaaaca cggcctgcgc aacagccagt ttgtcgcgct gatgcccacc
2941 gccgcctcgg cgcagatctc ggacgtcagc gagggctttg ccccccctgtt caccaacctg
3001 ttcagcaagg tgacccggga cggcgagacg ctgcgcccca acacgctcct gctaaaggaa
3061 ctggaacgca cgtttagcgg gaagcgcctc ctggaggtga tggacagtct cgacgccaag
3121 cagtggtccg tgccgcaggc gctcccgtgc ctggagccca cccacccccct ccggcgattc
3181 aagaccgcgt ttgactacga ccagaagttg ctgatcgacc tgtgtgcgga ccgcgccccc
3241 tacgtcgacc atagccaatc catgaccctg tatgtcacgg agaaggcgga cgggaccctc
3301 ccagcctcca ccctggtccg ccttctggtc cacgcatata agcgcggact aaaaacaggg
3361 atgtactact gcaaggttcg caaggcgacc aacagcgggg tctttggcgg cgacgacaac
3421 attgtctgca tgagctgcgc gctgtgaccg acaaaccccc tccgcgccag gcccgccgcc
3481 actgtcgtcg ccgtcccaag ctctcccctg ctgccatg
```

*FIG. 11C*

```
   1 gtgtgtttgg cgtgtgtctc tgaaatggcg gaaacccaca tgcaaatggg attcatggac
  61 acgttacacc cccctgactc aggagatagg catatcctcc ttagattgac tcagcacacg
 121 atcgcacccc acccctgtgt gccggggata aaagccaacg cgcgcggtct gggttaccac
 181 aacaggtggg tgcttcgggg acttgacggt cgccactctc ctgcgagccc tcacgtcttc
 241 gcccaccgat tcctgttgcg ttcctgtcgg ccggtgctgt cctgtcgaca gattgttggc
 301 gactgcccgg gtgattcgtc ggccggtgcg tcctttcggt cgtaccgccc accccgcctc
 361 ccacgggccc gccgctgttt ccgttcatcg cgtccgagcc accgtcacct tggttccaat
 421 ggccaaccgc cctgccgcat ccgccctcgc cggagcgcgg tctccgtccg aacgacagga
 481 accccgggag cccgaggtcg ccccccctgg cggcgaccac gtgttttgca ggaaagtcag
 541 cggcgtgatg gtgctttcca gcgatccccc cggccccgcg gcctaccgca ttagcgacag
 601 cagctttgtt caatgcggct ccaactgcag tatgataatc gacggagacg tggcgcgcgg
 661 tcatttgcgt gacctcgagg gcgctacgtc caccggcgcc ttcgtcgcga tctcaaacgt
 721 cgcagccggc ggggatggcc gaaccgccgt cgtggcgctc ggcggaacct cgggcccgtc
 781 cgcgactaca tccgtgggga cccagacgtc cggggagttc ctccacggga acccaaggac
 841 ccccgaaccc caaggacccc aggctgtccc cccgccccct cctcccccct ttccatgggg
 901 ccacgagtgc tgcgcccgtc gcgatgccag gggcggcgcc gagaaggacg tcggggccgc
 961 ggagtcatgg tcagacggcc cgtcgtccga ctccgaaacg gaggactcgg actcctcgga
1021 cgaggatacg ggctcgggtt cggagacgct gtctcgatcc tcttcgatct gggccgcagg
1081 ggcgactgac gacgatgaca gcgactccga ctcgcggtcg gacgactccg tgcagcccga
1141 cgttgtcgtt cgtcgcagat ggagcgacgg ccctgccccc gtggcctttc ccaagccccg
1201 gcgccccggc gactcccccg gaaaccccgg cctgggcgcc ggcaccgggc cgggctccgc
1261 gacggacccg cgcgcgtcgg ccgactccga ttccgcggcc cacgccgccg caccccaggc
1321 ggacgtggcg ccggttctgg acagccagcc cactgtggga acggaccccg gctacccagt
```

*FIG. 12A*

```
1381 cccccctagaa ctcacgcccg agaacgcgga ggcggtggcg cggtttctgg gggacgccgt
1441 cgaccgcgag cccgcgctca tgctggagta cttctgtcgg tgcgcccgcg aggagagcaa
1501 gcgcgtgccc ccacgaacct tcggcagcgc cccccgcctc acggaggacg actttgggct
1561 cctgaactac gcgctcgctg agatgcgacg cctgtgcctg gaccttcccc cggtcccccc
1621 caacgcatac acgccctatc atctgaggga gtatgcgacg cggctggtta acgggttcaa
1681 acccctggtg cggcggtccg cccgcctgta tcgcatcctg gggattctgg ttcacctgcg
1741 catccgtacc cgggaggcct cctttgagga atggatgcgc tccaaggagg tggacctgga
1801 cttcgggctg acggaaaggc ttcgcgaaca cgaggcccag ctaatgatcc tggcccaggc
1861 cctgaacccc tacgactgtc tgatccacag caccccgaac acgctcgtcg agcgggggct
1921 gcagtcggcg ctgaagtacg aagagtttta cctcaagcgc ttcggcgggc actacatgga
1981 gtccgtcttc cagatgtaca cccgcatcgc cgggttcctg gcgtgccggg cgacccgcgg
2041 catgcgccac atcgccctgg ggcgacaggg gtcgtggtgg gaaatgttca agttcttttt
2101 ccaccgcctc tacgaccacc agatcgtgcc gtccaccccc gccatgctga acctcggaac
2161 ccgcaactac tacacgtcca gctgatacct ggtaaacccc caggccacca ctaaccaggc
2221 caccctccgg gccatcaccg gcaacgtgag cgccatcctc gcccgcaacg gggcatcgg
2281 gctgtgcatg caggcgttca acgacgccag ccccggcacc gccagcatca tgccggccct
2341 gaaggtcctg gactccctgg tggcggcgca caacaaacag agcacgcgcc ccaccggggc
2401 gtgcgtgtac ctggaaccct ggcacagcga cgttcgggcc gtgctcagaa tgaagggcgt
2461 cctcgccggc gaggaggccc agcgctgcga caacatcttc agcgccctct ggatgccgga
2521 cctgttcttc aagcgcctga tccgccacct cgacggcgag aaaaacgtca cctggtccct
2581 gttcgaccgg gacaccagca tgtcgctcgc cgactttcac ggcgaggagt tcgagaagct
2641 gtacgagcac ctcgaggcca tggggttcgg cgaaacgatc cccatccagg acctggcgta
2701 cgccatcgtg cgcagcgcgg ccaccaccgg aagccccttc atcatgttta aggacgcggt
```

*FIG. 12B*

```
2761 aaacagccac tacatctacg acacgcaagg ggcggccatt gccggctcca acctctgcac
2821 ggagatcgtc cacccgtcct ccaaacgctc cagcggggtc tgcaacctgg gcagcgtgaa
2881 tctggcccga tgcgtctccc ggcggacgtt cgattttggc atgctccgcg acgccgtgca
2941 ggcgtgcgtg ctaatggtta atatcatgat agacagcacg ctgcagccga cgccccagtg
3001 cgcccgcggc cacgacaacc tgcggtccat gggcattggc atgcagggcc tgcacacggc
3061 gtgcctgaag atgggcctgg atctggagtc ggccgagttc cgggacctga acacacacat
3121 cgccgaggtg atgctgctcg cggccatgaa gaccagtaac gcgctgtgcg ttcgcggggc
3181 gcgtcccttc agccacttta agcgcagcat gtaccgggcc ggccgctttc actgggagcg
3241 cttttcgaac gccagcccgc ggtacgaggg cgagtgggag atgctacgcc agagcatgat
3301 gaaacacggc ctgcgcaaca gccagttcat cgcgctcatg cccaccgccg cctcggccca
3361 gatctcggac gtcagccagg gctttgcccc cctgttcacc aacctgttca gcaaggtgac
3421 cagggacggc gagacgctgc gccccaacac gctcttgctg aaggaactcg agcgcacgtt
3481 cggcgggaag cggctcctgg acgcgatgga cgggctcgag gccaagcagt ggtctgtggc
3541 ccaggccctg ccttgcctgg accccgccca cccctccgg cggttcaaga cggccttcga
3601 ctacgaccag gaactgctga tcgacctgtg tgcagaccgc gccccctatg ttgatcacag
3661 ccaatccatg actctgtatg tcacagagaa ggcggacggg acgctccccg cctccaccct
3721 ggtccgcctt ctcgtccacg catataagcg cggcctgaag acggggatgt actactgcaa
3781 ggttcgcaag gcgaccaaca gcggggtgtt cgccggcgac gacaacatcg tctgcacaag
3841 ctgcgcgctg taagcaacag cgctccgatc ggggtcaggc gtcgctctcg gtcccgcata
3901 tcgccatgga tcccgccgtc tcccccgcga gcaccgaccc cctagatacc cacgcgtcgg
3961 gggccggggc ggccccgatt ccggtgtgcc ccaccccgga gcggtacttc tacacctccc
4021 agtgccccga catcaaccac cttcgctccc tcagcatcct gaaccgctgg ctggagaccg
4081 agctcgtgtt cgtcggggac gaggaggacg tctccaagct ctccgagggc gagctcggct
```

*FIG. 12C*

```
4141 tctaccgctt tctgtttgcc ttcctgtcgg ccgcggacga cctggtgacg gaaaacctgg
4201 gcggcctctc cggcctcttc gaacagaagg acattcttca ctactacgtg gagcaggaat
4261 gcatcgaggt cgtccactcc cgcgtctaca acatcatcca gctggtgctc tttcacaaca
4321 acgaccaggc gcgccgcgcc tatgtggccc gcaccatcaa ccacccggcc attcgcgtca
4381 aggtggactg gctggaggcg cgggtgcggg aatgcgactc gatcccggag aagttcatcc
4441 tcatgatcct catcgagggc gtcttttttg ccgcctcgtt cgccgccatc gcgtacctgc
4501 gcaccaacaa cctcctgcgg gtcacctgcc agtcgaacga cctcatcagc cgccacgagg
4561 ccgtgcatac gacagcctcg tgctacatct acaacaacta cctcgggggc cacgccaagc
4621 ccgaggcggc gcgcgtgtac cggctgtttc gggaggcggt ggatatcgag atcgggttca
4681 tccgatccca ggccccgacg gacagctcta tcctgagtcc gggggccctg gcggccatcg
4741 agaactacgt gcgattcagc gcggatcgcc tgctgggcct gatccatatg cagcccctgt
4801 attccgcccc cgcccccgac gccagctttc ccctcagcct catgtccacc gacaaacaca
4861 ccaacttctt cgagtgccgc agcacctcgt acgccggggc cgtcgtcaac gatctgtgag
4921 ggtctgggcg cccttgtagc gatgtctaac cgaaataaag ggtcgaaacg ggactgttgg
4981 gtctccggtg tgattattac gcaggggagg ggggtggcgg ctggggaaag ggaaggaacg
5041 cccgaaacca gagaaaggga ccaaaaggga aacgcgtcca accgataaat caagcgccga
5101 ccagaacccc gagatgcata ataacaaacg attttattac tcttattatt aacaggtcgg
5161 gcatcgggag gggatggggg cgcgcgtttc ctccgttccg gctactcgtc ccagaattta
5221 gccaggacgt ccttgtaaaa cgcgggcggg ggcgcgtggg cccacacctg cgccagaaac
5281 cggtcggcga tgtccggggc ggtgatatga cgagtcacga tggagcgcgc taaatcttcg
5341 tcgcggaggt cctgatagat gggcagtctt tttagaagag tccagggtcc ccgctccttg
5401 gggctgataa gcgatatgac gtacttgacg tatctgtgct ccaccagctc ggcgatggtc
5461 atcggatcgg gcagccagtc cagggcctcc ggggcgtcgt ggatgacgtg gcggcgacgt
```

FIG. 12D

```
5521 ccggcgacat agccgcggtg ttccgcgacc cgctgcgcgt tggggacctg cacgagctcg
5581 ggcggggtga gtatctccga ggaggacgac cgggcgccgt cgcgcggccc accggcgacg
5641 tccgggggct ggaggggggg gtcttcttcg tagtcgtcct cgcccgcgat ctgttgggcc
5701 agaatttcgg tccacgagat gcgcgtctcg aggccgaccg gggccgcggt cagcgtaggc
5761 atgctctcca gggagcgcga gttggcgcgc tcccgccggg ccgcccggcg ggcctgggat
5821 cggctcgggg cggtccagtg acactcgcgc agcacgtcct cgacggacgc gtaggtgtta
5881 ttggggtgca ggtctgtgtg gcagcggacg aacagcgcca ggaactgcgg gtaactcatc
5941 ttgaagtacc ctgcag
```

*FIG. 12E*

```
   1 aaaccactgt tctttacact ttatgctcta gtttttggta atagtgtctt ggaacacttt
  61 taccctaaac gaaattatgg ctttggattt tttgagcacc gactgtccac tggggattgt
 121 ttccgatatt atatccaacg tgaataccat caaagagtat ggatattcca gcgaattatc
 181 aacaacgctg gcacctcgcc cgtctcgaga acaggtgtta gagtatatca ccagagtcgt
 241 ggataaactc aagccgctgt gcagagtcga cgaacgcctt tacattgcgt gcggggagct
 301 tgtacaccta cgaattaaag cacgcaacac agacctgaaa tattggctaa aatcgtctga
 361 gattgatctt agcgatgtcg tggaacaggc catattggaa cacattgact ttgttcagaa
 421 aaccctcaac tcgtttgaaa catcggaata ccgagatttg tgttcattag gcctgcaatc
 481 tgcgctaaag tatgaagaaa tgtatttagc caaaatgcga ggcggacgtc tagagtccat
 541 ggggcaattt tttcttagac ttgcaactac tgctacgcac tatactatgg aacaaccagc
 601 aatggctcgc gtgttggtta gcggtgaggt tggctggaca tatattttca gagccttttt
 661 tactgcgcta gccggacagg ttgtcattcc ggccacgcca attatgctgt ttggtgggag
 721 agactgtggg tctatggcca gctgttattt gctaaacccc agggtaacag atatgaactc
 781 tgcaattccg gctcttatgg aagaggttgg acccattttg tgcaaccgag gaggaattgg
 841 actgtcttta cagaggttta acactccacc cacagaaggt tgttcacggg gtgtcatggc
 901 tctcctaaag ctactagact ctatgaccat ggccattaac agcgacggtg aaagaccaac
 961 aggagtgtgt gtttatttcg aaccctggca cgcagacatc cgcgccattt taaatatgcg
1021 cggaatgctg gccagagacg aaactgtgcg ctgcgacaac atctttgctt gtatgtggac
1081 cccagacctg ttttttgacc gctatcaacg gtacgtcgat ggagaaagcg gcataatgtg
1141 gactctgttt gatgatactg catcgcacct ctgccatatg tacggaaatg atttcacacg
1201 ggaatatgag cgcctggagc ggtgtggatt tgggatagac gctattccca tacaggacat
1261 ggcctttatc atagttagaa gtgctgtaat gacaggaagc ccatttttga tgtttaaaga
1321 cgcgtgcaac aggcactacc actttgacat gcggcagaga ggtgcgataa tggggtctaa
```

*FIG. 13A*

```
1381 tctatgcaca gaaattatcc agcatgccga cgaaacccaa aacggggtgt gtaatctagc
1441 cagcatcaac ctcccaaaat gtctagccct tccacctcca aatattgcag gtgtgccata
1501 ttttgacttc gccgctctgg gccgcgctgc cgcaactgcc acaatttttg tcaatgcgat
1561 gatgtgtgcc agcacatatc caactgttaa atcccagaag ggcgttgaag aaaaccggtc
1621 gctgggactt ggaattcagg ggctacatac cacgtttttg atgctggacc tggatatggc
1681 atctccagag gcgcaccaac taaacaagca aatagcagaa aggctgttat tgaactctat
1741 gaaggccagc gcaacgctct gcaagctggg tatgcaaccc tttaaagggt ttgaagacag
1801 caagtacagt cggggggaac taccctttga tgcctaccca aatgtaacac taacaaaccg
1861 caacgcctgg cgtagacttc gcactgacat aaaacaatac ggcttgtaca attctcagtt
1921 tgtagcctat atgccaacag tatcttcgtc acaggttacc gagagcagcg aggggttttc
1981 tcctgtttac acaaacctgt ttagcaaagt tactgctacc ggggaagtac tcaggcccaa
2041 tgtactgcta atgcgcacca tcagaagtat ttttccacag gaatgcgcgc gcttacaagc
2101 gctatctacg ctagaagctg cgaaatggtc agttgtggga gcgtttggtg atttgccagt
2161 tggtcacccc ctcagtaagt ttaaaacagc atttgagtac gaccagacta tgctaattaa
2221 catgtgtgct gacagggctg cgtttgtgga ccagagccaa tccatgtctt tgtttataac
2281 tgagcctgct gacggaaaac tccccgcctc cagaattatg aatcttttgg tccacgcata
2341 taaacgcgga cttaaaacag gcatgtacta ctgcaaaatc aagaaggcaa caaacaacgg
2401 agtctttgtt ggcggagacc tagtctgcac cagctgcagc ttgtagggca gcctcgccat
2461 tttgcccagg gcgggaaaat aattatggcc ctcgaaaact ctaaaaaaac agattttgct
2521 gacgagttat tgataaatgc gtatttctat acgccggaat gtcccgatat tgaacaccta
2581 cgcttgttga gcgttgccaa ccgctggctg gatacggacc ttccaatttc tgatgacctc
2641 aaggacgttg ctaaactcgc gccagccgag cgagagtttt accggttttt gtttgccttt
```

*FIG. 13B*

```
2701 ttatctgctg ctgacgactt ggtaaattta aacctgggag atttatccgc actatttact
2761 caaaaggaca ttcttcacta ctacattgag caagagtcta ttgaagtaac gcactccaga
2821 gtatatagcg ctatacagct tatgttgttt ggaaacgacg caacagcgcg cgctaggtat
2881 gtcgcatctg ttgtcaaaga cgtggccata gacctaaagg tatcttggtt gcaagcaaag
2941 gtgcgagaat gcaaatctgt ggcggaaaag tatattttga tgatattaat agagggcgtt
3001 ttcttcgcgt cgtcctttcc gtccatcgca tatcttcgca cccacaatct ctttgtggta
3061 acctgtcaaa gtaatgattt aattagccgc gacgaagcaa ttcacaccaa cgcctcgtgc
3121 tgtatctaca acaactacct tgggcgtttt gaaaagccag ctccaacgag gatttatgcg
3181 ctgttttctg aggccgtaaa catcgagtgt gaatttttgc tttcccatgc ccccaaaagc
3241 agccacctgt tggacattga agccatcata tgct-acgtac gctatagcgc ggacaggctt
3301 ttgggggaaa ttggactatc tccgctgttt aatgctccca aacccccacc aagcttcccc
3361 ctagctttca tgactgtgga aaaacatacc aactttttg aaaggcgaag caccgcatac
3421 tcgggaactc ttataaacga tctgtaatgt aaaaataaaa actaatttg attcacttat
3481 ttgtcttgtt tgcgtgttgg atgtacgcga tttaaaaaa tactgagaaa agatactccc
3541 gatttaactt tatttaagac cattgtcttc ggtgtccaca gtcatcccag tagttaacca
3601 acacagtgtt gtaatcagtg ggggtgggaa tgtggttcca aaacatatta gcaagctctc
3661 tgacaatttc gtgttcgg
```

*FIG. 13C*

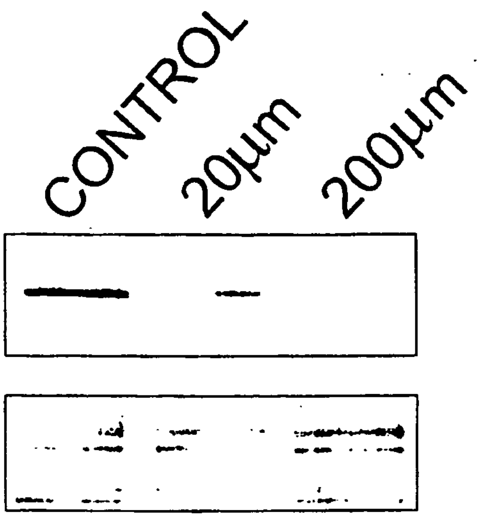


*FIG. 14*

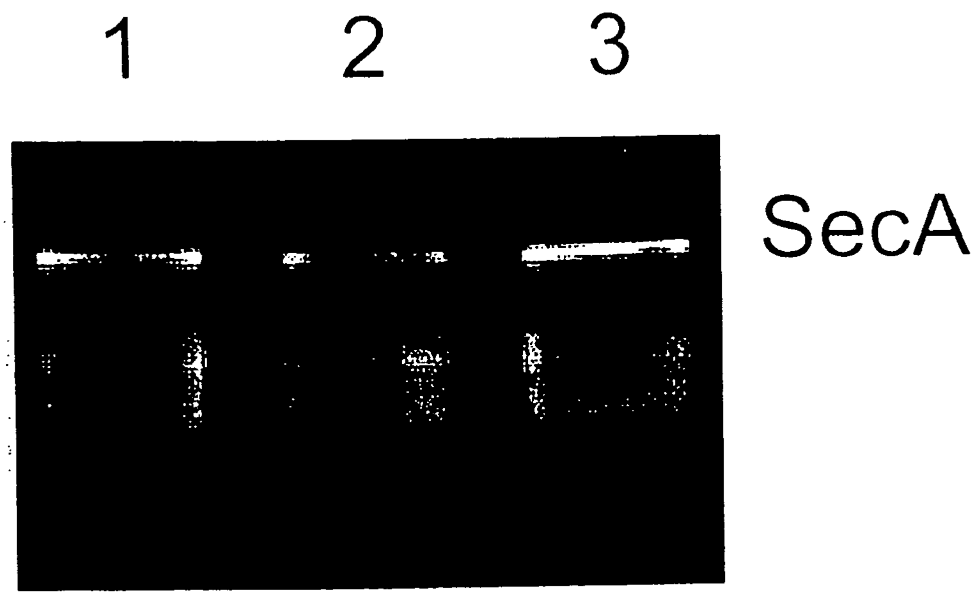


*FIG. 17*

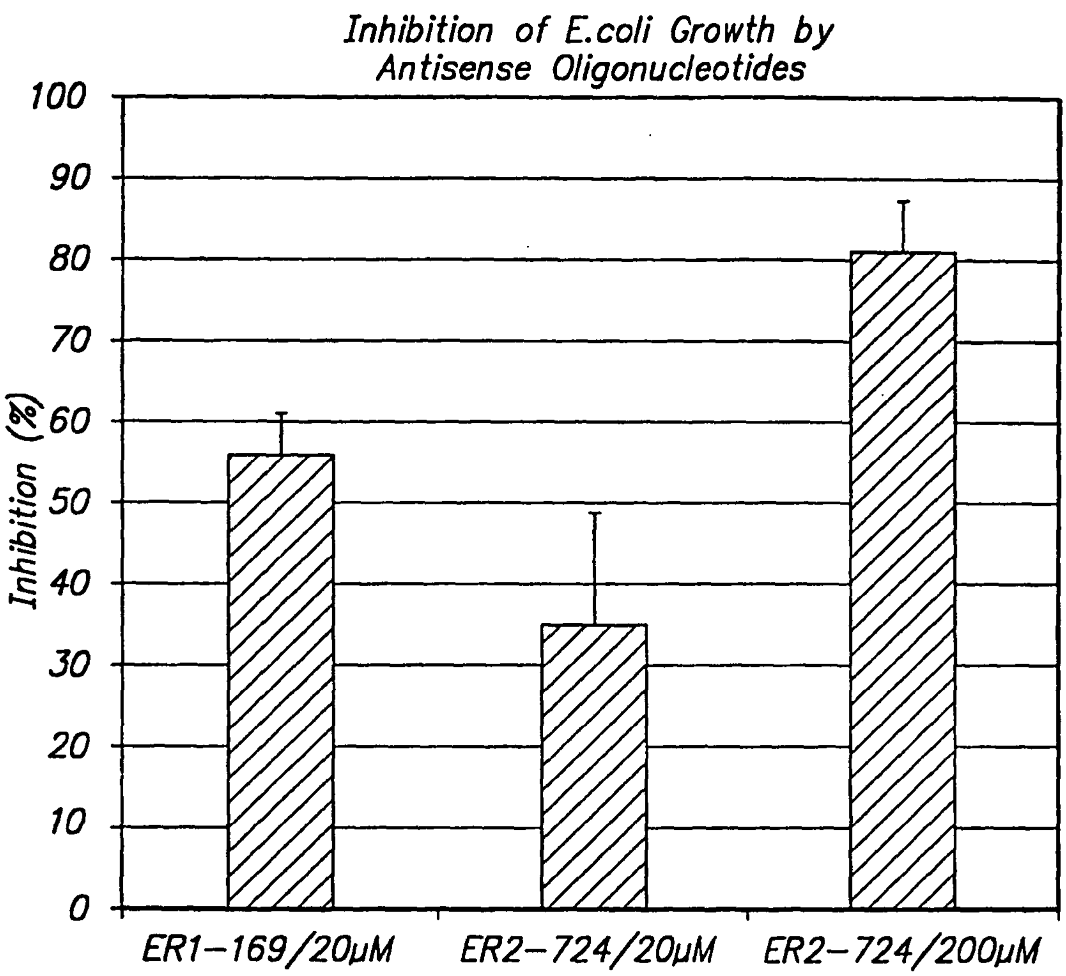
Inhibition of E.coli Growth by
Antisense Oligonucleotides
100
90
80
70
60
50
40
30
20
10
0
Inhibition (%)
ER1–169/20μM
ER2–724/20μM
ER2–724/200μM

*FIG. 15*

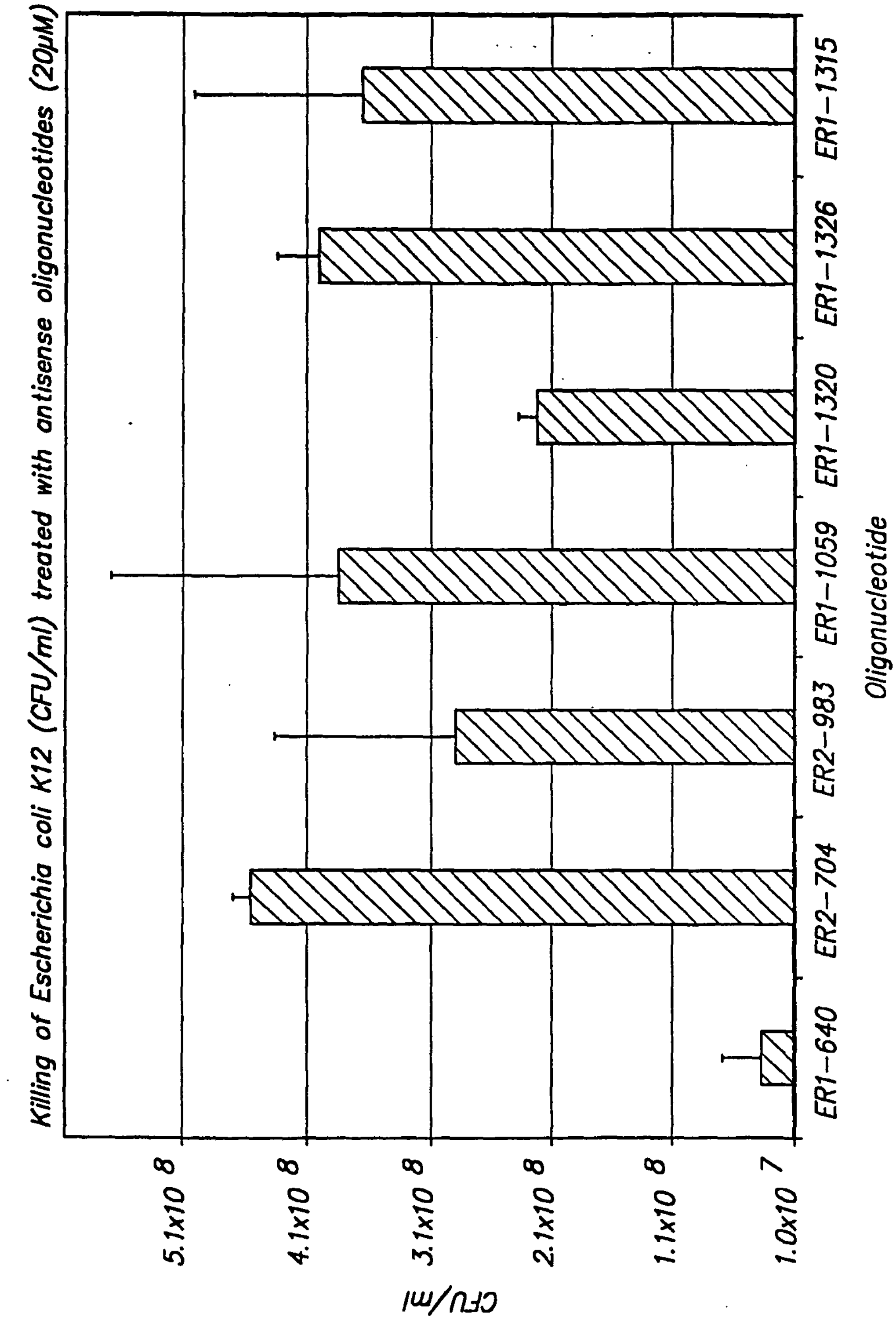
Killing of Escherichia coli K12 (CFU/ml) treated with antisense oligonucleotides (20μM)
CFU/ml
5.1x10 8
4.1x10 8
3.1x10 8
2.1x10 8
1.1x10 8
1.0x10 7
ER1-640
ER2-704
ER2-983
ER1-1059
ER1-1320
ER1-1326
ER1-1315
Oligonucleotide

FIG. 16

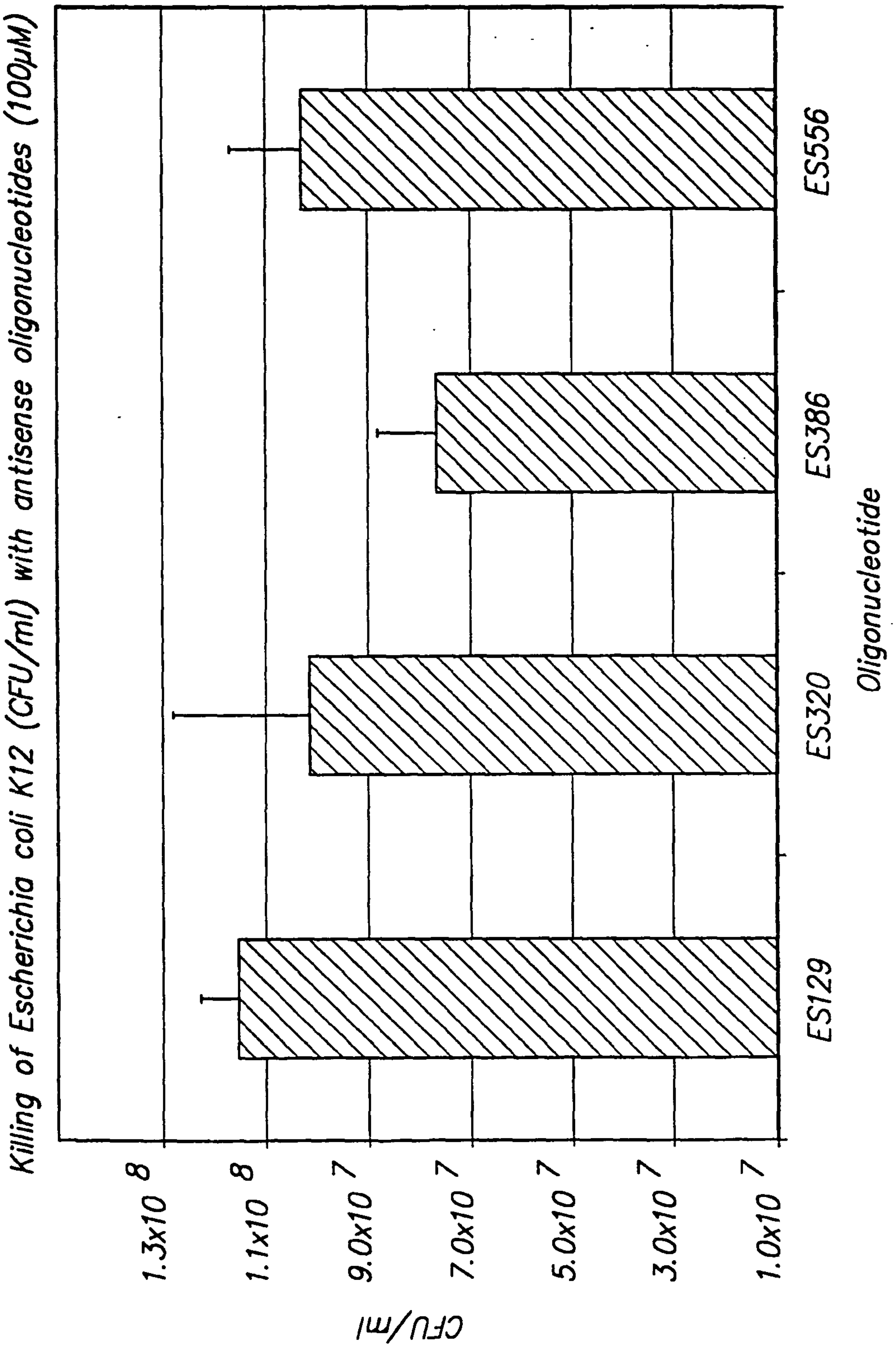
Killing of Escherichia coli K12 (CFU/ml) with antisense oligonucleotides (100μM)
CFU/ml
1.3x10 8
1.1x10 8
9.0x10 7
7.0x10 7
5.0x10 7
3.0x10 7
1.0x10 7
ES129
ES320
ES386
ES556
Oligonucleotide

FIG. 18A

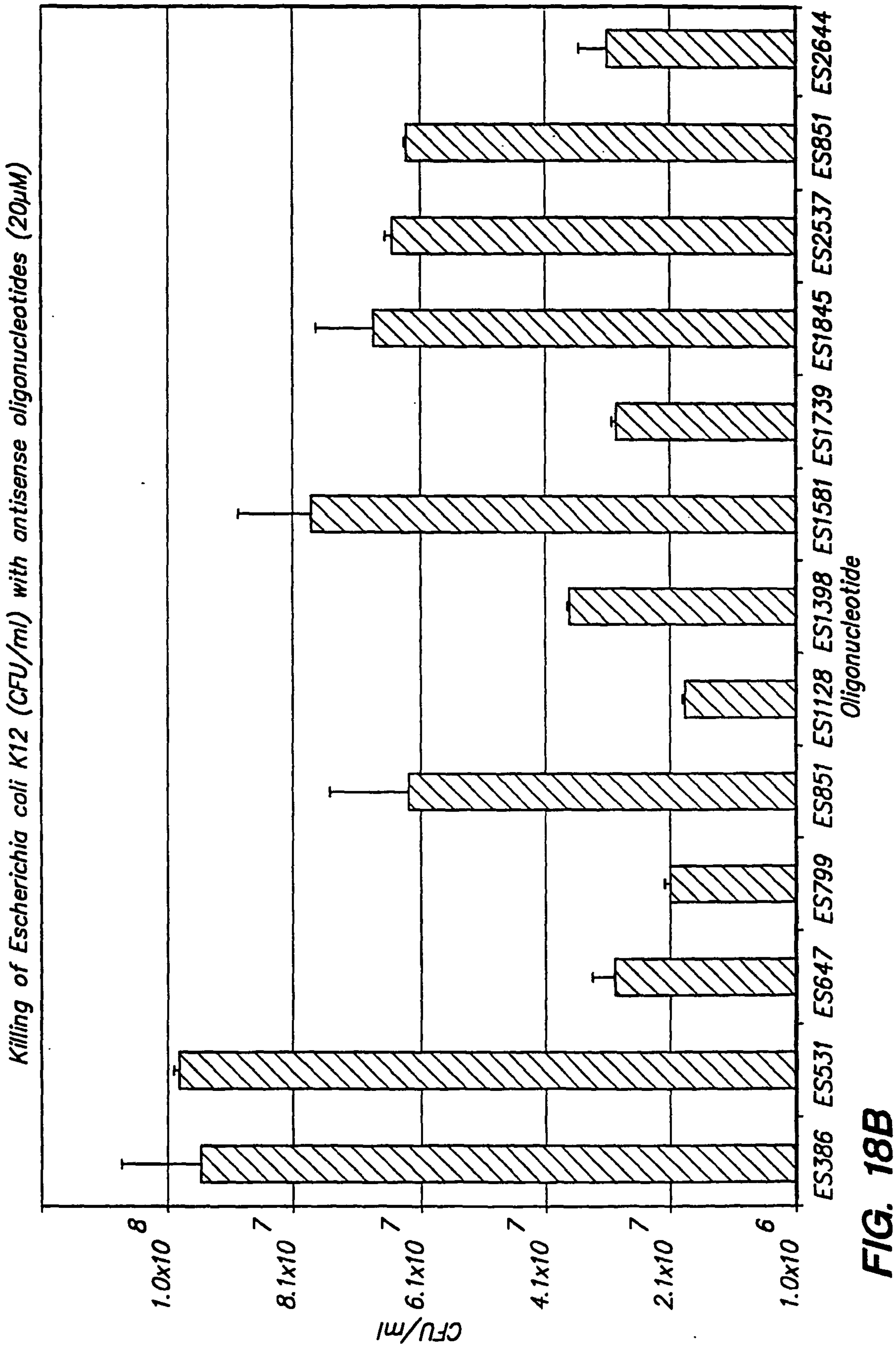

Killing of Escherichia coli K12 (CFU/ml) with antisense oligonucleotides (20μM)
CFU/ml
1.0x10^8
8.1x10^7
6.1x10^7
4.1x10^7
2.1x10^7
1.0x10^6
ES386
ES531
ES647
ES799
ES851
ES1128
ES1398
ES1581
ES1739
ES1845
ES2537
ES851
ES2644
Oligonucleotide


FIG. 18B

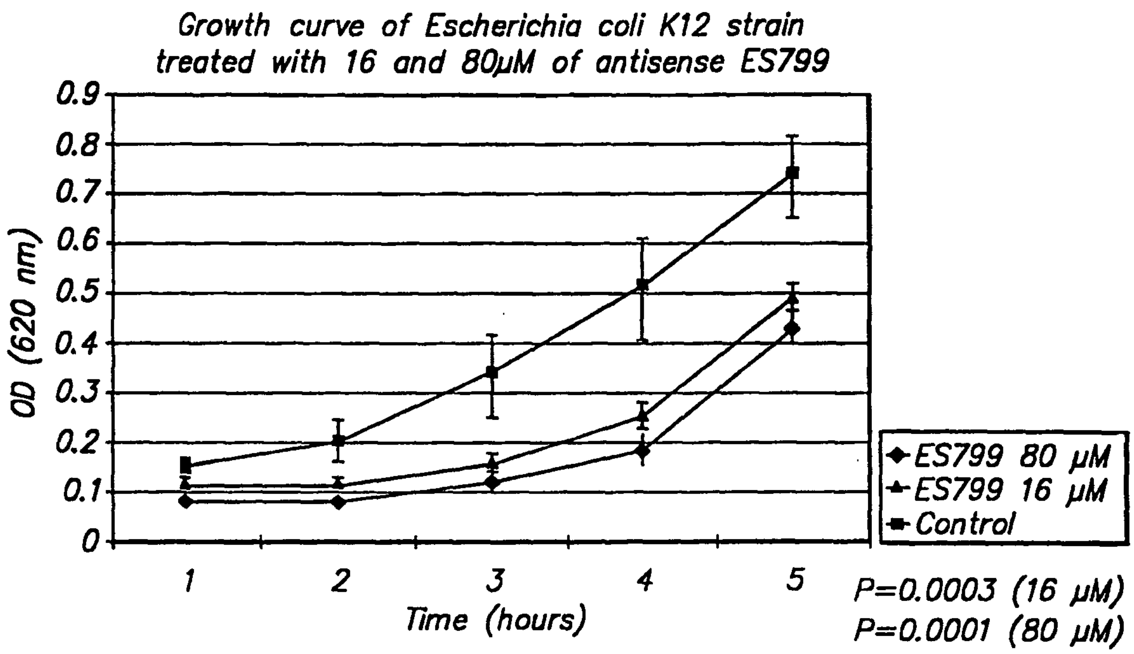
Growth curve of Escherichia coli K12 strain
treated with 16 and 80μM of antisense ES799
OD (620 nm)
0.9
0.8
0.7
0.6
0.5
0.4
0.3
0.2
0.1
0
1
2
3
4
5
Time (hours)
ES799 80 μM
ES799 16 μM
Control
P=0.0003 (16 μM)
P=0.0001 (80 μM)

FIG. 19A

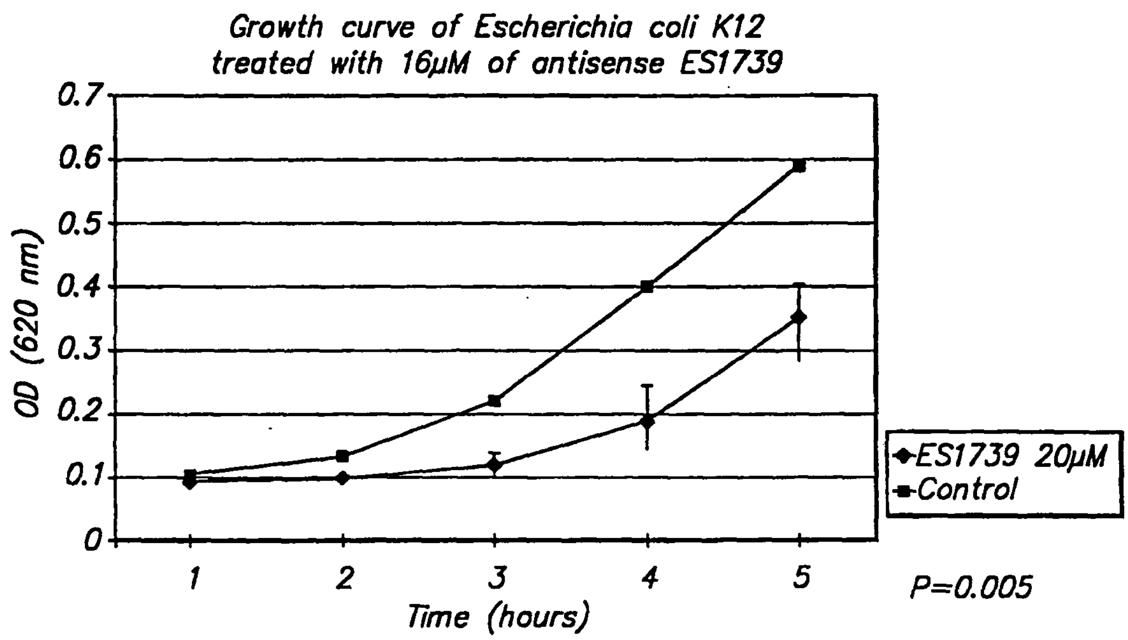
Growth curve of Escherichia coli K12
treated with 16μM of antisense ES1739
OD (620 nm)
0.7
0.6
0.5
0.4
0.3
0.2
0.1
0
1
2
3
4
5
Time (hours)
ES1739 20μM
Control
P=0.005

FIG. 19B

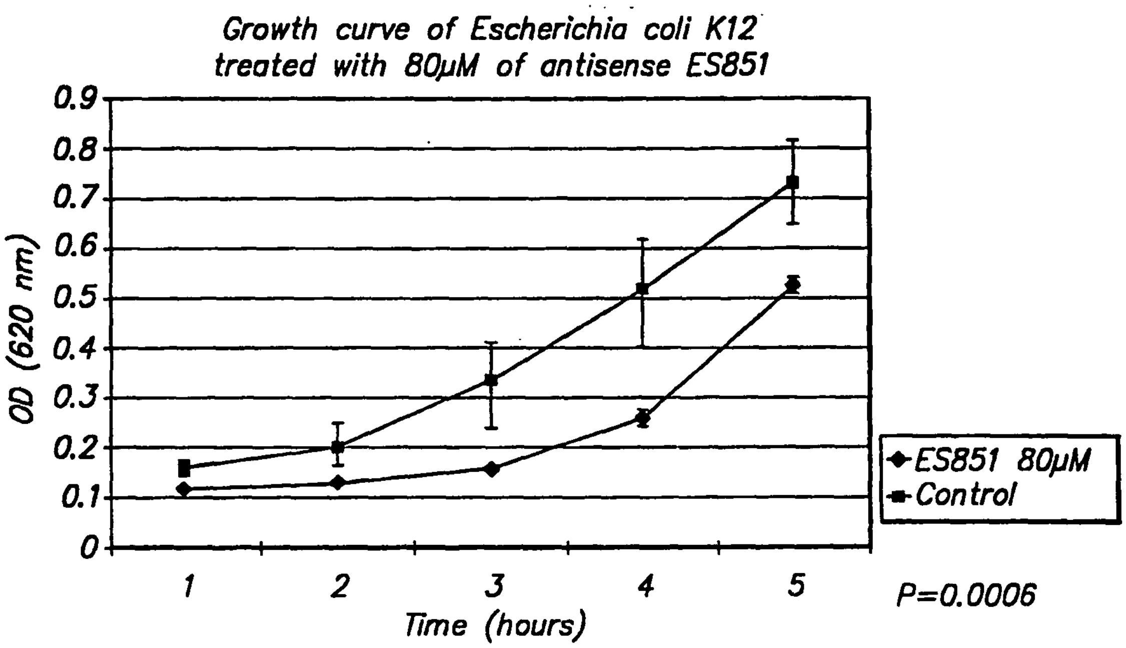
Growth curve of Escherichia coli K12
treated with 80μM of antisense ES851
OD (620 nm)
0.9
0.8
0.7
0.6
0.5
0.4
0.3
0.2
0.1
0
1
2
3
4
5
Time (hours)
ES851 80μM
Control
P=0.0006

FIG. 19C

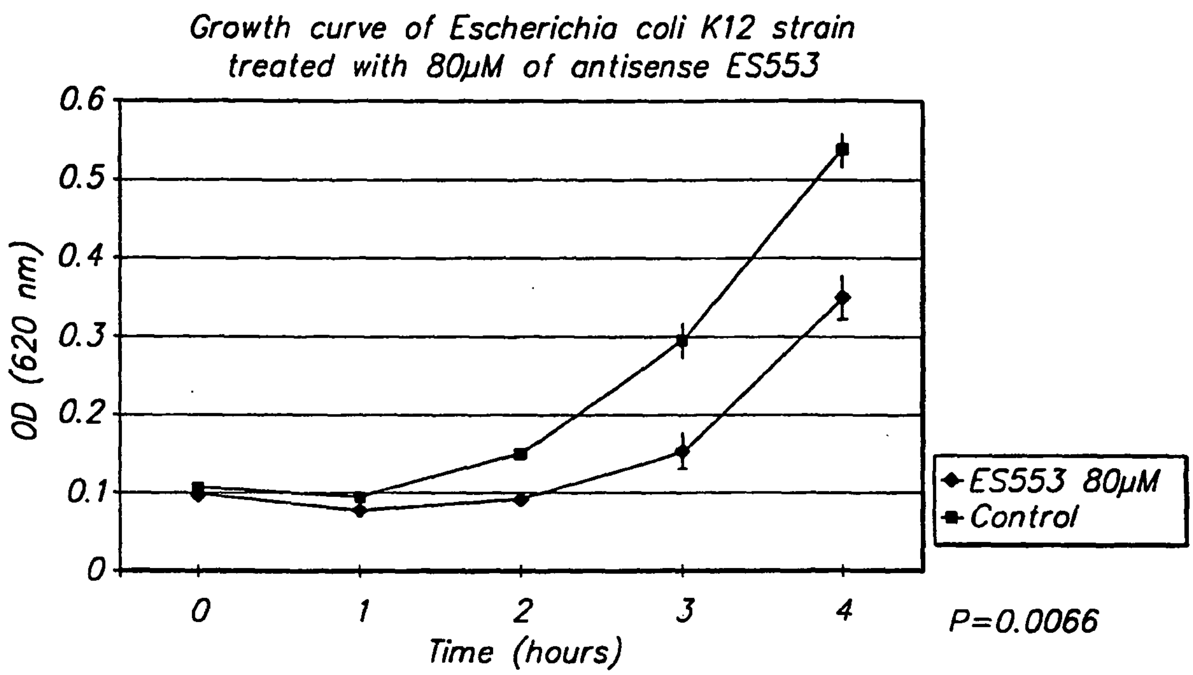
Growth curve of Escherichia coli K12 strain
treated with 80µM of antisense ES553
0.6
0.5
0.4
0.3
0.2
0.1
0
OD (620 nm)
0
1
2
3
4
Time (hours)
ES553 80µM
Control
P=0.0066

FIG. 19D

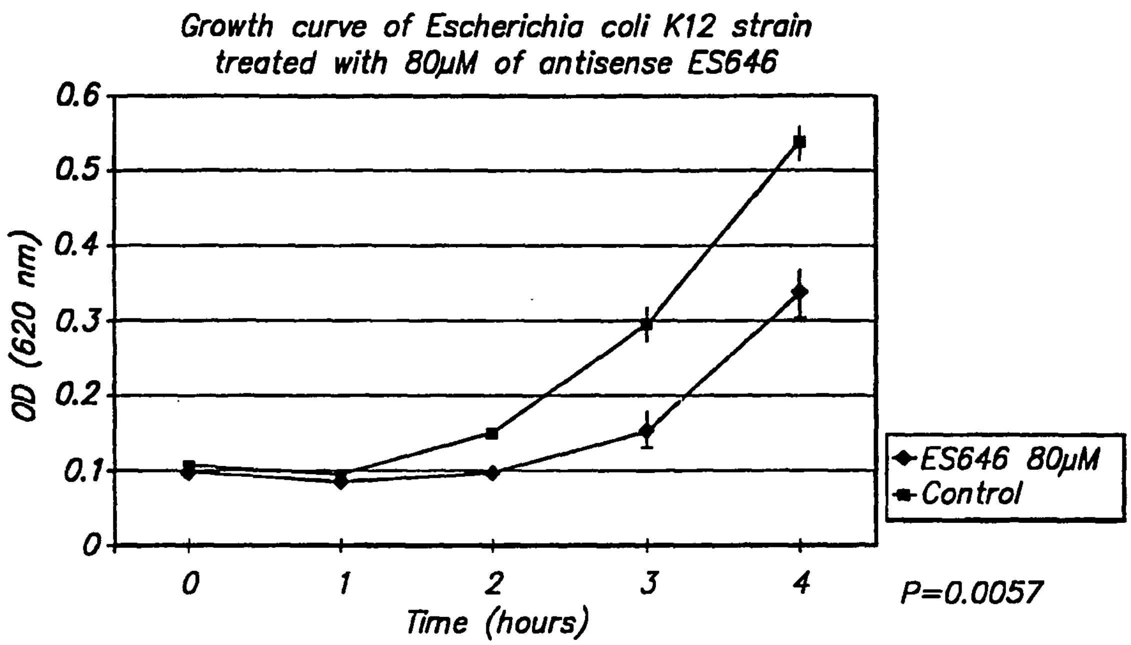

Growth curve of Escherichia coli K12 strain
treated with 80μM of antisense ES646
0.6
0.5
0.4
0.3
0.2
0.1
0
OD (620 nm)
0
1
2
3
4
Time (hours)
ES646 80μM
Control
P=0.0057


FIG. 19E

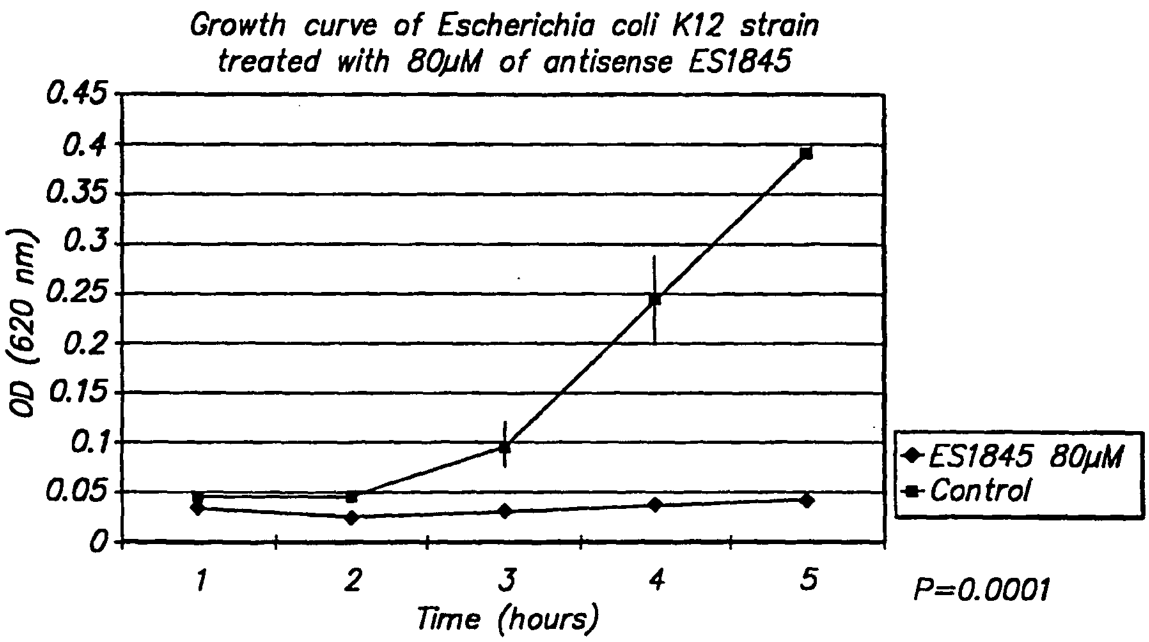
Growth curve of Escherichia coli K12 strain treated with 80μM of antisense ES1845
OD (620 nm)
0.45
0.4
0.35
0.3
0.25
0.2
0.15
0.1
0.05
0
1
2
3
4
5
Time (hours)
ES1845 80μM
Control
P=0.0001

FIG. 19F

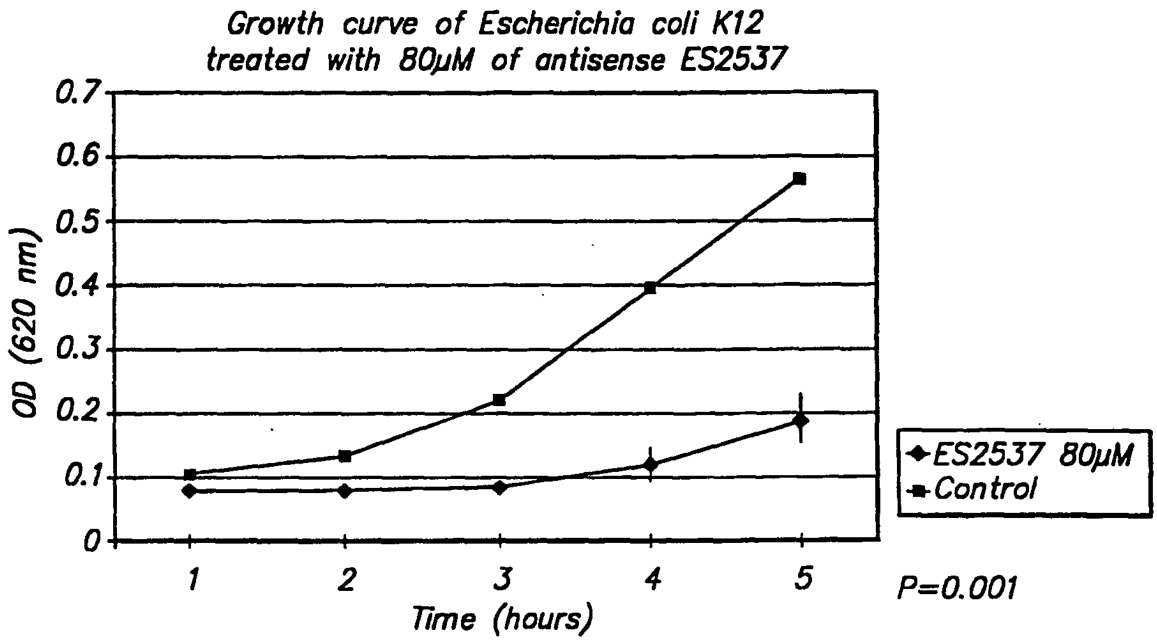
Growth curve of Escherichia coli K12
treated with 80μM of antisense ES2537
0.7
0.6
0.5
0.4
0.3
0.2
0.1
0
OD (620 nm)
1
2
3
4
5
Time (hours)
ES2537 80μM
Control
P=0.001

FIG. 19G